# EUROPEAN PATENT APPLICATION

(11) **EP 4 464 705 A1**
(43) Date of publication of application: **20.11.2024**
(21) Application number: 23740030.4
(22) Date of filing: 10.01.2023
(51) Int. Cl.: C07D 471/04, C07D 403/00, A61K 45/06, A61P 9/00, A61P 25/00, A61P 27/02, A61P 35/00, A61P 17/16, A61P 11/00, A61P 3/00, A61P 37/02, A61P 9/10, A61P 13/12, A61P 19/00, A61P 15/10, A61P 7/02

(54) **SALT OF ROCK INHIBITOR, CRYSTAL FORM OF SALT, COMPOSITION, AND PHARMACEUTICAL USE**

(30) Priority: 13.01.2022 CN 202210039840; 05.01.2023 CN 202310016976
(71) Applicant: Wuhan Createrna Science and Technology Co.,Ltd., Wuhan, Hubei 430075 (CN)
(72) Inventor: CHENG, Yuan, Wuhan, Hubei 430075 (CN); LOU, Jun, Wuhan, Hubei 430075 (CN); WANG, Liang, Wuhan, Hubei 430075 (CN); ZHANG, Yihan, Wuhan, Hubei 430075 (CN); CHEN, Yongkai, Wuhan, Hubei 430075 (CN); PENG, Wei, Wuhan, Hubei 430075 (CN); WANG, Chaodong, Wuhan, Hubei 430075 (CN)
(74) Representative: Gille Hrabal Partnerschaftsgesellschaft mbB Patentanwälte
(86) International application number: PCT/CN2023/071689
(87) International publication number: WO 2023/134688

(57) **Abstract**

A salt of a ROCK inhibitor, a crystal form of the salt, a composition, and a pharmaceutical use. The salt and the crystal form of the salt are an acid addition salt of a compound I and any acid in the following and a crystal form thereof: hydrochloric acid, sulfuric acid, p-toluenesulfonic acid, benzenesulfonic acid, maleic acid, tartaric acid, oxalic acid, ethanesulfonic acid, 2-hydroxyethanesulfonic acid, camphorsulfonic acid, and 1, 5-naphthalene disulfonic acid. A free alkali crystal form of the compound I, the salt, and the crystal form of the salt have stable physical and chemical properties.

## Description

The present application claims priority to the following two prior applications: the prior application with the patent application No. 202210039840.3 and entitled "SALT OF ROCK INHIBITOR, CRYSTAL FORM OF SALT, COMPOSITION, AND PHARMACEUTICAL USE" filed with China National Intellectual Property Administration on January 13, 2022; and the prior application with the patent application No. 202310016976.7 and entitled "SALT OF ROCK INHIBITOR, CRYSTAL FORM OF SALT, COMPOSITION, AND PHARMACEUTICAL USE" filed with China National Intellectual Property Administration on January 5, 2023, which are incorporated herein by reference in their entirety.

### TECHNICAL FIELD

The present disclosure relates to the field of pharmaceutical chemistry, and particularly to a salt of a ROCK inhibitor, a crystal form of the salt, a composition, and pharmaceutical use.

### BACKGROUND

Idiopathic interstitial pulmonary fibrosis (IPF) is a chronic and diffuse pulmonary interstitial disease with unknown cause and its characteristic pathological change is common interstitial pneumonia. It shows characteristic manifestation of common interstitial pneumonia according to lung histology and/or chest high-resolution CT (HRCT). The onset of IPF in patients is insidious, with the main clinical manifestations being dry cough and progressive dyspnea, which become pronounced after physical activity. The disease condition of IPF progresses irreversibly due to the complex pathogenesis, and early diagnosis is difficult. The survival rate of patients diagnosed with IPF is remarkably decreased over time, the 3-year survival rate is 50%, and the 5-year survival rate is only 20%, which is lower than that of most cancers (such as leukemia, breast cancer, colon cancer, uterine tumor, renal cancer, and the like). Therefore, IPF is called "non-cancerous cancer". At present, there are no definitely significantly effective drugs or methods for the treatment of IPF. According to the Chinese Expert Consensus on the Diagnosis and Treatment of Idiopathic Pulmonary Fibrosis published in 2016, only 3 types of drugs, namely pirfenidone, nintedanib and antacid medications, are "conditionally recommended for use". However, there is currently insufficient evidence to confirm that antacid medications can slow the decline in lung function in IPF patients. Currently, the only globally approved drugs on the market for IPF indications are pirfenidone and nintedanib; however, pirfenidone and nintedanib still have certain deficiencies in terms of efficacy and safety, and treatment options remain limited. There is an urgent need to develop drugs with other target mechanisms to meet the pressing needs of clinical patients and potential combination therapy requirements in the later period.

Rho-associated protein kinase (ROCK), also called Rho-associated kinase, is a serine/threonine protein kinase with a molecular mass of about 160 kD, and is a Rho downstream effector molecule that is studied most detailedly in terms of functional study at present. ROCK is distributed throughout various tissues in the body, and includes ROCK1 (ROKβ, p160-ROCK) and ROCK2 (ROKα) isoforms. It modulates actin filament assembly and actomyosin contraction, regulates the injury response of various cells, particularly epithelial cells, endothelial cells and fibroblasts, and mediates the fibrotic process in the pathogenesis of IPF. ROCK also plays a role in many signaling pathways involved in autoimmunity and inflammation. ROCK signals can interfere with a variety of fibrosis-initiating conditions, reduce fibroblast activation and collagen deposition, and improve organ function. Preclinical studies have shown that both ROCK1 and ROCK2 play a role in pulmonary fibrosis. Studies on pulmonary fibrosis in ROCK1+/- and ROCK2+/- mice have shown that both isoforms exhibit protective effects on vascular leakage, myofibroblast differentiation, and fibrosis, and ROCK1+/- mice demonstrate a greater attenuation of epithelial cell apoptosis. ROCK kinases are activated in the lungs of IPF patients and animal models associated with the disease, whereas ROCK kinase inhibitors can prevent the process of tissue fibrosis in the models and induce the regression of established fibrosis. Regarding the safety of inhibiting ROCK1 and ROCK2, there is evidence suggesting that while ROCK inhibitors can induce vasodilation, they do not necessarily cause systemic hypotension. Therefore, ROCK kinase inhibitors have great potential for the treatment of idiopathic interstitial pulmonary fibrosis.

Currently, three ROCK inhibitors have been marketed globally, including fasudil, ripasudil, and netarsudil, and the indications of these three drugs are cerebral vasospasm, glaucoma, and high intraocular pressure. Currently, there are no ROCK inhibitors on the market for the indications of IPF.

The development of new medicaments requires careful optimization of the chemical and biological properties of lead compounds. Further, the compounds must have desired pharmacokinetic and pharmacodynamic characteristics. This laborious development process typically requires extensive experimentation. In many cases, the process of determining the optimal compound often requires the preparation of thousands of structurally similar compounds. Therefore, improving ROCK kinase inhibitors and developing novel backbone compounds having inhibitory effects on ROCK1 and/or ROCK2 kinases is of positive significance for the treatment of the diseases described above. Meanwhile, developing pharmaceutical forms of these compounds suitable for drug preparation, such as those with improved stability (e.g., storage stability), hygroscopicity, and/or efficacy (e.g., *in-vivo* bioavailability), so as to achieve favorable results in drug manufacturing and medication stages, has become an urgent technical problem to be solved.

### SUMMARY

The present disclosure provides a salt of compound I: 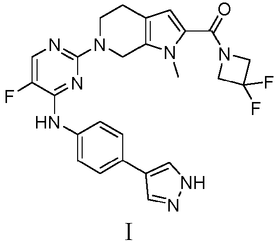 wherein the salt is an acid addition salt; for example, the salt is an acid addition salt of compound I with any one of the following acids: hydrochloric acid, sulfuric acid, *p*-toluenesulfonic acid, benzenesulfonic acid, maleic acid, tartaric acid (including L-tartaric acid or R-tartaric acid), oxalic acid, ethanesulfonic acid, 2-hydroxyethanesulfonic acid, camphorsulfonic acid, and 1,5-naphthalenedisulfonic acid, preferably with sulfuric acid, tartaric acid (including L-tartaric acid or R-tartaric acid), ethanesulfonic acid, or 2-hydroxyethanesulfonic acid.

According to an embodiment of the present disclosure, a hydrochloride of compound I is an acid addition salt of compound I with hydrochloric acid, wherein preferably, the molar ratio of compound I to the hydrochloric acid is 1:(0.9-1.2), such as 1: 1.

According to an embodiment of the present disclosure, a sulfate of compound I is an acid addition salt of compound I with sulfuric acid, wherein preferably, the molar ratio of compound I to the sulfuric acid is 1:(0.4-0.6), such as 1:0.5.

According to an embodiment of the present disclosure, a *p*-toluenesulfonate of compound I is an acid addition salt of compound I with *p*-toluenesulfonic acid, wherein preferably, the molar ratio of compound I to the *p*-toluenesulfonic acid is 1:(0.9-1.2), such as 1: 1.

According to an embodiment of the present disclosure, a maleate of compound I is an acid addition salt of compound I with maleic acid, wherein preferably, the molar ratio of compound I to the maleic acid is 1:(0.9-1.2), such as 1:1.

According to an embodiment of the present disclosure, an oxalate of compound I is an acid addition salt of compound I with oxalic acid, wherein preferably, the molar ratio of compound I to the oxalic acid is 1:(0.9-1.2), such as 1:1.

According to an embodiment of the present disclosure, a camphorsulfonate of compound I is an acid addition salt of compound I with camphorsulfonic acid, wherein preferably, the molar ratio of compound I to the camphorsulfonic acid is 1:(0.9-1.2), such as 1: 1.

According to an embodiment of the present disclosure, a 2-hydroxyethanesulfonate of compound I is an acid addition salt of compound I with 2-hydroxyethanesulfonic acid, wherein preferably, the molar ratio of compound I to the 2-hydroxyethanesulfonic acid is 1:(0.9-1.2), such as 1: 1.

According to an embodiment of the present disclosure, an ethanesulfonate of compound I is an acid addition salt of compound I with ethanesulfonic acid, wherein preferably, the molar ratio of compound I to the ethanesulfonic acid may be 1:(0.9-1.2), such as 1: 1 or 1:1.05.

According to an embodiment of the present disclosure, a tartrate of compound I is an acid addition salt of compound I with tartaric acid, wherein preferably, the molar ratio of compound I to the tartaric acid may be 1:(0.9-1.2), such as 1: 1, wherein the tartaric acid is L-tartaric acid or R-tartaric acid. According to an embodiment of the present disclosure, a 1,5-naphthalenedisulfonate of compound I is an acid addition salt of compound I with 1,5-naphthalenedisulfonic acid, wherein preferably, the molar ratio of compound I to the 1,5-naphthalenedisulfonic acid may be 1:(0.8-1.1), such as 1:0.9. According to an embodiment of the present disclosure, a benzenesulfonate of compound I is an acid addition salt of compound I with benzenesulfonic acid, wherein preferably, the molar ratio of compound I to the benzenesulfonic acid may be 1:(0.9-1.2), such as 1: 1.

According to an embodiment of the present disclosure, the salt of compound I may comprise water of crystallization or may not comprise water of crystallization (e.g., the salt of compound I may be an organic solvate); for example, it contains 1, 1.5, 2, 2.5, 3, or more molecules of water of crystallization.

According to an embodiment of the present disclosure, the salt of compound I may be in an amorphous form or a crystal form.

The present disclosure further provides a free base crystal form of compound I described above. According to an embodiment of the present disclosure, the free base crystal form is a free base crystal form I.

According to an embodiment of the present disclosure, by X-ray powder diffraction using Cu-Kα radiation, the free base crystal form I has characteristic peaks at 2θ angles of 13.1±0.2° and 20.5±0.2°, further has a characteristic peak at a 2θ angle of 8.4±0.2°, and still further has characteristic peaks at 2θ angles of 6.2±0.2°, 6.5±0.2°, 10.9±0.2°, 12.3±0.2°, 14.1±0.2°, 14.4±0.2°, 24.2±0.2°, and 25.3±0.2°.

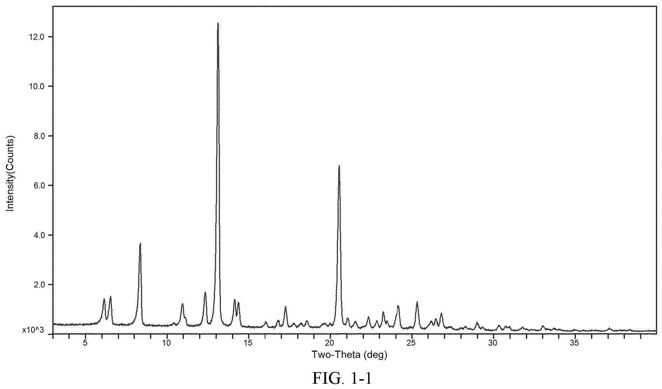

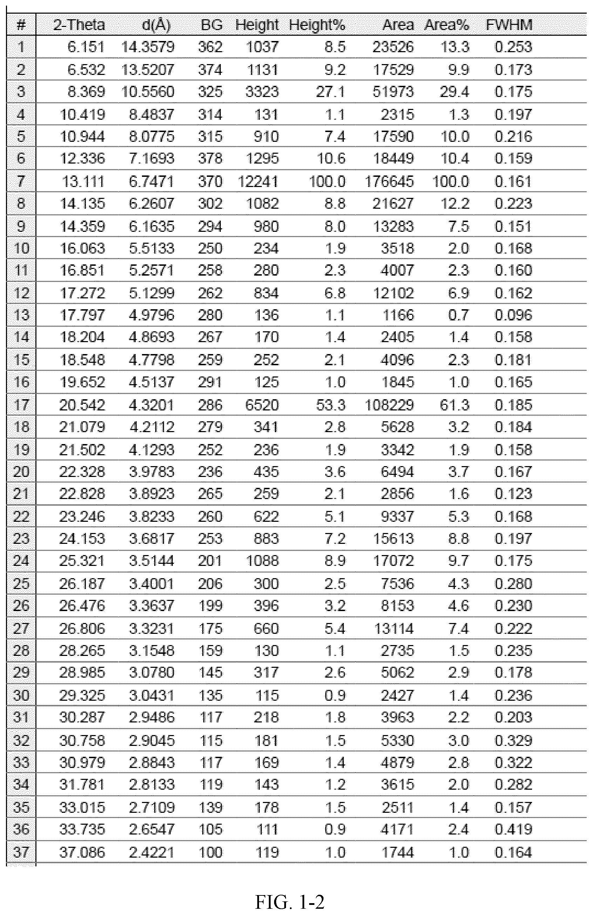

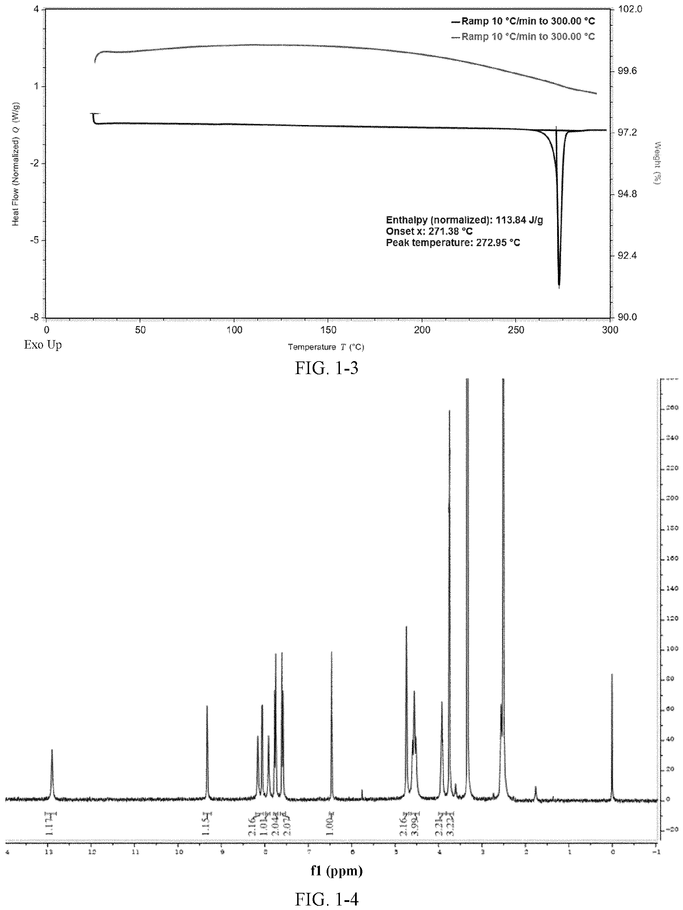

According to an embodiment of the present disclosure, the free base crystal form I has an X-ray powder diffraction pattern substantially as shown in FIG. 1-1.

According to an embodiment of the present disclosure, by X-ray powder diffraction using Cu-Kα radiation, the free base crystal form I has characteristic peaks at 2θ angles as shown in FIG. 1-2, with an error range of ±0.2°.

According to an embodiment of the present disclosure, the free base crystal form I is an anhydrate. According to an embodiment of the present disclosure, the free base crystal form I has almost no weight loss before a temperature of 150±2 °C.

According to an embodiment of the present disclosure, the free base crystal form I has only one endothermic peak with a peak temperature of about 273±5 °C, such as 273±2 °C.

Preferably, the free base crystal form I has a DSC-TGA profile substantially as shown in FIG. 1-3.

According to an embodiment of the present disclosure, the free base crystal form I contains a residual organic solvent, wherein, for example, the organic solvent is dichloromethane and/or tetrahydrofuran. For example, the content of the organic solvent is 0. 1%-3%, such as 1.2%. Illustratively, the free base crystal form I contains 0.5% dichloromethane and 0.7% tetrahydrofuran.

According to an embodiment of the present disclosure, the free base crystal form I has a ¹H-NMR spectrum substantially as shown in FIG. 1-4.

The present disclosure further provides a crystal form of the salt of compound I described above. According to an embodiment of the present disclosure, the crystal form of the salt is selected from crystal forms of acid addition salts of compound I.

According to an embodiment of the present disclosure, the crystal form of the salt is selected from one, two, or more of a crystal form of hydrochloride, a crystal form of sulfate, a crystal form of *p-*toluenesulfonate, a crystal form of benzenesulfonate, a crystal form of maleate, a crystal form of oxalate, a crystal form of camphorsulfonate, a crystal form of 2-hydroxyethanesulfonate, a crystal form of ethanesulfonate, a crystal form of tartrate, and a crystal form of 1,5-naphthalenedisulfonate of compound I described above.

According to an embodiment of the present disclosure, the crystal form of the salt may comprise or not comprise a solvent 1, wherein, for example, the solvent 1 is selected from an organic solvent 1 or water.

According to an embodiment of the present disclosure, the organic solvent 1 is selected from one, two, or more of methanol, ethanol, isopropanol, butanol, acetone, butanone, ethyl acetate, isopropyl acetate, methyl *tert*-butyl ether, dichloromethane, acetonitrile, tetrahydrofuran, *n*-heptane, dimethylsulfoxide, 2-methyl-tetrahydrofuran, and chloroform.

According to an embodiment of the present disclosure, the water may be water of crystallization or non-crystal water.

According to an embodiment of the present disclosure, the crystal form of hydrochloride is a crystal form I of hydrochloride.

According to an embodiment of the present disclosure, by X-ray powder diffraction using Cu-Kα radiation, the crystal form I of hydrochloride has characteristic peaks at 2θ angles of 13.1±0.2°, 22.6±0.2°, 23.0±0.2°, and 24.4±0.2°, further has characteristic peaks at 2θ angles of 8.0±0.2°, 8.3±0.2°, 18.2±0.2°, 18.7±0.2°, and 30.3±0.2°, and still further has characteristic peaks at 2θ angles of 15.8±0.2°, 16.2±0.2°, and 21.5±0.2°.

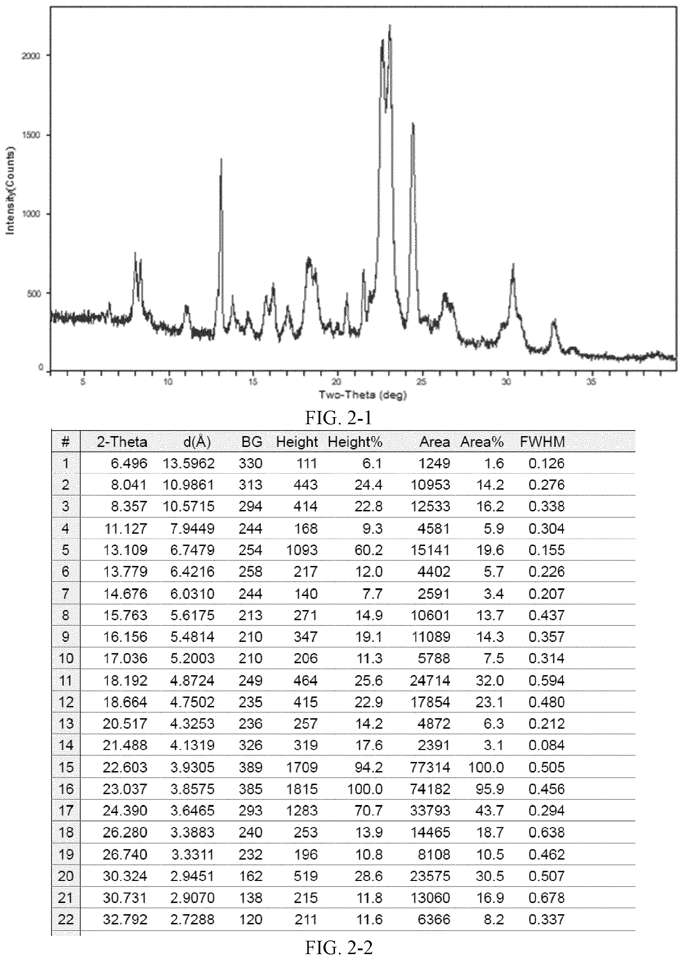

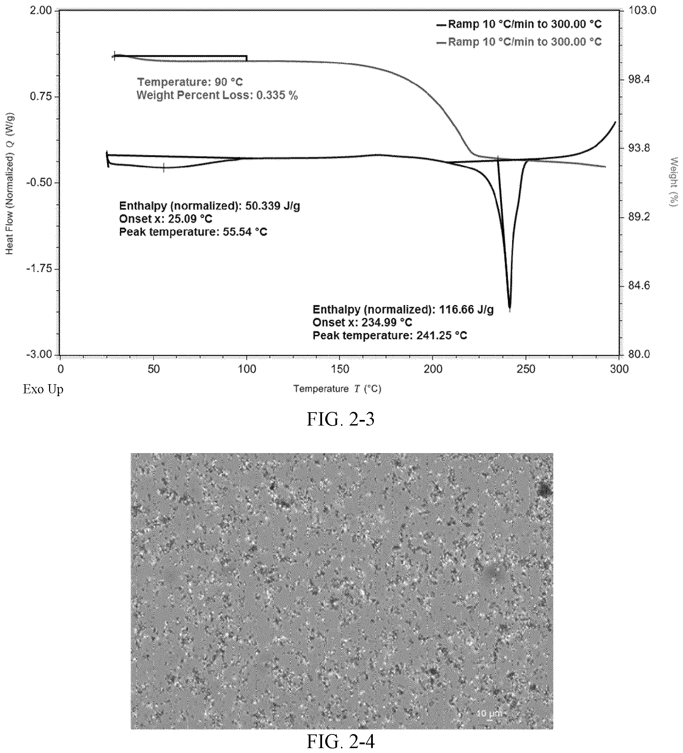

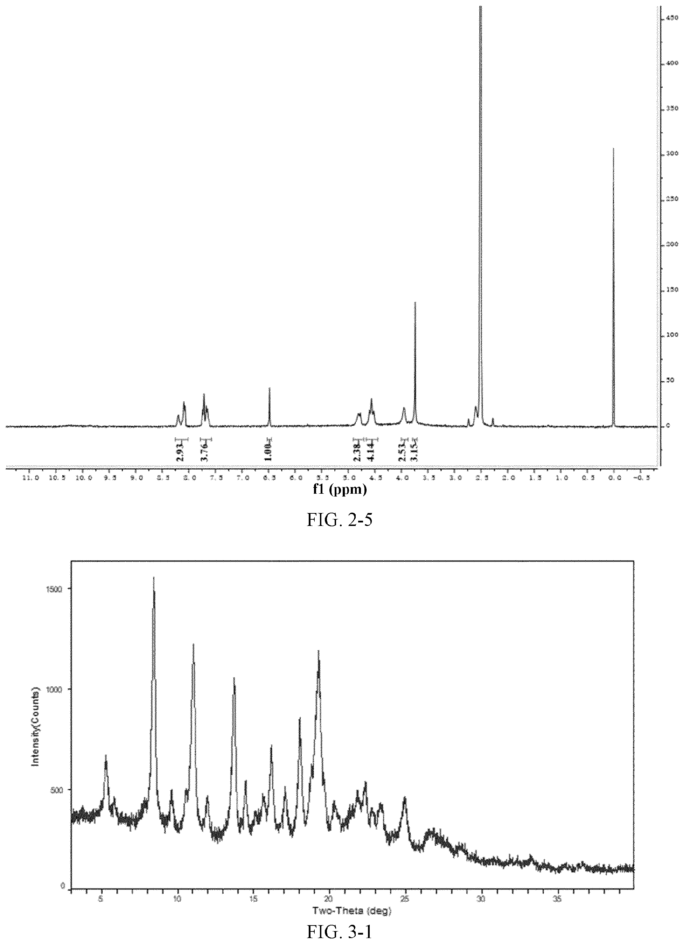

According to an embodiment of the present disclosure, the crystal form I of hydrochloride has an X-ray powder diffraction pattern substantially as shown in FIG. 2-1.

According to an embodiment of the present disclosure, by X-ray powder diffraction using Cu-Kα radiation, the crystal form I of hydrochloride has characteristic peaks at 2θ angles as shown in FIG. 2-2, with an error range of ±0.2°.

According to an embodiment of the present disclosure, the crystal form I of hydrochloride is an anhydrate.

According to an embodiment of the present disclosure, the crystal form I of hydrochloride has a weight loss of about 0.1%-1.0%, such as 0.3%, in a temperature range of from room temperature to about 90±2 °C.

According to an embodiment of the present disclosure, the crystal form I of hydrochloride has two endothermic peaks with peak temperatures of about 56±5 °C and 241±5 °C, respectively, such as endothermic peaks at 56±2 °C and 241±2 °C, respectively.

Preferably, the crystal form I of hydrochloride has a DSC-TGA profile substantially as shown in FIG. 2-3.

According to one embodiment of the present disclosure, the crystal form I of hydrochloride has a size of <10 µm. For example, in one embodiment, the crystal form I of hydrochloride has a polarizing microscope morphology substantially as shown in FIG. 2-4.

According to an embodiment of the present disclosure, the crystal form I of hydrochloride contains no residual organic solvent.

According to an embodiment of the present disclosure, the crystal form I of hydrochloride has a ¹H-NMR spectrum substantially as shown in FIG. 2-5.

According to an embodiment of the present disclosure, the crystal form of sulfate is a crystal form I of sulfate.

According to an embodiment of the present disclosure, by X-ray powder diffraction using Cu-Kα radiation, the crystal form I of sulfate has characteristic peaks at 2θ angles of 8.4±0.2°, 11.0±0.2°, 13.7±0.2°, and 19.3±0.2°, further has characteristic peaks at 2θ angles of 5.3±0.2°, 16.2±0.2°, and 18.0±0.2°, and still further has characteristic peaks at 2θ angles of 10.6±0.2°, 14.5±0.2°, 17.0±0.2°, 18.8±0.2°, 19.6±0.2°, 22.3±0.2°, and 24.9±0.2°.

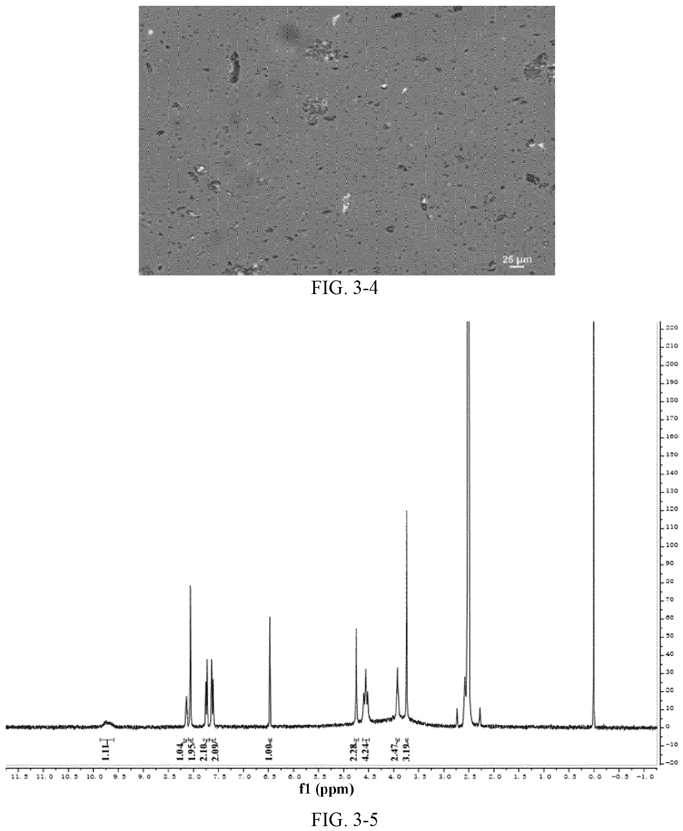

According to an embodiment of the present disclosure, the crystal form I of sulfate has an X-ray powder diffraction pattern substantially as shown in FIG. 3-1.

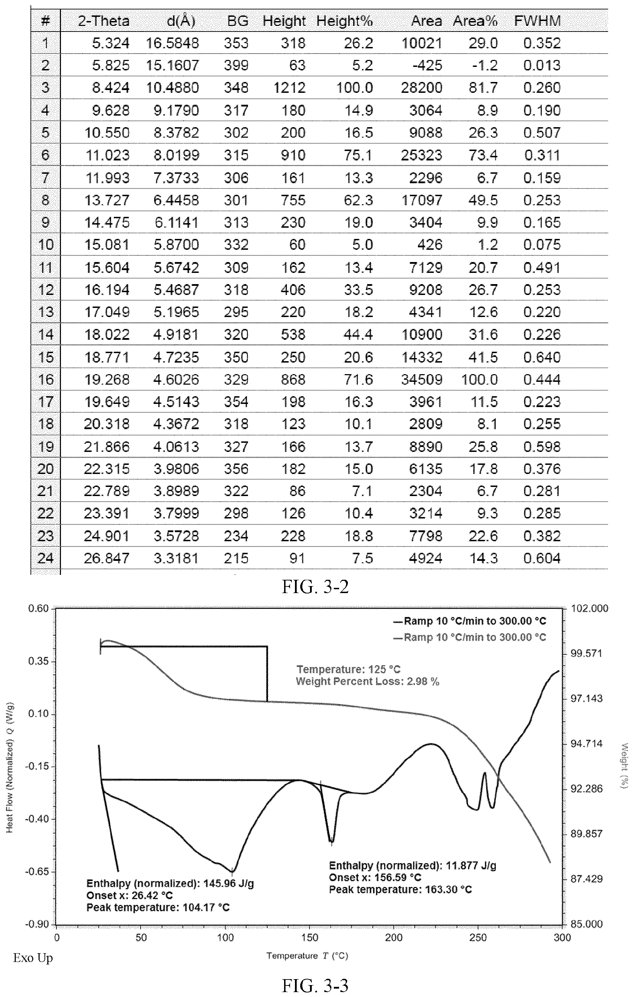

According to an embodiment of the present disclosure, by X-ray powder diffraction using Cu-Kα radiation, the crystal form I of sulfate has characteristic peaks at 2θ angles as shown in FIG. 3-2, with an error range of ±0.2°.

According to an embodiment of the present disclosure, the crystal form I of sulfate is a hydrate, such as a monohydrate, a dihydrate, or a trihydrate, preferably a monohydrate.

According to an embodiment of the present disclosure, the crystal form I of sulfate has a weight loss of about 0.1%-5.0%, such as 3%, in a temperature range of from room temperature to about 125±2 °C. According to an embodiment of the present disclosure, the crystal form I of sulfate has two endothermic peaks with peak temperatures of about 104±5 °C and 163±5 °C, such as 104±2 °C and 163±2 °C, respectively.

Preferably, the crystal form I of sulfate has a DSC-TGA profile substantially as depicted in FIG. 3-3. According to an embodiment of the present disclosure, the crystal form I of sulfate is in the form of irregular crystals. For example, in one embodiment, the crystal form I of sulfate has a polarizing microscope morphology substantially as shown in FIG. 3-4.

According to an embodiment of the present disclosure, the crystal form I of sulfate contains no residual organic solvent.

According to an embodiment of the present disclosure, the crystal form I of sulfate has a ¹H-NMR spectrum substantially as shown in FIG. 3-5.

According to an embodiment of the present disclosure, the crystal form of *p*-toluenesulfonate is a crystal form I of*p*-toluenesulfonate.

According to an embodiment of the present disclosure, by X-ray powder diffraction using Cu-Kα radiation, the crystal form I of *p*-toluenesulfonate has characteristic peaks at 2θ angles of 5.1±0.2° and 26.1±0.2°, further has characteristic peaks at 2θ angles of 11.8±0.2° and 26.5±0.2°, and still further has characteristic peaks at 2θ angles of 10.2±0.2°, 11.0±0.2°, 18.9±0.2°, 19.2±0.2°, and 21.0±0.2°.

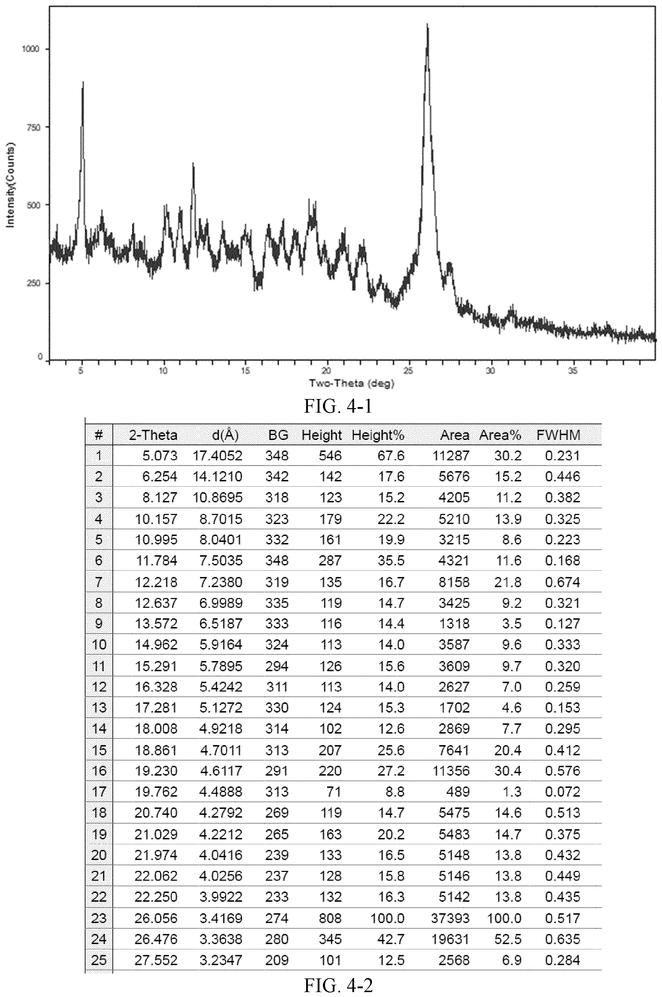

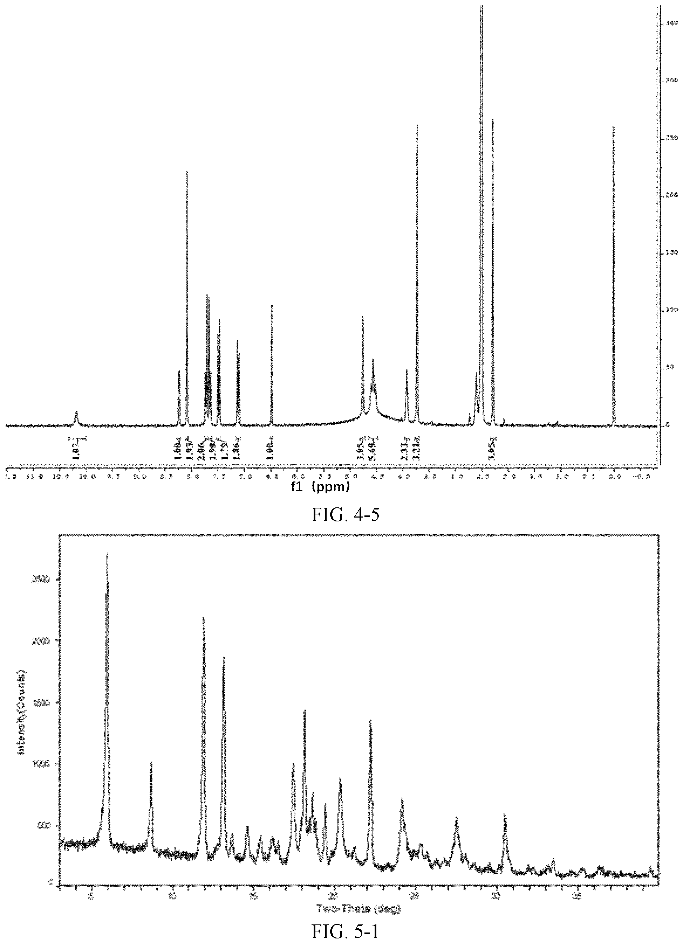

According to an embodiment of the present disclosure, the crystal form I of *p*-toluenesulfonate has an X-ray powder diffraction pattern substantially as shown in FIG. 4-1.

According to an embodiment of the present disclosure, by X-ray powder diffraction using Cu-Kα radiation, the crystal form I of *p*-toluenesulfonate has characteristic peaks at 2θ angles as shown in FIG. 4-2, with an error range of ±0.2°.

According to an embodiment of the present disclosure, the crystal form I of *p*-toluenesulfonate is a hydrate, such as a 0.5-hydrate, a 0.75-hydrate, or a monohydrate, preferably a 0.75-hydrate or a monohydrate.

According to an embodiment of the present disclosure, the crystal form I of *p*-toluenesulfonate has a weight loss of about 0.5%-5%, such as 2%, in a temperature range of from room temperature to about 110± 2 °C.

According to an embodiment of the present disclosure, the crystal form I of *p*-toluenesulfonate has two endothermic peaks with peak temperatures of about 94±5 °C and 198±5 °C, such as 94±2 °C and 198±2 °C, respectively.

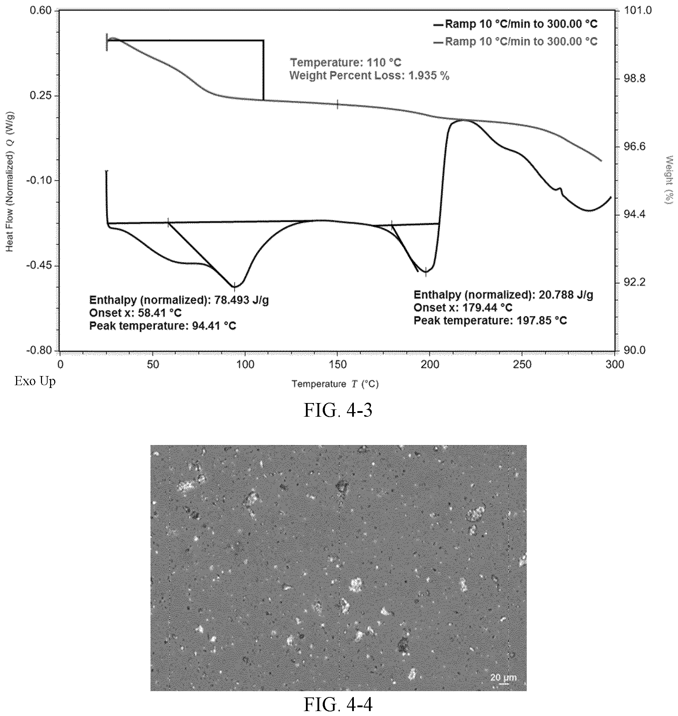

Preferably, the crystal form I of *p*-toluenesulfonate has a DSC-TGA profile substantially as depicted in FIG. 4-3.

According to an embodiment of the present disclosure, the crystal form I of *p*-toluenesulfonate is in the form of irregular crystals. For example, in one embodiment, the crystal form I of *p-*toluenesulfonate has a polarizing microscope morphology substantially as shown in FIG. 4-4. According to an embodiment of the present disclosure, the crystal form I of *p*-toluenesulfonate has a ¹H-NMR spectrum substantially as shown in FIG. 4-5, and contains a residual organic solvent (in a very small amount).

According to an embodiment of the present disclosure, the crystal form of benzenesulfonate is a crystal form I of benzenesulfonate.

According to an embodiment of the present disclosure, by X-ray powder diffraction using Cu-Kα radiation, the crystal form I of benzenesulfonate has characteristic peaks at 2θ angles of 6.0±0.2° and 12.0±0.2°, further has characteristic peaks at 2θ angles of 13.2±0.2°, 18.2±0.2°, and 22.2±0.2°, and still further has characteristic peaks at 2θ angles of 8.7±0.2°, 17.4±0.2°, 18.6±0.2°, 19.4±0.2°, 20.3±0.2°, 24.2±0.2°, and 30.5±0.2°.

According to an embodiment of the present disclosure, the crystal form I of benzenesulfonate has an X-ray powder diffraction pattern substantially as shown in FIG. 5-1.

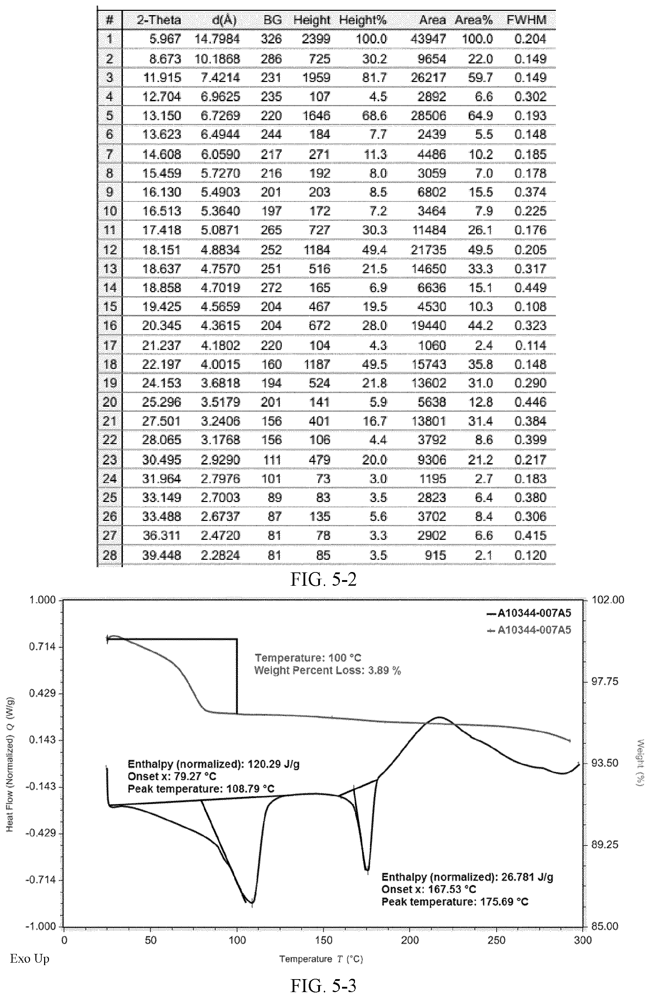

According to an embodiment of the present disclosure, by X-ray powder diffraction using Cu-Kα radiation, the crystal form I of benzenesulfonate has characteristic peaks at 2θ angles as shown in FIG. 5-2, with an error range of ±0.2°.

According to an embodiment of the present disclosure, the crystal form I of benzenesulfonate is a hydrate, such as a monohydrate, a 1.5-hydrate, a dihydrate, or a trihydrate, preferably a 1.5-hydrate or a monohydrate.

According to an embodiment of the present disclosure, the crystal form I of benzenesulfonate has a weight loss of about 1%-5%, such as 3.9%, in a temperature range of from room temperature to about 100±2 °C.

According to an embodiment of the present disclosure, the crystal form I of benzenesulfonate has two endothermic peaks with peak temperatures of about 109±5 °C and 176±5 °C, such as 109±2 °C and 176±2 °C, respectively.

Preferably, the crystal form I of benzenesulfonate has a DSC-TGA profile substantially as depicted in FIG. 5-3.

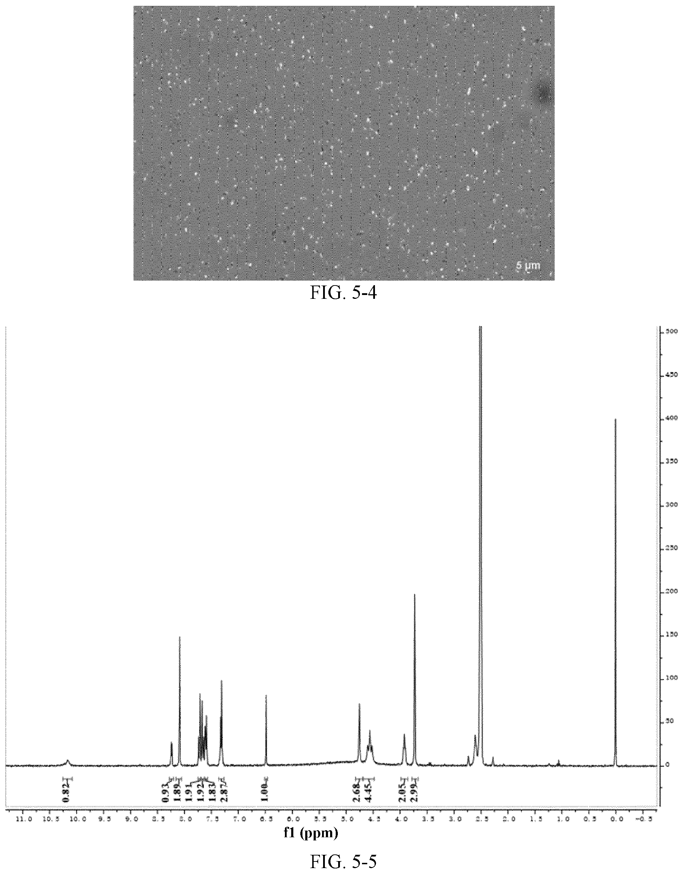

According to one embodiment of the present disclosure, the crystal form I of benzenesulfonate is in the form of fine crystals, for example, with a size of less than 20 µm, for another example, with a size of less than 10 µm. For example, in one embodiment, the crystal form I of benzenesulfonate has a polarizing microscope morphology substantially as shown in FIG. 5-4.

According to an embodiment of the present disclosure, the crystal form I of benzenesulfonate has a ¹H-NMR spectrum substantially as shown in FIG. 5-5.

According to an embodiment of the present disclosure, the crystal form of maleate comprises a crystal form I of maleate, a crystal form II of maleate, and a crystal form III of maleate.

According to an embodiment of the present disclosure, by X-ray powder diffraction using Cu-Kα radiation, the crystal form I of maleate has a characteristic peak at a 2θ angle of 24.8±0.2°, further has characteristic peaks at 2θ angles of 12.0±0.2° and 20.5±0.2°, and still further has characteristic peaks at 2θ angles of 9.3±0.2°, 13.2±0.2°, 17.4±0.2°, and 20.0±0.2°.

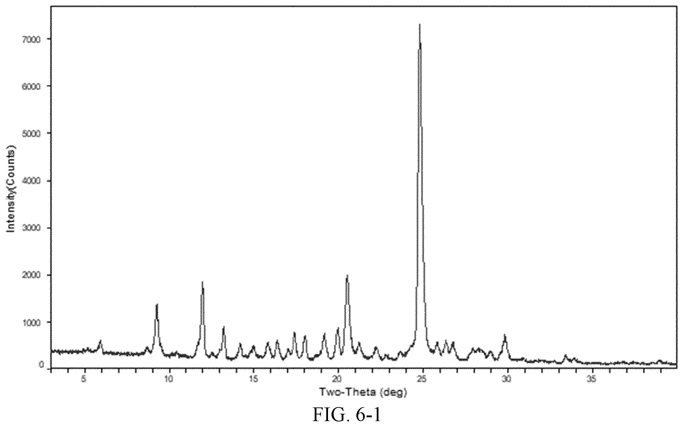

According to an embodiment of the present disclosure, the crystal form I of maleate has an X-ray powder diffraction pattern substantially as shown in FIG. 6-1.

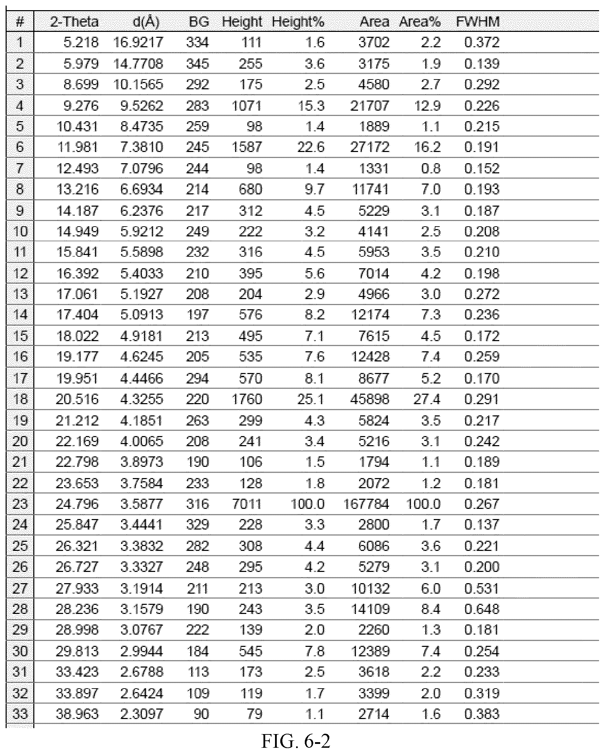

According to an embodiment of the present disclosure, by X-ray powder diffraction using Cu-Kα radiation, the crystal form I of maleate has characteristic peaks at 2θ angles as shown in FIG. 6-2, with an error range of ±0.2°.

According to an embodiment of the present disclosure, the crystal form I of maleate is a solvate, preferably an organic solvate, such as a methanol solvate, an ethanol solvate, or an isopropanol solvate. For example, the amount of the organic solvent is 2.0%-4.0%, such as 2.9%.

According to an embodiment of the present disclosure, the crystal form I of maleate has a weight loss of about 0.5%-5%, such as 2.6%, in a temperature range of from room temperature to about 180±2 °C. According to an embodiment of the present disclosure, the crystal form I of maleate has an endothermic peak at a peak temperature of about 198±5 °C.

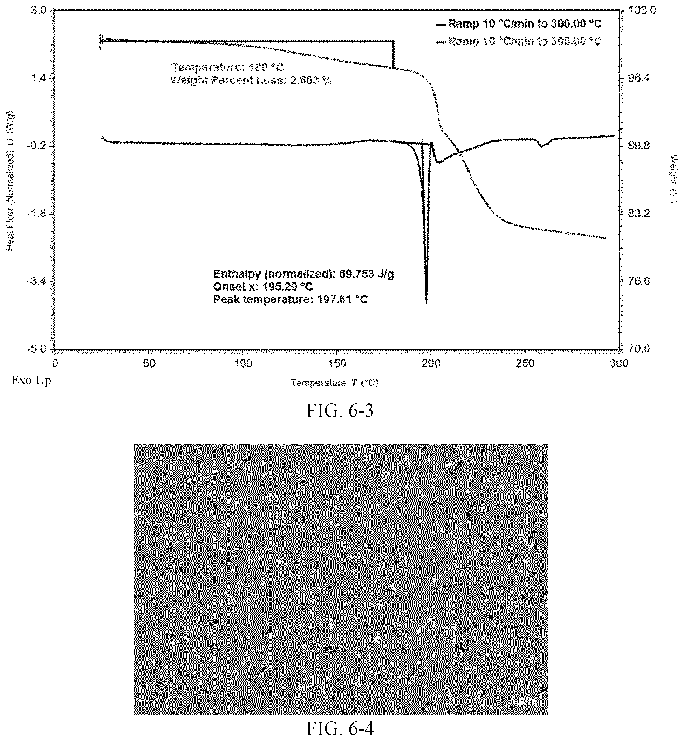

Preferably, the crystal form I of maleate has a DSC-TGA profile substantially as depicted in FIG. 6-3.

According to an embodiment of the present disclosure, the crystal form I of maleate is in the form of fine crystals; for example, in one embodiment, the crystal form I of maleate has a size of less than 20 µm, such as less than 10 µm. For example, in one embodiment, the crystal form I of maleate has a polarizing microscope morphology substantially as shown in FIG. 6-4.

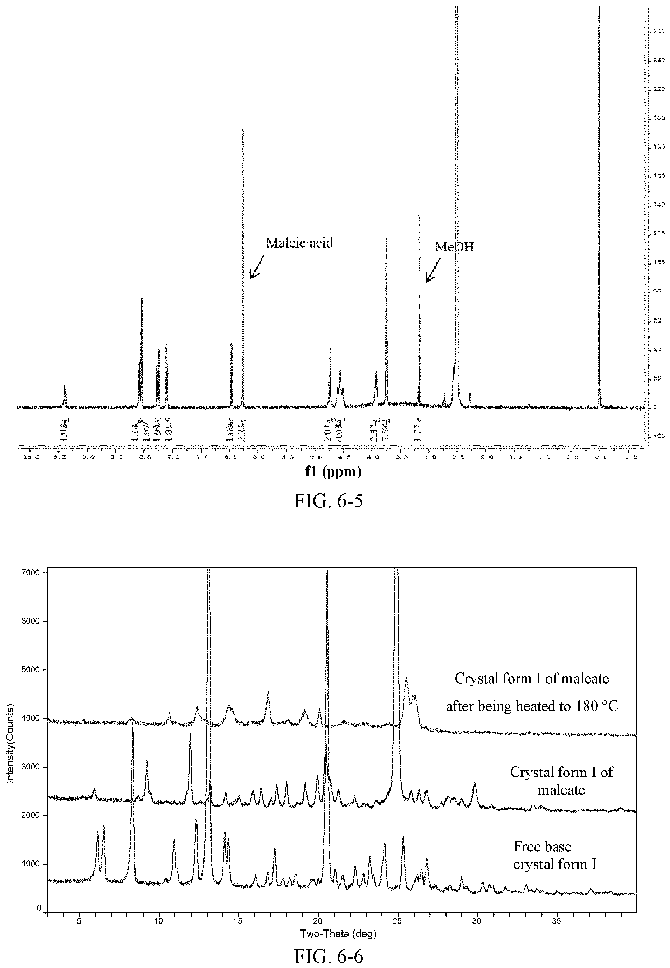

According to an embodiment of the present disclosure, the crystal form I of maleate has a ¹H-NMR spectrum substantially as shown in FIG. 6-5.

According to an embodiment of the present disclosure, by X-ray powder diffraction using Cu-Kα radiation, the crystal form II of maleate has characteristic peaks at 2θ angles of 11.0±0.2°, 14.4±0.2°, 16.2±0.2°, and 16.8±0.2°, further has characteristic peaks at 2θ angles of 5.4±0.2°, 12.1±0.2°, 13.4±0.2°, 17.2±0.2°, 20.3±0.2°, and 20.6±0.2°, and still further has characteristic peaks at 2θ angles of 7.2±0.2°, 17.8±0.2°, and 25.0±0.2°.

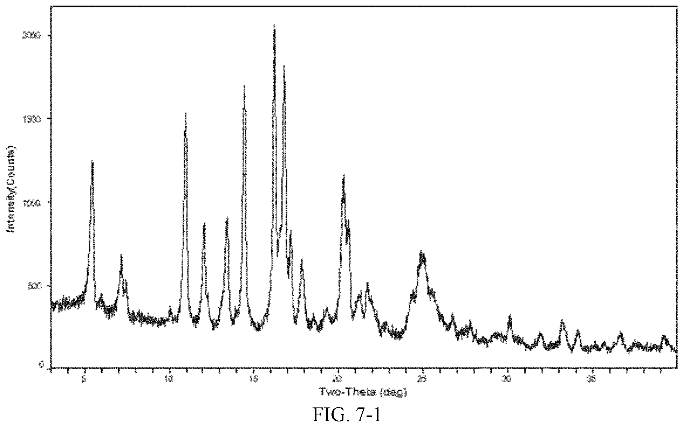

According to an embodiment of the present disclosure, the crystal form II of maleate has an X-ray powder diffraction pattern substantially as shown in FIG. 7-1.

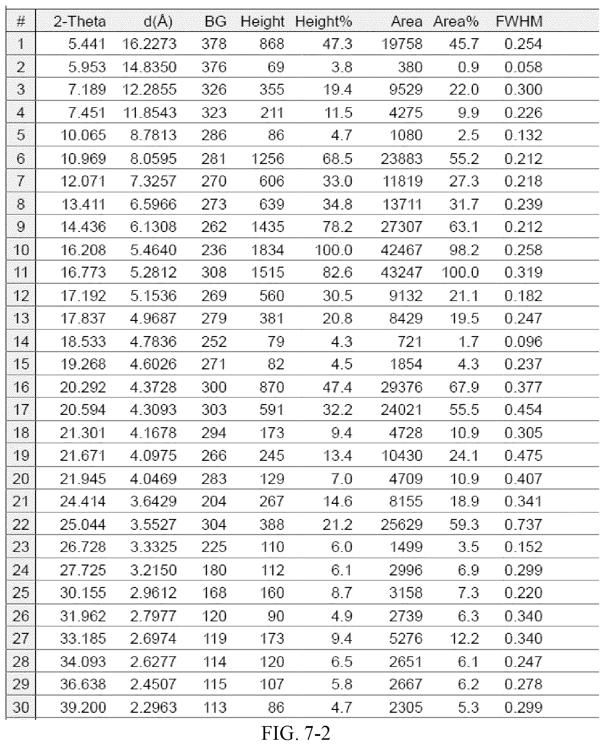

According to an embodiment of the present disclosure, by X-ray powder diffraction using Cu-Kα radiation, the crystal form II of maleate has characteristic peaks at 2θ angles as shown in FIG. 7-2, with an error range of ±0.2°.

According to an embodiment of the present disclosure, the crystal form II of maleate is an anhydrate. According to an embodiment of the present disclosure, the crystal form II of maleate has no significant weight loss in a temperature range of from room temperature to about 100±2 °C. According to an embodiment of the present disclosure, the crystal form II of maleate has two endothermic peaks with peak temperatures of about 48±5 °C and 190±5 °C, such as 48±2 °C and 190±2 °C, respectively.

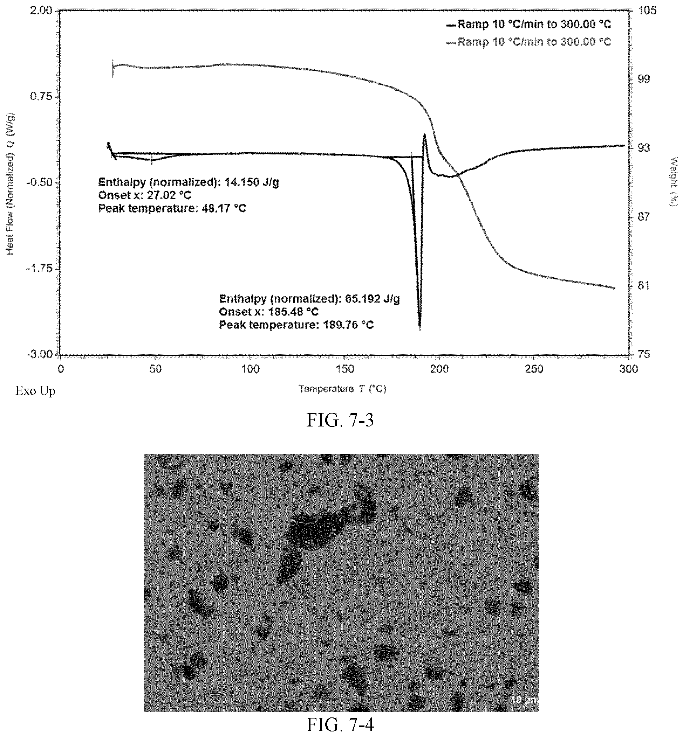

Preferably, the crystal form II of maleate has a DSC-TGA profile substantially as depicted in FIG. 7-3.

According to an embodiment of the present disclosure, the crystal form II of maleate is in the form of agglomerated particles. Preferably, the crystal form II of maleate has a polarizing microscope morphology substantially as shown in FIG. 7-4.

According to an embodiment of the present disclosure, the crystal form II of maleate contains a residual organic solvent. For example, the residual amount of the organic solvent is 2.0%-3.5%, such as 2.8%. For example, the organic solvent is one, two, or more (e.g., three) of acetonitrile, tetrahydrofuran, isopropanol, isopropyl acetate, and butanone.

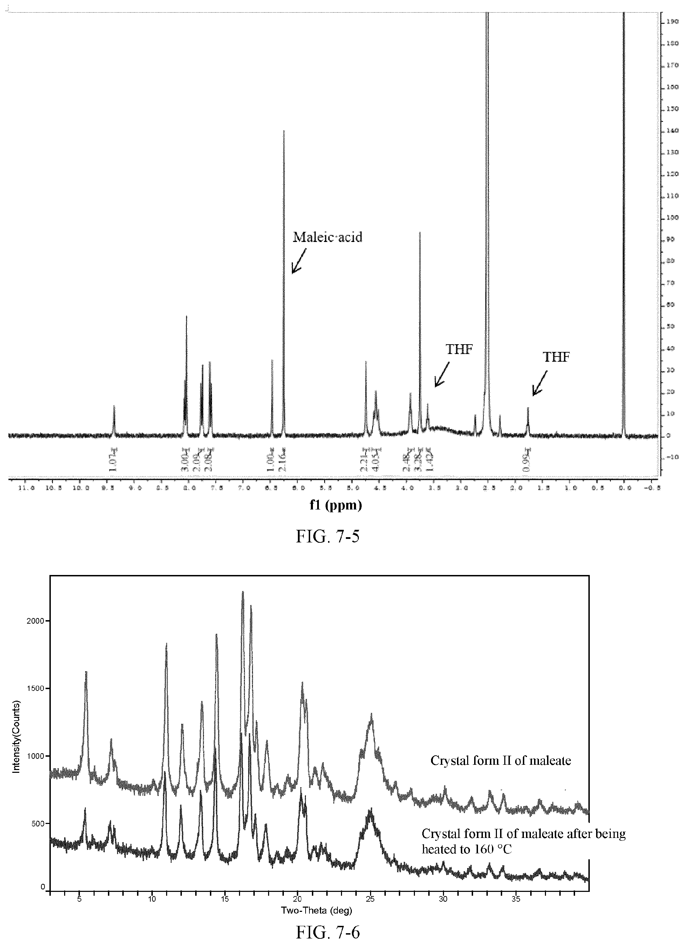

According to an embodiment of the present disclosure, the crystal form II of maleate has a ¹H-NMR spectrum substantially as shown in FIG. 7-5.

According to an embodiment of the present disclosure, by X-ray powder diffraction using Cu-Kα radiation, the crystal form III of maleate has characteristic peaks at 2θ angles of 14.6±0.2°, 16.9±0.2°, and 25.8±0.2°, further has characteristic peaks at 2θ angles of 5.3±0.2°, 10.7±0.2°, and 26.3±0.2°, and still further has characteristic peaks at 2θ angles of 12.4±0.2°, 16.1±0.2°, 19.2±0.2°, and 20.2±0.2°.

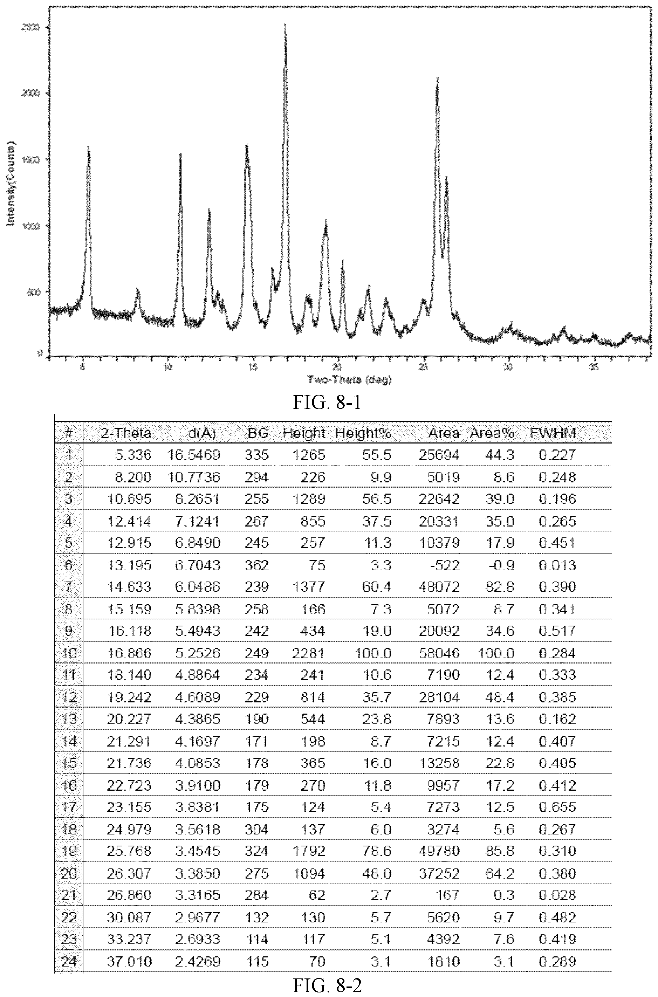

According to an embodiment of the present disclosure, the crystal form III of maleate has an X-ray powder diffraction pattern substantially as shown in FIG. 8-1.

According to an embodiment of the present disclosure, by X-ray powder diffraction using Cu-Kα radiation, the crystal form III of maleate has characteristic peaks at 2θ angles as shown in FIG. 8-2, with an error range of ±0.2°.

According to an embodiment of the present disclosure, the crystal form III of maleate is an anhydrate. According to an embodiment of the present disclosure, the crystal form III of maleate has no significant weight loss in a temperature range of from room temperature to about 150±2 °C. According to an embodiment of the present disclosure, the crystal form III of maleate has an endothermic peak at a peak temperature of about 197±5 °C.

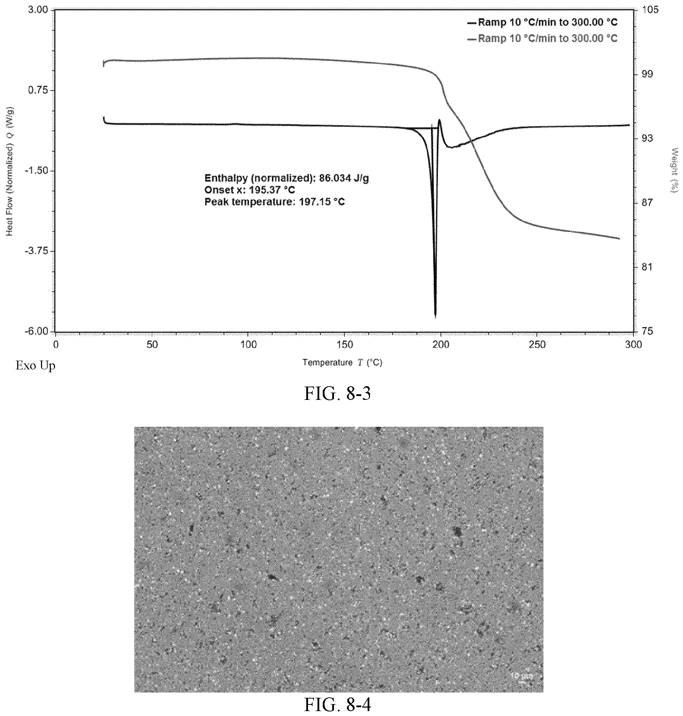

Preferably, the crystal form III of maleate has a DSC-TGA profile substantially as depicted in FIG. 8-3.

According to an embodiment of the present disclosure, the crystal form III of maleate is in the form of fine crystals. For example, in one embodiment, the crystal form III of maleate has a polarizing microscope morphology substantially as shown in FIG. 8-4.

According to an embodiment of the present disclosure, the crystal form III of maleate contains a residual organic solvent. For example, the residual amount of the organic solvent is 0.05%-1%, such as 0.5%. For example, the organic solvent is one, two, or more (e.g., three) of acetone, isopropyl acetate, butanol, acetonitrile, and butanone.

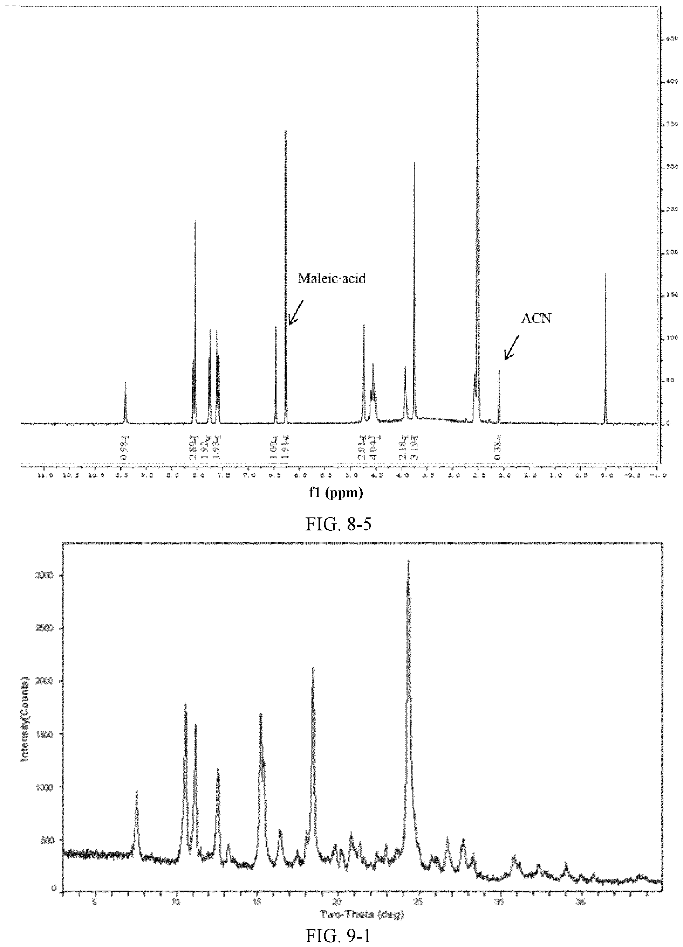

According to an embodiment of the present disclosure, the crystal form III of maleate has a ¹H-NMR spectrum substantially as shown in FIG. 8-5.

According to an embodiment of the present disclosure, the crystal form of oxalate is a crystal form I of oxalate.

According to an embodiment of the present disclosure, by X-ray powder diffraction using Cu-Kα radiation, the crystal form I of oxalate has characteristic peaks at 2θ angles of 18.4±0.2° and 24.3±0.2°, further has characteristic peaks at 2θ angles of 10.6±0.2° and 15.2±0.2°, and still further has characteristic peaks at 2θ angles of 7.6±0.2°, 11.2±0.2°, and 12.6±0.2°.

According to an embodiment of the present disclosure, the crystal form I of oxalate has an X-ray powder diffraction pattern substantially as shown in FIG. 9-1.

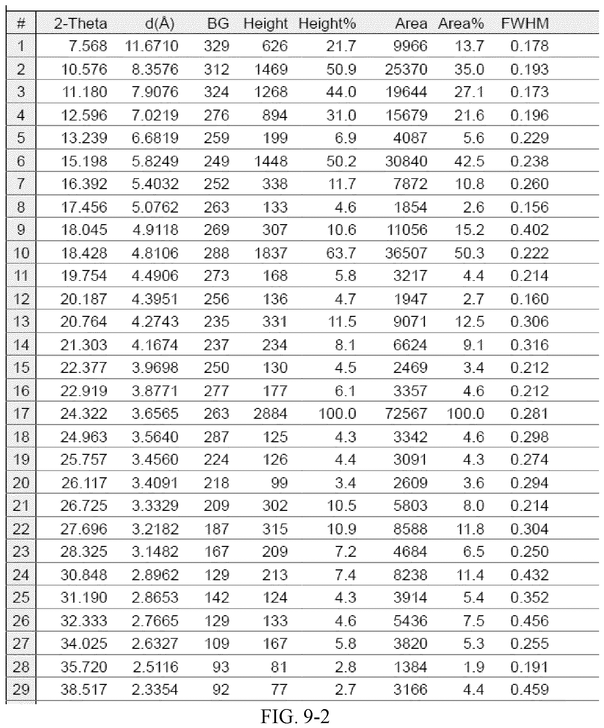

According to an embodiment of the present disclosure, by X-ray powder diffraction using Cu-Kα radiation, the crystal form I of oxalate has characteristic peaks at 2θ angles as shown in FIG. 9-2, with an error range of ±0.2°.

According to an embodiment of the present disclosure, the crystal form I of oxalate is a solvate, such as an organic solvate, preferably a methanol solvate, an ethanol solvate, or an isopropanol solvate. For example, the amount of the organic solvent is 2.0%-5.0%, such as 3.9%.

According to an embodiment of the present disclosure, the crystal form I of oxalate has a weight loss of about 2%-6%, such as 4.1%, in a temperature range of 60-160 °C.

According to an embodiment of the present disclosure, the crystal form I of oxalate has a broad endothermic peak at a peak temperature of about 127±5 °C.

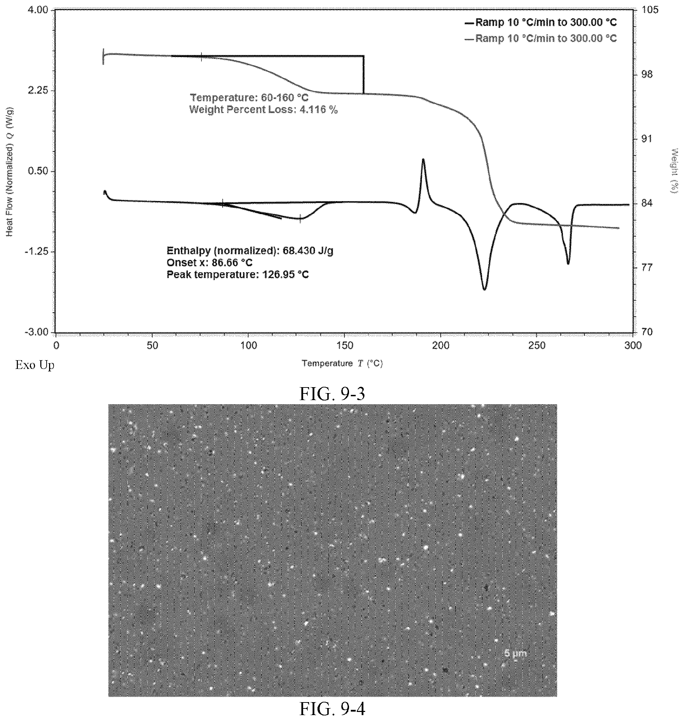

Preferably, the crystal form I of oxalate has a DSC-TGA profile substantially as depicted in FIG. 9-3.

According to an embodiment of the present disclosure, the crystal form I of oxalate is in the form of fine crystals. For example, in one embodiment, the crystal form I of oxalate has a polarizing microscope morphology substantially as shown in FIG. 9-4.

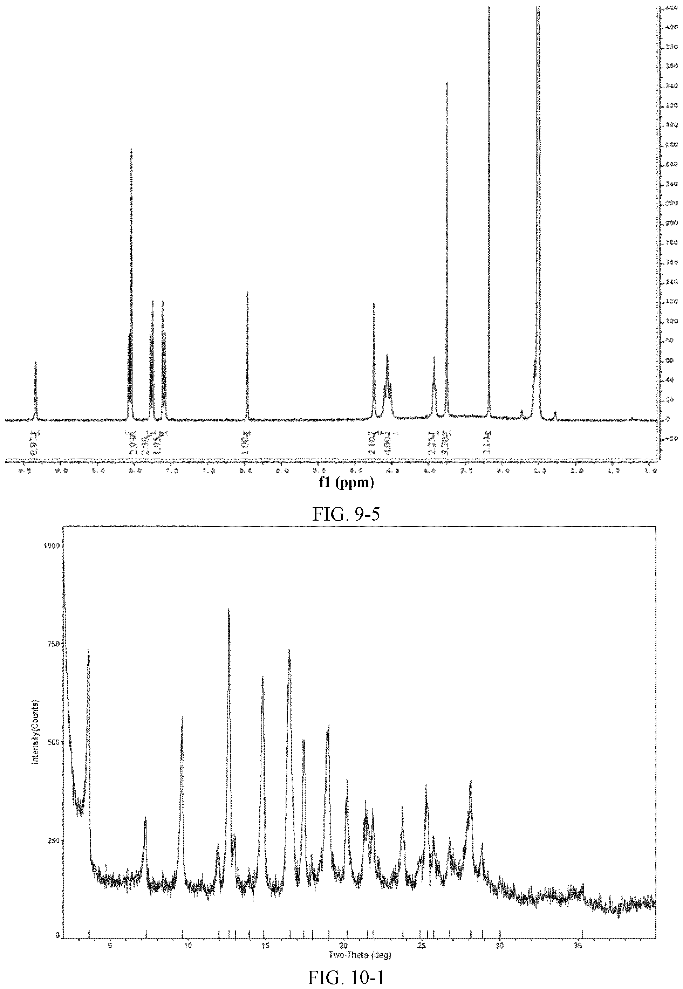

According to an embodiment of the present disclosure, the crystal form I of oxalate has a ¹H-NMR spectrum substantially as shown in FIG. 9-5.

According to an embodiment of the present disclosure, the crystal form of camphorsulfonate is a crystal form I of camphorsulfonate.

According to an embodiment of the present disclosure, by X-ray powder diffraction using Cu-Kα radiation, the crystal form I of camphorsulfonate has characteristic peaks at 2θ angles of 3.6±0.2°, 12.6±0.2°, and 16.5±0.2°, further has characteristic peaks at 2θ angles of 9.6±0.2°, 14.8±0.2°, 17.4±0.2°, and 19.0±0.2°, and still further has characteristic peaks at 2θ angles of 7.3±0.2°, 20.2±0.2°, 21.5±0.2°, 21.8±0.2°, 23.8±0.2°, 25.4±0.2°, and 28.1±0.2°.

According to an embodiment of the present disclosure, the crystal form I of camphorsulfonate has an X-ray powder diffraction pattern substantially as shown in FIG. 10-1.

According to an embodiment of the present disclosure, the crystal form I of camphorsulfonate has characteristic peaks at 2θ angles as shown in Table 1 by X-ray powder diffraction, with an error range of ±0.2°:

**Table 1**

| **2θ/°** | | | | | | | | | |
|---|---|---|---|---|---|---|---|---|---|
| 3.648 | 7.299 | 9.636 | 11.956 | 12.619 | 13.020 | 13.943 | 14.835 | 16.530 | 17.411 |
| 17.983 | 19.037 | 20.221 | 21.492 | 21.848 | 23.765 | 24.853 | 25.353 | 25.737 | 26.795 |
| 28.111 | 28.881 | 30.020 | 35.253 | | | | | | |

. preferably, the crystal form I of camphorsulfonate has characteristic peaks at 2θ angles as shown in Table 1' by X-ray powder diffraction, with an error range of ±0.2°.

According to an embodiment of the present disclosure, the crystal form I of camphorsulfonate is a hydrate, such as a monohydrate, a dihydrate, or a trihydrate, preferably a dihydrate.

According to an embodiment of the present disclosure, the crystal form I of camphorsulfonate has a weight loss of about 5% in a temperature range of from room temperature to 180± 2 °C.

According to an embodiment of the present disclosure, the crystal form I of camphorsulfonate has two endothermic peaks with peak temperatures of about 83±5 °C and 198±5 °C, such as 83±2 °C and 198±2 °C, respectively.

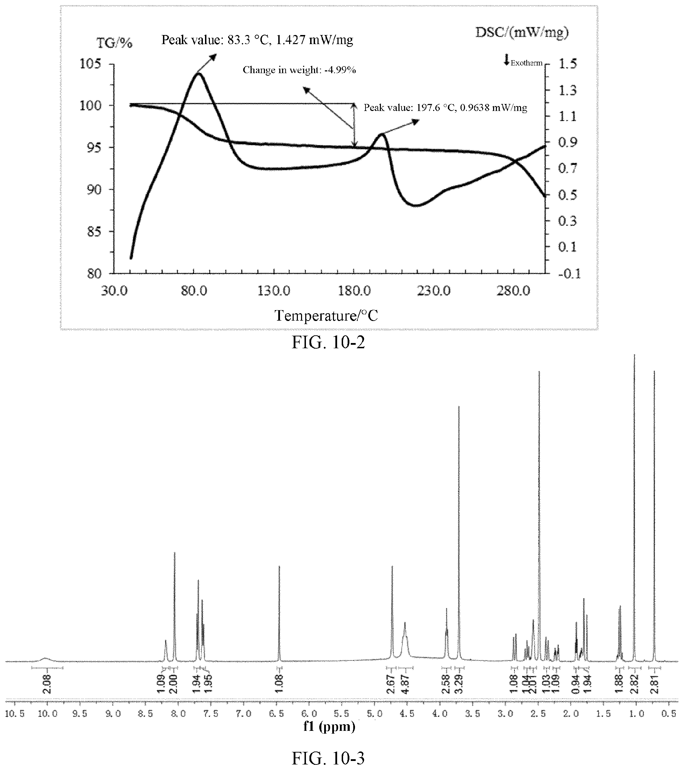

Preferably, the crystal form I of camphorsulfonate has a DSC-TGA profile substantially as depicted in FIG. 10-2.

According to an embodiment of the present disclosure, the crystal form I of camphorsulfonate contains no residual organic solvent.

According to an embodiment of the present disclosure, the crystal form I of camphorsulfonate has a ¹H-NMR spectrum substantially as shown in FIG. 10-3.

According to an embodiment of the present disclosure, the crystal form of 2-hydroxyethanesulfonate is a crystal form I of 2-hydroxyethanesulfonate.

According to an embodiment of the present disclosure, by X-ray powder diffraction using Cu-Kα radiation, the crystal form I of 2-hydroxyethanesulfonate has characteristic peaks at 2θ angles of 16.0±0.2° and 25.4±0.2°, further has characteristic peaks at 2θ angles of 18.0±0.2° and 18.8±0.2°, still further has characteristic peaks at 2θ angles of 8.5±0.2°, 8.9±0.2°, and 13.2±0.2°, and yet still further has characteristic peaks at 2θ angles of 15.4±0.2°, 29.7±9.2°, 22.3±9.2°, 22.6±9.2°, 24.7±9.2°, and 26.1±0.2°.

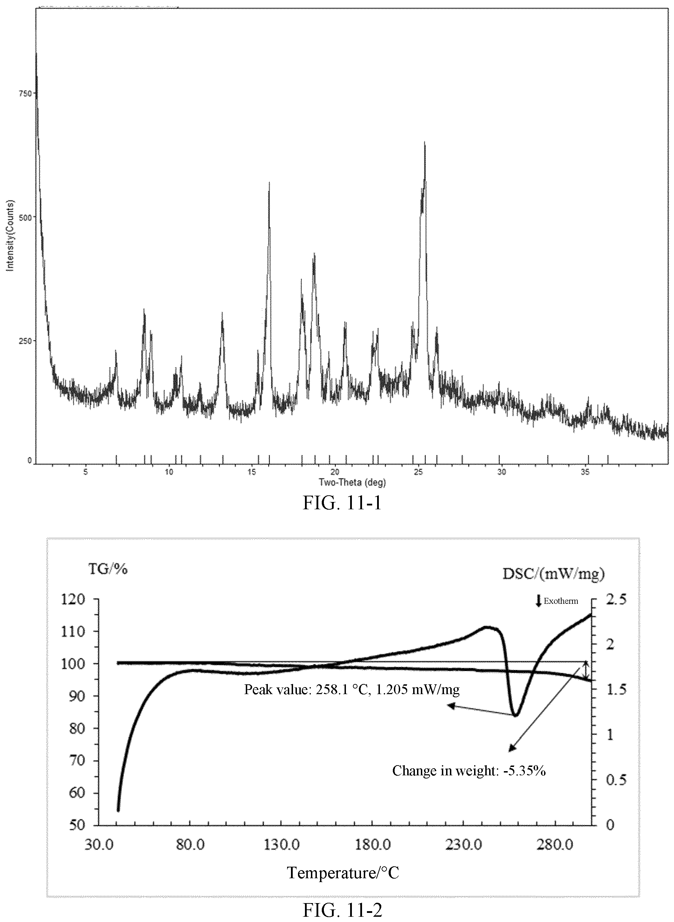

According to an embodiment of the present disclosure, the crystal form I of 2-hydroxyethanesulfonate has an X-ray powder diffraction pattern substantially as shown in FIG. 11-1.

According to one embodiment of the present disclosure, the crystal form I of 2-hydroxyethanesulfonate has characteristic peaks at 2θ angles as shown in Table 2 by X-ray powder diffraction, with an error range of ±0.2°:

**Table 2**

| **2θ/°** | | | | | | | | | |
|---|---|---|---|---|---|---|---|---|---|
| 6.828 | 8.548 | 8.914 | 10.410 | 10.751 | 11.875 | 13.220 | 15.362 | 16.042 | 17.972 |
| 18.750 | 19.626 | 20.650 | 22.266 | 22.555 | 24.657 | 25.367 | 26.088 | 27.628 | 29.833 |
| 32.735 | 35.205 | 36.359 | | | | | | | |

. preferably, the crystal form I of 2-hydroxyethanesulfonate has characteristic peaks at 2θ angles as shown in Table 2' by X-ray powder diffraction, with an error range of ±0.2°.

According to an embodiment of the present disclosure, the crystal form I of 2-hydroxyethanesulfonate is an anhydrate.

According to an embodiment of the present disclosure, the crystal form I of 2-hydroxyethanesulfonate has a weight loss of 0-0.5%, for example, almost no weight loss or a weight loss of 0.18%±0.02%, in a temperature range of from room temperature to 150±2 °C, and has a weight loss of 5.4%±0.2%, such as 5.4%, in a temperature range of from room temperature to 300±2 °C.

According to an embodiment of the present disclosure, the crystal form I of 2-hydroxyethanesulfonate has an exothermic peak at a peak temperature of about 258±5 °C.

Preferably, the crystal form I of 2-hydroxyethanesulfonate has a DSC-TGA profile substantially as depicted in FIG. 11-2.

According to an embodiment of the present disclosure, the crystal form I of 2-hydroxyethanesulfonate contains no residual organic solvent.

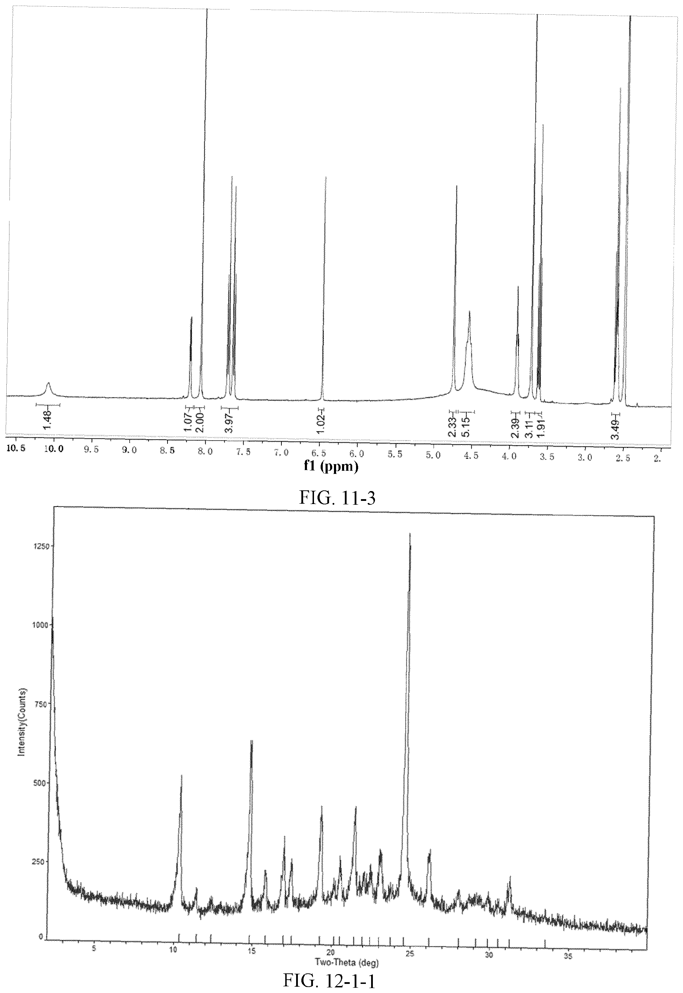

According to an embodiment of the present disclosure, the crystal form I of 2-hydroxyethanesulfonate has a ¹H-NMR spectrum substantially as shown in FIG. 11-3.

According to an embodiment of the present disclosure, the crystal form of ethanesulfonate comprises a crystal form I of ethanesulfonate and a crystal form II of ethanesulfonate.

According to an embodiment of the present disclosure, by X-ray powder diffraction using Cu-Kα radiation, the crystal form I of ethanesulfonate has a characteristic peak at a 2θ angle of 24.6±0.2°, further has characteristic peaks at 2θ angles of 10.4±0.2° and 14.8±0.2°, still further has characteristic peaks at 2θ angles of 16.9±0.2°, 19.3±0.2°, and 21.4±0.2°, and yet still further has characteristic peaks at 2θ angles of 15.8±0.2°, 17.4±0.2°, 20.5±0.2°, 23.0±0.2°, and 26.2±0.2°.

According to an embodiment of the present disclosure, the crystal form I of ethanesulfonate has an X-ray powder diffraction pattern substantially as shown in FIG. 12-1-1 or FIG. 12-1-2.

According to one embodiment of the present disclosure, the crystal form I of ethanesulfonate has characteristic peaks at 2θ angles as shown in Table 4 by X-ray powder diffraction, with an error range of ±0.2°:

**Table 4**

| **2θ/°** | | | | | | | | | |
|---|---|---|---|---|---|---|---|---|---|
| 10.383 | 11.436 | 12.372 | 14.810 | 15.794 | 16.936 | 17.448 | 19.249 | 20.143 | 20.510 |
| 21.427 | 21.969 | 22.447 | 23.005 | 23.722 | 24.580 | 26.180 | 28.034 | 29.150 | 29.879 |
| 30.524 | 31.274 | 33.520 | | | | | | | |

. preferably, the crystal form I of ethanesulfonate has characteristic peaks at 2θ angles as shown in Table 4' by X-ray powder diffraction, with an error range of ±0.2°.

According to another embodiment of the present disclosure, the crystal form I of ethanesulfonate has characteristic peaks at 2θ angles as shown in Table 5 by X-ray powder diffraction, with an error range of ±0.2°:

**Table 5**

| **2θ/°** | | | | | | | | | |
|---|---|---|---|---|---|---|---|---|---|
| 10.435 | 11.498 | 12.421 | 14.836 | 15.44 | 15.822 | 16.989 | 17.463 | 18.055 | 19.327 |
| 20.154 | 20.61 | 21.479 | 22.031 | 22.54 | 23.132 | 23.726 | 24.644 | 26.168 | 28.097 |
| 28.796 | 29.924 | 30.543 | 31.275 | 33.637 | 37.431 | | | | |

. preferably, the crystal form I of ethanesulfonate has characteristic peaks at 2θ angles as shown in Table 6' by X-ray powder diffraction, with an error range of ±0.2°.

According to an embodiment of the present disclosure, the crystal form I of ethanesulfonate is an anhydrate.

According to an embodiment of the present disclosure, the crystal form I of ethanesulfonate has a weight loss of about 1.4%±0.4% before 230±2 °C, and preferably has a weight loss of 1.4%±0.2% before 230±2 °C.

According to an embodiment of the present disclosure, the crystal form I of ethanesulfonate has an exothermic peak at a peak temperature of about 265±5 °C, such as 265±2 °C or 268±2 °C. According to an embodiment of the present disclosure, the crystal form I of ethanesulfonate has an endothermic peak at a peak temperature of about 260±5 °C, such as 259±2 °C or 263±2 °C.

The peak temperatures of the exothermic peak and the endothermic peak of the crystal form I of ethanesulfonate described above are different, preferably with the peak temperature of the endothermic peak being lower than that of the exothermic peak.

Preferably, the crystal form I of ethanesulfonate has a DSC-TGA profile substantially as depicted in FIG. 12-2-1 or FIG. 12-2-2.

According to an embodiment of the present disclosure, the crystal form I of ethanesulfonate contains no residual organic solvent, or the crystal form I of ethanesulfonate contains a residual organic solvent. For example, the content of the organic solvent is 0.1%-2.5%, such as 0.9%-1.6%, illustratively 1.3%. For example, the organic solvent is selected from one, two, or more of methanol, ethanol, isopropanol, acetonitrile, tetrahydrofuran, butanone, acetone, 2-methyltetrahydrofuran, isopropyl acetate, ethyl acetate, methyl *tert*-butyl ether, and dimethylsulfoxide, and is preferably methanol, isopropanol, ethanol, acetonitrile, or butanone. Illustratively, the crystal form I of ethanesulfonate contains about 1.3% ethanol.

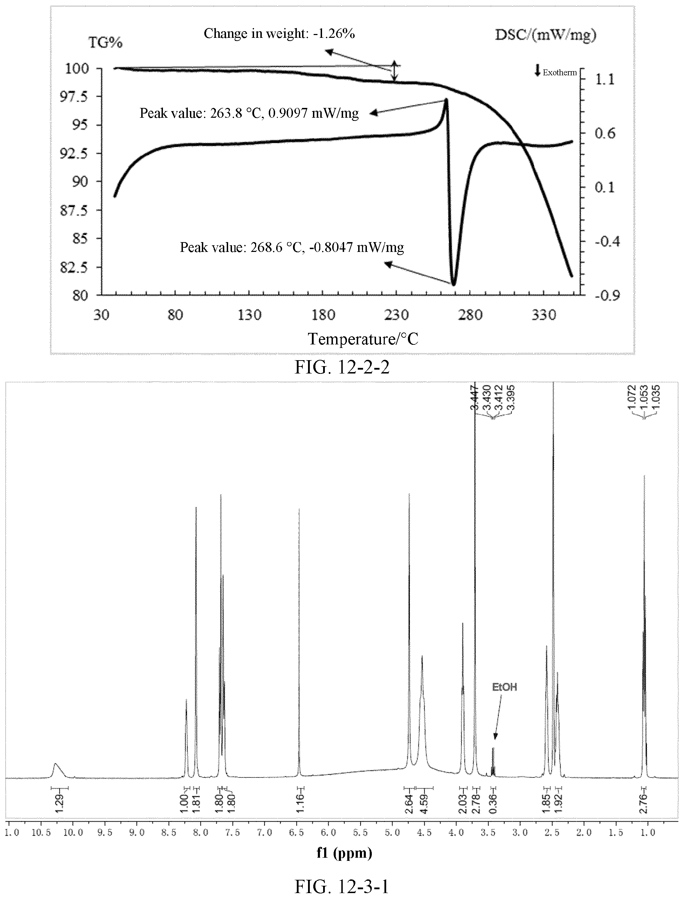

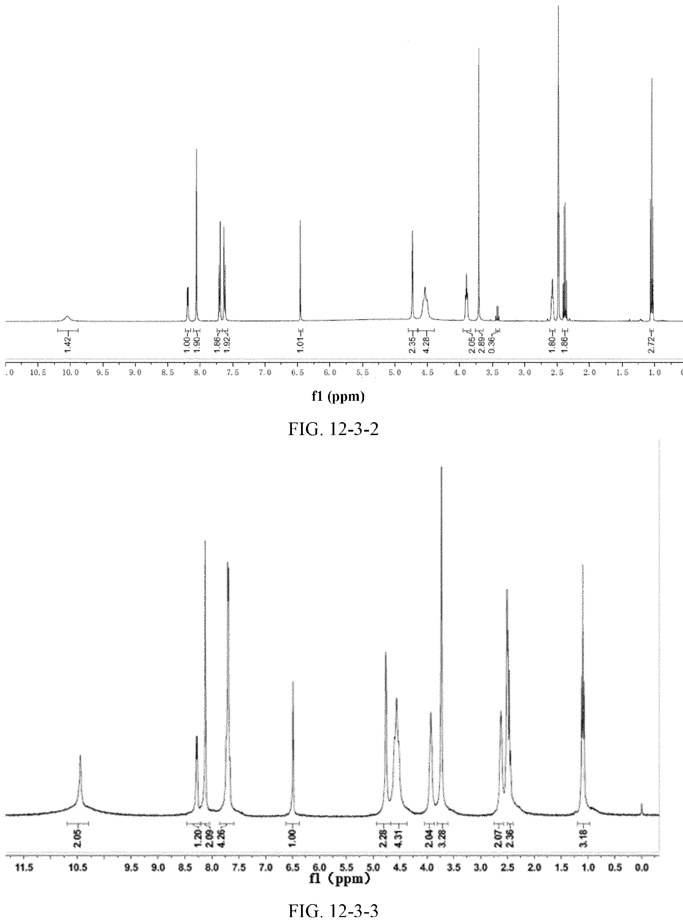

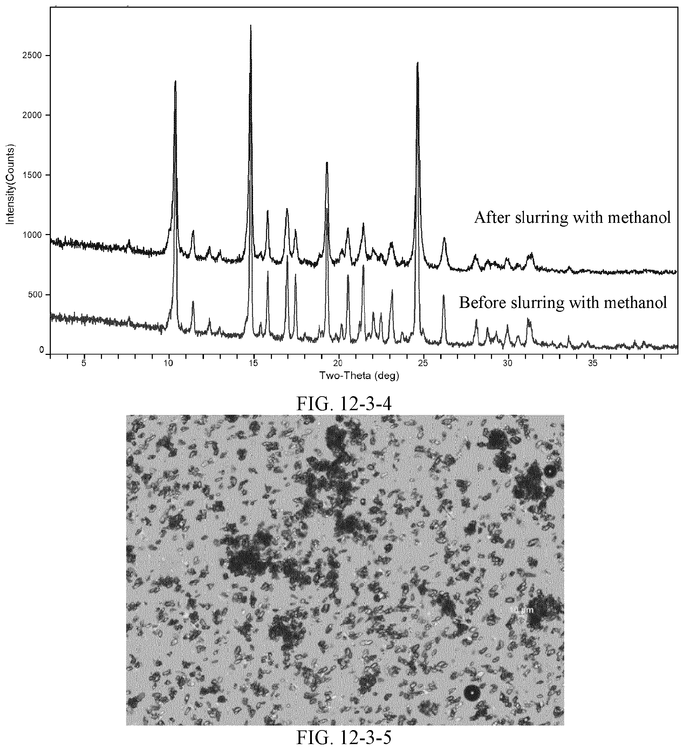

According to an embodiment of the present disclosure, the crystal form I of ethanesulfonate is in the form of fine crystals. For example, in one embodiment, the crystal form I of ethanesulfonate has a polarizing microscope morphology substantially as shown in FIG. 12-3-5.

According to an embodiment of the present disclosure, the crystal form I of ethanesulfonate has a ¹H-NMR spectrum substantially as shown in FIG. 12-3-1 or 12-3-2 or 12-3-3.

According to an embodiment of the present disclosure, by X-ray powder diffraction using Cu-Kα radiation, the crystal form II of ethanesulfonate has characteristic peaks at 2θ angles of 10.6±0.2° and 17.5±0.2°, further has characteristic peaks at 2θ angles of 4.5±9.2°, 9.0±0.2°, and 19.2±0.2°, and still further has characteristic peaks at 2θ angles of 13.5±0.2°, 14.7±0.2°, 16.2±0.2°, 18.0±0.2°, 22.7±0.2°, 25.2±0.2°, and 26.9±0.2°.

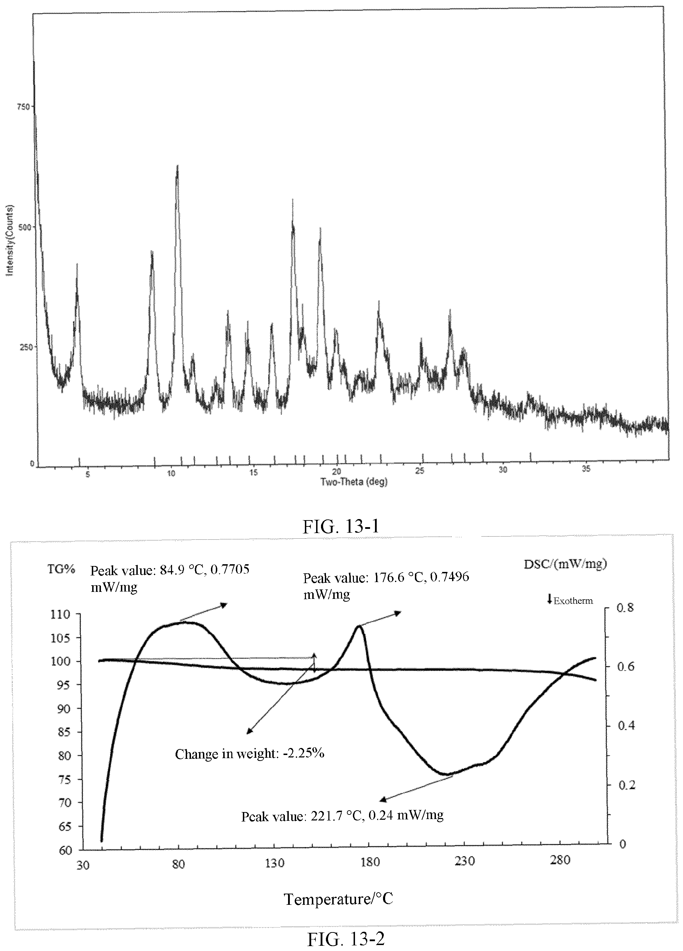

According to an embodiment of the present disclosure, the crystal form II of ethanesulfonate has an X-ray powder diffraction pattern substantially as shown in FIG. 13-1.

According to an embodiment of the present disclosure, the crystal form II of ethanesulfonate has characteristic peaks at 2θ angles as shown in Table 6 by X-ray powder diffraction, with an error range of ±0.2°:

**Table 6**

| **2θ/°** | | | | | | | | | |
|---|---|---|---|---|---|---|---|---|---|
| 4.489 | 9.007 | 10.634 | 11.419 | 12.771 | 13.549 | 14.732 | 16.226 | 17.528 | 18.051 |
| 19.181 | 20.049 | 20.507 | 21.48 | 22.66 | 25.17 | 26.929 | 27.706 | 28.776 | 31.642 |

. preferably, the crystal form II of ethanesulfonate has characteristic peaks at 2θ angles as shown in Table 5' by X-ray powder diffraction, with an error range of ±0.2°.

According to an embodiment of the present disclosure, the crystal form II of ethanesulfonate is a solvate, such as an organic solvate or a hydrate. For example, the organic solvent is selected from one, two, or more of ethanol, acetonitrile, tetrahydrofuran, butanone, acetone, 2-methyltetrahydrofuran, isopropyl acetate, ethyl acetate, and methyl *tert*-butyl ether. For example, the content of the organic solvent is 1.5%-3.5%, such as 2.3%. Illustratively, the crystal form II of ethanesulfonate contains about 2.3% isopropyl acetate.

For example, the hydrate is a monohydrate, a 0.5-hydrate, or a dihydrate, preferably a monohydrate. According to an embodiment of the present disclosure, the crystal form II of ethanesulfonate has a weight loss of about 2.3%±0.2% before 150±2 °C.

According to an embodiment of the present disclosure, the crystal form II of ethanesulfonate has an endothermic peak at a peak temperature of about 85±5 °C, an endothermic peak at a peak temperature of about 177±5 °C, and an exothermic peak at a peak temperature of about 222±5 °C. For example, the crystal form II of ethanesulfonate has an endothermic peak at a peak temperature of about 85±2 °C, an endothermic peak at a peak temperature of about 177±2 °C, and an exothermic peak at a peak temperature of about 222±2 °C.

Preferably, the crystal form II of ethanesulfonate has a DSC-TGA profile substantially as depicted in FIG. 13-2.

According to an embodiment of the present disclosure, the crystal form of tartrate comprises a crystal form of L-tartrate and a crystal form of D-tartrate, preferably a crystal form I of L-tartrate.

According to an embodiment of the present disclosure, by X-ray powder diffraction using Cu-Kα radiation, the crystal form I of L-tartrate has characteristic peaks at 2θ angles of 11.6±0.2°, 15.5±0.2°, and 20.8±0.2°, further has characteristic peaks at 2θ angles of 9.2±0.2°, 18.7±0.2°, and 21.1±0.2°, and still further has characteristic peaks at 2θ angles of 7.2±0.2°, 7.7±0.2°, 12.1±0.2°, 14.5±0.2°, 19.4±0.2°, and 22.6±0.2°.

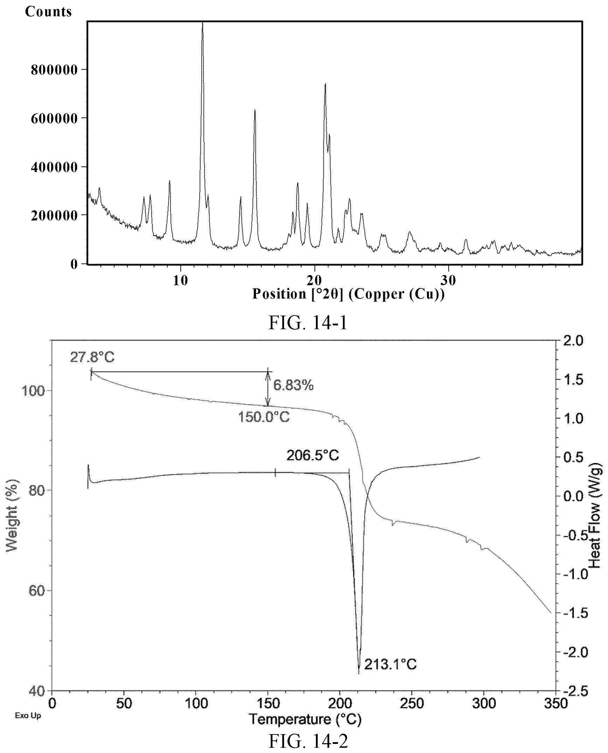

According to an embodiment of the present disclosure, the crystal form I of L-tartrate has an X-ray powder diffraction pattern substantially as shown in FIG. 14-1.

According to an embodiment of the present disclosure, the crystal form I of L-tartrate has characteristic peaks at 2θ angles as shown in Table 11-3 by X-ray powder diffraction, with an error range of ±0.2°.

According to an embodiment of the present disclosure, the crystal form I of L-tartrate is an anhydrate. According to an embodiment of the present disclosure, the crystal form I of L-tartrate has a weight loss of about 2%-10%, such as a weight loss of 3.6%±0.2% or 6.8%±0.2%, in a temperature range of from room temperature to 150±2 °C.

According to an embodiment of the present disclosure, the crystal form I of L-tartrate has an endothermic peak at a peak temperature of about 213±5 °C.

Preferably, the crystal form I of L-tartrate has a DSC-TGA profile substantially as shown in FIG. 14-2.

According to an embodiment of the present disclosure, the crystal form I of L-tartrate contains a residual organic solvent. For example, the content of the organic solvent is 0.3%-1.5%, such as 0.7%. Illustratively, the crystal form I of L-tartrate contains 0.7% butanone or ethyl acetate.

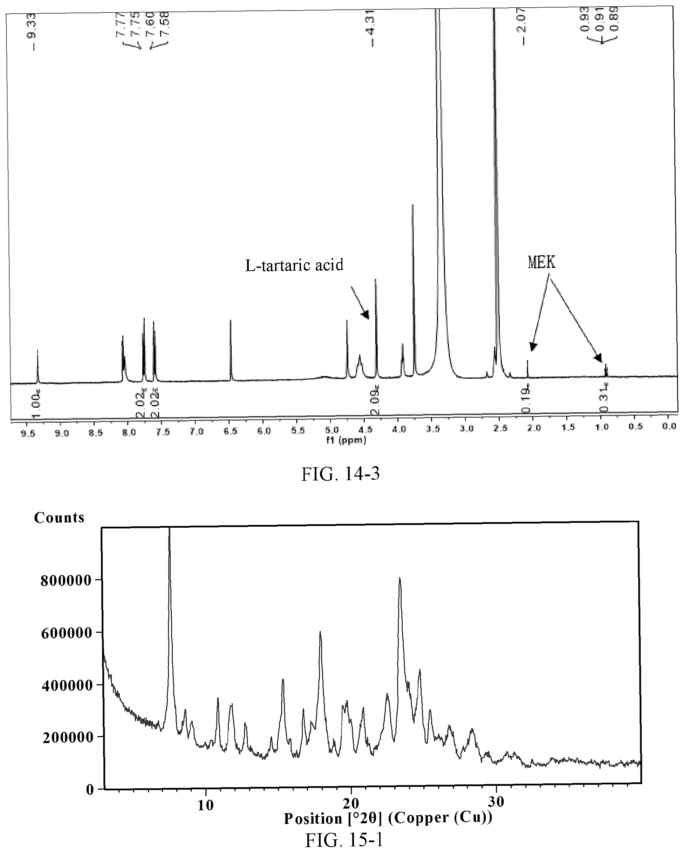

According to an embodiment of the present disclosure, the crystal form I of L-tartrate has a ¹H-NMR spectrum substantially as shown in FIG. 14-3.

According to an embodiment of the present disclosure, the crystal form of 1,5-naphthalenedisulfonate comprises a crystal form I of 1,5-naphthalenedisulfonate and a crystal form II of 1,5-naphthalenedisulfonate.

According to an embodiment of the present disclosure, by X-ray powder diffraction using Cu-Kα radiation, the crystal form I of 1,5-naphthalenedisulfonate has characteristic peaks at 2θ angles of 7.7±0.2°, 18.0±0.2°, and 23.5±0.2°, further has characteristic peaks at 2θ angles of 15.4±0.2°, 24.0±0.2°, and 24.8±0.2°, and still further has characteristic peaks at 2θ angles of 8.0±0.2°, 8.6±0.2°, 10.8±0.2°, 11.8±0.2°, 16.8±0.2°, 17.7±0.2°, 19.8±0.2°, and 22.5±0.2°; or the crystal form also has characteristic peaks at 2θ angles of 8.0±0.2°, 8.6±0.2°, 10.9±0.2°, 11.9±0.2°, 16.8±0.2°, 17.7±0.2°, 19.8±0.2°, and 22.6±0.2°.

According to an embodiment of the present disclosure, the crystal form I of 1,5-naphthalenedisulfonate has an X-ray powder diffraction pattern substantially as shown in FIG. 15-1. According to an embodiment of the present disclosure, the crystal form I of 1,5-naphthalenedisulfonate has characteristic peaks at 2θ angles as shown in Table 12-3 by X-ray powder diffraction, with an error range of ±0.2°.

According to an embodiment of the present disclosure, the crystal form I of 1,5-naphthalenedisulfonate is a hydrate, such as a monohydrate, a dihydrate, a trihydrate, or a tetrahydrate, preferably a tetrahydrate.

According to an embodiment of the present disclosure, the crystal form I of 1,5-naphthalenedisulfonate has a weight loss of about 2%-10%, such as a weight loss of 8.3%±0.2%, in a temperature range of from room temperature to 150±2 °C.

According to an embodiment of the present disclosure, the crystal form I of 1,5-naphthalenedisulfonate has two endothermic peaks at peak temperatures of about 84±5 °C and 119±5 °C, such as about 84.0±2 °C and 119.6±2 °C, respectively.

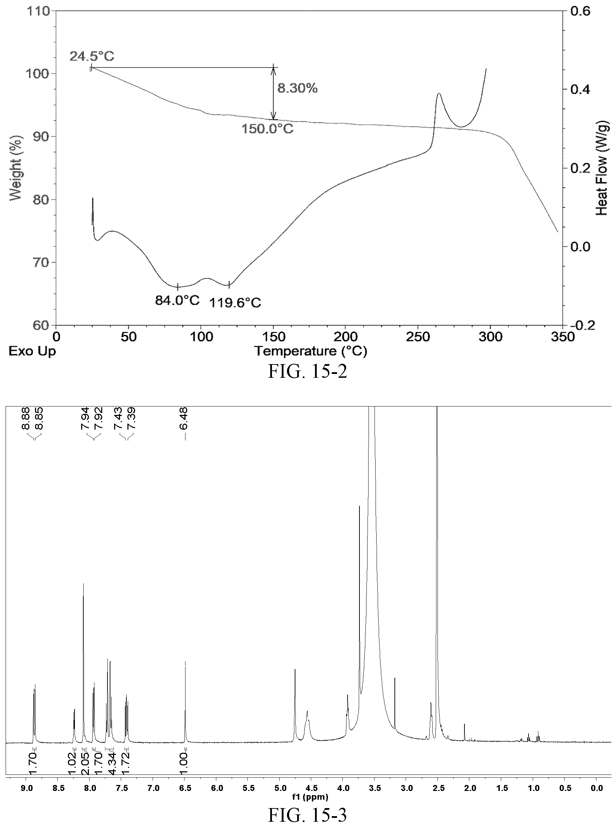

Preferably, the crystal form I of 1,5-naphthalenedisulfonate has a DSC-TGA profile substantially as depicted in FIG. 15-2.

According to an embodiment of the present disclosure, the crystal form I of 1,5-naphthalenedisulfonate contains no residual organic solvent.

According to an embodiment of the present disclosure, the crystal form I of 1,5-naphthalenedisulfonate has a ¹H-NMR spectrum substantially as shown in FIG. 15-3.

According to an embodiment of the present disclosure, by X-ray powder diffraction using Cu-Kα radiation, the crystal form II of 1,5-naphthalenedisulfonate has characteristic peaks at 2θ angles of 25.5±0.2° and 10.2±0.2°, further has characteristic peaks at 2θ angles of 3.1±0.2°, 3.9±0.2°, 8.6±0.2°, 12.7±0.2°, and 13.9±0.2°, and still further has characteristic peaks at 2θ angles of 8.4±0.2°, 11.4±0.2°, 14.4±0.2°, 15.6±0.2°, 20.5±0.2°, 20.9±0.2°, 22.0±0.2°, and 23.8±0.2°.

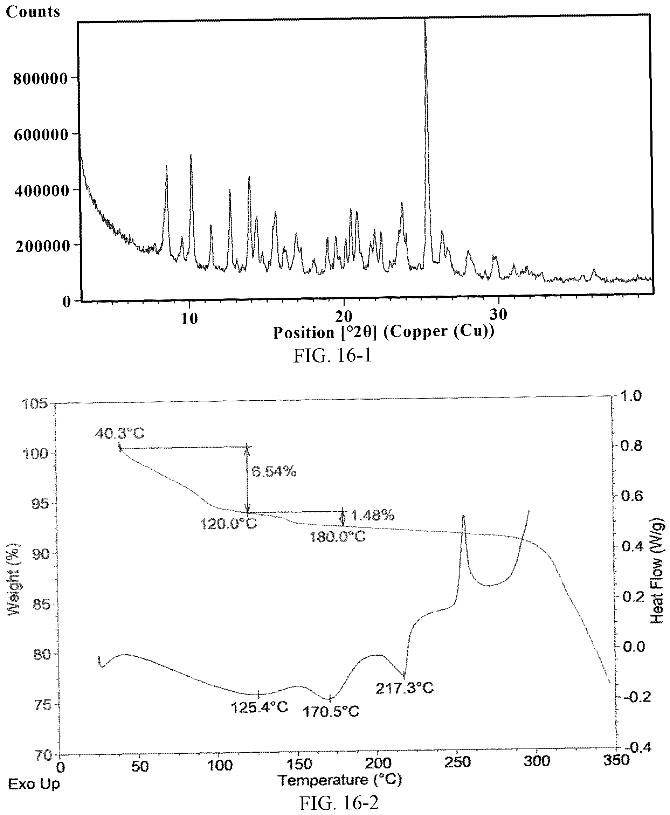

According to an embodiment of the present disclosure, the crystal form II of 1,5-naphthalenedisulfonate has an X-ray powder diffraction pattern substantially as shown in FIG. 16-1. According to an embodiment of the present disclosure, the crystal form II of 1,5-naphthalenedisulfonate has characteristic peaks at 2θ angles as shown in Table 12-4 by X-ray powder diffraction, with an error range of ±0.2°.

According to an embodiment of the present disclosure, the crystal form II of 1,5-naphthalenedisulfonate is a hydrate, such as a monohydrate, a dihydrate, a trihydrate, or a tetrahydrate, preferably a trihydrate.

According to an embodiment of the present disclosure, the crystal form II of 1,5-naphthalenedisulfonate has a weight loss of about 2%-12%, such as a weight loss of 8.0%±0.2%, in a temperature range of from room temperature to 180±2 °C.

According to an embodiment of the present disclosure, the crystal form II of 1,5-naphthalenedisulfonate has three endothermic peaks with peak temperatures of about 125±5 °C, 170±5 °C and 217±5 °C, such as about 125.4±2 °C, 170.5±2 °C and 217.3±2 °C, respectively. Preferably, the crystal form II of 1,5-naphthalenedisulfonate has a DSC-TGA profile substantially as depicted in FIG 16-2.

According to an embodiment of the present disclosure, the crystal form II of 1,5-naphthalenedisulfonate contains a residual organic solvent. For example, the content of the organic solvent is 0.2%-1.0% by mass, such as 0.6% by mass. Illustratively, the crystal form II of 1,5-naphthalenedisulfonate contains butanone.

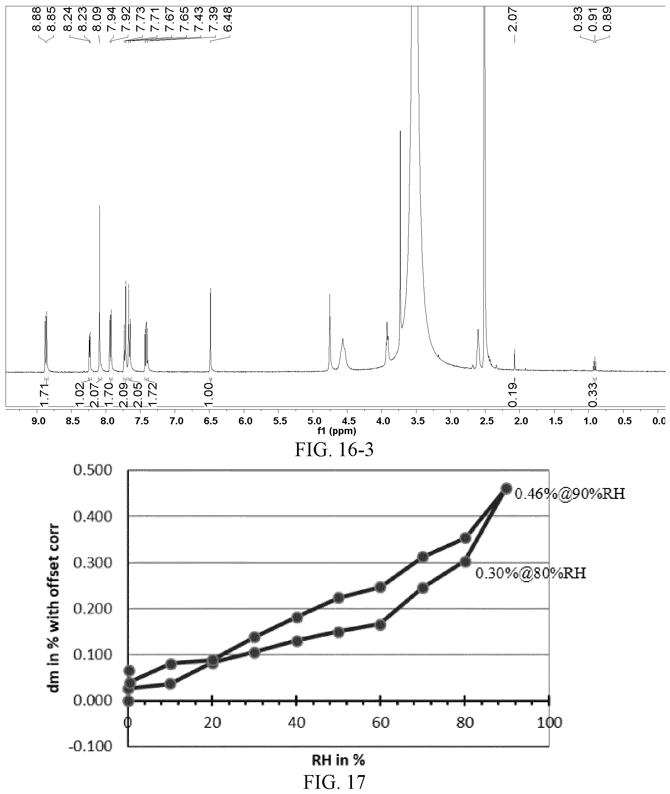

According to an embodiment of the present disclosure, the crystal form II of 1,5-naphthalenedisulfonate has a ¹H-NMR spectrum substantially as shown in FIG. 16-3.

The present disclosure further provides a preparation method for the salt of compound I or the crystal form of the salt described above, comprising mixing and reacting compound I with an acid to give the salt of compound I or the crystal form of the salt.

According to an embodiment of the present disclosure, the acid is defined as described above. According to an embodiment of the present disclosure, compound I and the acid are in a molar ratio as described above.

According to an embodiment of the present disclosure, the reaction is performed in a solvent 2.

For example, the solvent 2 is selected from one, two, or more of an organic solvent 2, water, and a mixed solvent of the organic solvent 2 and water.

According to an embodiment of the present disclosure, the organic solvent 2 is selected from one, two, or more of methanol, ethanol, isopropanol, butanol, acetone, butanone, ethyl acetate, isopropyl acetate, methyl *tert*-butyl ether, dichloromethane, acetonitrile, tetrahydrofuran, *n*-heptane, dimethylsulfoxide, 2-methyltetrahydrofuran, and chloroform.

Preferably, the organic solvent 2 is selected from one, two, or more of methanol, ethanol, acetonitrile, tetrahydrofuran, isopropanol, acetone, butanol (comprising *n*-butanol or isobutanol), butanone, isopropyl acetate, ethyl acetate, dimethylsulfoxide, and 2-methyltetrahydrofuran.

According to an embodiment of the present disclosure, the solvent for preparing the hydrochloride or the crystal form of hydrochloride (e.g., the crystal form I of hydrochloride) of compound I is selected from one, two or more (e.g., three) of methanol, ethanol, isopropanol, acetonitrile, butanone, tetrahydrofuran, and 2-methyltetrahydrofuran, preferably one, two, or three of methanol, ethanol, and isopropanol.

According to an embodiment of the present disclosure, the solvent for preparing the sulfate or the crystal form of sulfate (e.g., the crystal form I of sulfate) of compound I is selected from one, two, or more of methanol, ethanol, isopropanol, acetonitrile, and butanone, preferably one, two, or three of methanol, ethanol, and isopropanol.

According to an embodiment of the present disclosure, the solvent for preparing the *p-*toluenesulfonate or the crystal form of *p*-toluenesulfonate (e.g., the crystal form I of *p-*toluenesulfonate) of compound I is selected from one, two or more (e.g., three) of methanol, ethanol, isopropanol, acetonitrile, butanone, tetrahydrofuran, and 2-methyltetrahydrofuran, preferably one, two, or three of methanol, ethanol, and isopropanol.

According to an embodiment of the present disclosure, the solvent for preparing the benzenesulfonate or the crystal form of benzenesulfonate (e.g., the crystal form I of benzenesulfonate) of compound I is selected from one, two or more (e.g., three) of methanol, ethanol, isopropanol, acetonitrile, tetrahydrofuran, and 2-methyltetrahydrofuran, preferably one, two, or three of methanol, ethanol, and isopropanol.

According to an embodiment of the present disclosure, the solvent for preparing the maleate or the crystal form of maleate of compound I is selected from one, two, or more of methanol, ethanol, acetonitrile, tetrahydrofuran, isopropanol, acetone, butanol, butanone, and isopropyl acetate. Illustratively, the solvent for preparing the crystal form I of maleate is one, two, or three of methanol, ethanol, and isopropanol;
illustratively, the solvent for preparing the crystal form II of maleate is one, two, or three of acetonitrile, tetrahydrofuran, isopropanol, isopropyl acetate, and butanone;
illustratively, the solvent for preparing the crystal form III of maleate is one, two, or three of acetone, isopropyl acetate, butanol, acetonitrile, and butanone.

According to an embodiment of the present disclosure, the solvent for preparing the oxalate or the crystal form of oxalate (e.g., the crystal form I of oxalate) of compound I is one, two, or three of methanol, ethanol, and isopropanol.

According to an embodiment of the present disclosure, the solvent for preparing the camphorsulfonate or the crystal form of camphorsulfonate (e.g., the crystal form I of camphorsulfonate) of compound I is selected from one, two, or more of methanol, isopropanol, ethanol, acetonitrile, tetrahydrofuran, butanone, isopropyl acetate, and ethyl acetate.

According to an embodiment of the present disclosure, the solvent for preparing the 2-hydroxyethanesulfonate or the crystal form of 2-hydroxyethanesulfonate (e.g., the crystal form I of 2-hydroxyethanesulfonate) of compound I is selected from one, two, or more of ethanol, acetonitrile, tetrahydrofuran, butanone, and isopropyl acetate, preferably one, two, or three of ethanol, methanol, and isopropanol.

According to an embodiment of the present disclosure, the solvent for preparing the ethanesulfonate or the crystal form of ethanesulfonate of compound I is selected from one, two, or more of ethanol, acetonitrile, tetrahydrofuran, butanone, and isopropyl acetate.

Illustratively, the solvent for preparing the crystal form I of ethanesulfonate is selected from one, two, or more of dimethylsulfoxide, methanol, isopropanol, ethyl acetate, isopropyl acetate, ethanol, acetonitrile, tetrahydrofuran, butanone, acetone, and dichloromethane; preferably, the solvent is dimethylsulfoxide, methanol, ethanol, isopropanol, acetonitrile, butanone, or a mixed solvent thereof. Illustratively, the solvent for preparing the crystal form II of ethanesulfonate is selected from isopropyl acetate.

According to an embodiment of the present disclosure, the solvent for preparing the L-tartrate salt or the crystal form of L-tartrate (e.g., the crystal form I of L-tartrate) of compound I is selected from one, two, or three of butanone, tetrahydrofuran, and ethyl acetate.

According to an embodiment of the present disclosure, the solvent for preparing the 1,5-naphthalenedisulfonate or the crystal form of 1,5-naphthalenedisulfonate of compound I is selected from one, two, or more of methanol, ethanol, isopropanol, butanone, and water; for example, the solvent is methanol or a mixture of butanone and water.

Illustratively, the solvent for preparing the crystal form I of 1,5-naphthalenedisulfonate is one, two, or three of methanol, ethanol, and isopropanol;
illustratively, the solvent for preparing the crystal form II of 1,5-naphthalenedisulfonate is a mixture of butanone and water, preferably in a volume ratio of (10-25):1, such as 19:1.

According to an embodiment of the present disclosure, the mass-to-volume ratio of compound I to the solvent is (15-150) mg: 1 mL, such as (20-100) mg: 1 mL, illustratively 30 mg: 1.5 mL, 30 mg:0.6 mL, 20 mg:0.4 mL, 20 mg:0.5 mL, 250 mg:6 mL, 50 mg:0.5 mL, 50 mg:1 mL, 50 mg:1.75 mL, or 500 mg: 12 mL.

According to an embodiment of the present disclosure, the preparation method comprises: mixing and reacting a solution of compound I with a solution of the acid to give the salt of compound I or the crystal form of the salt; or mixing and reacting a solution of compound I with a solid of the acid to give the salt of compound I or the crystal form of the salt;

According to an embodiment of the present disclosure, the solvent in the solution of compound I and the solvent in the solution of the acid is identical.

According to an embodiment of the present disclosure, the preparation method comprises a step of ultrasonic mixing.

According to an embodiment of the present disclosure, the solution of the acid is added slowly to the solution of compound I.

According to an embodiment of the present disclosure, the reaction is performed at a temperature of 20-35 °C, such as a temperature of 21-30 °C.

According to an embodiment of the present disclosure, the reaction is performed under stirring or suspension slurring.

According to an embodiment of the present disclosure, the reaction is performed for a period of 10 hours to 10 days, such as a period of 15 hours to 6 days.

According to an embodiment of the present disclosure, the preparation method comprises steps of performing filtration to collect a solid product after the reaction is completed and drying the solid product.

The present disclosure further provides a pharmaceutical composition, comprising an active ingredient and optionally a pharmaceutically acceptable carrier, wherein the active ingredient is the salt of compound I, the crystal form of the salt, or the free base crystal form.

For example, the pharmaceutically acceptable carrier includes, but is not limited to, one, two, or more of an excipient, a lubricant, an adhesive, a disintegrant, a solvent, a dissolution aid, a suspending agent, an isotonic agent, a buffer, a preservative, an antioxidant, a colorant, a foaming agent, a flavoring agent (e.g., a sweetener or an acidulant), and the like.

For another example, the pharmaceutically acceptable carrier includes one, two, or more of a watersoluble polymer, an inorganic salt, and the like.

According to an embodiment of the present disclosure, the pharmaceutical composition may further comprise an additional active ingredient, such as an additional ROCK inhibitor.

The present disclosure further provides use of the salt of compound I, the crystal form of the salt, or the free base crystal form, or the pharmaceutical composition described above for the manufacturing of a preparation.

The present disclosure further provides a preparation, comprising the salt of compound I, the crystal form of the salt, or the free base crystal form described above.

According to an embodiment of the present disclosure, the preparation comprises the pharmaceutical composition described above.

According to an embodiment of the present disclosure, the preparation may be in the dosage form of a powder, a tablet (e.g., a coated tablet, a sustained-release or controlled-release tablet), a lozenge, a capsule (e.g., a soft capsule or a hard capsule), a granule, a pill, a dispersible powder, a suspension, a solution, an emulsion, an elixir, a syrup, an aerosol, a cream, an ointment, a gel, an injection, a lyophilized powder for injection, a suppository, or the like.

According to an embodiment of the present disclosure, the preparation may be applied in any one of the following modes: oral administration, buccal administration, sublingual administration, inhalation, topical application; intravenous, subcutaneous, acupoint or intramuscular injection by parenteral routes; and rectal administration.

According to an embodiment of the present disclosure, the preparation is a ROCK antagonist. Preferably, the ROCK antagonist is for use in the prevention and/or treatment of one or more diseases caused by high expression or excessive activation of ROCK. preferably, the disease is selected from cardiovascular and cerebrovascular diseases, neurological diseases, fibrosis diseases, ocular diseases, tumors, arterial thrombotic disorders, radiation damage, respiratory system diseases, autoimmune diseases, microbial infections, muscular dystrophy, and diseases related to impaired lymphatic drainage, including atherosclerosis, acute coronary syndrome, hypertension, cerebral vasospasm, cerebral ischemia, ischemic stroke, restenosis, heart disease, heart failure, myocardial hypertrophy, myocardial ischemia-reperfusion injury, diabetes, diabetic nephropathy, cancer, neuronal degeneration, nerve injury diseases, spinal cord injury, erectile dysfunction, platelet aggregation, leukocyte aggregation, glaucoma, ocular hypertension, asthma, osteoporosis, pulmonary fibrosis (e.g., idiopathic interstitial pulmonary fibrosis), hepatic fibrosis, renal fibrosis, COPD, renal dialysis, glomerulosclerosis, neuronal degeneration inflammation, fungal infections, bacterial infections, viral infections, Duchenne muscular dystrophy, fatty liver disease, and steatohepatitis.

The present disclosure further provides a method for preventing and/or treating a disease caused by high expression or excessive activation of ROCK, comprising administering to a subject a therapeutically effective amount of the salt of compound I, the crystal form of the salt, or the free base crystal form, the pharmaceutical composition, or the preparation.

### Description of Terms

The term "crystal form" refers to crystal forms that have identical chemical composition but different spatial arrangements of crystal-forming molecules and/or ions.

The term "amorphous form" refers to a solid form of molecules and/or ions that is not crystalline. An amorphous solid does not show a defined X-ray powder diffraction pattern with a clear maximum value.

The term "X-ray powder diffraction pattern substantially as shown in the figure" means that at least 50%, or at least 60%, or at least 70%, or at least 80%, or at least 90%, or at least 95%, or at least 99% of the main peaks shown in an X-ray powder diffraction pattern appear in the X-ray powder diffraction pattern. The main peak refers to a peak with a relative intensity greater than 10%, preferably greater than 20%, and more preferably greater than 30%, using the highest peak as a reference (the relative intensity of the highest peak is designated as 100%).

The terms "patient" and "subject" used herein have the same meaning and refer to a specific human or other warm-blooded mammals. The human described herein as a "subject" includes adults, infants, and children, and the warm-blooded mammals include, but are not limited to, non-human primates such as chimpanzees, other apes or monkeys, and other zoo animals, domestic animals or laboratory animals such as cats, pigs, dogs, cattle, sheep, mice, rats, guinea pigs, and the like. Preferably, the "subject" described herein is a human.

Those skilled in the art can determine appropriate amounts of any one of or a mixture (in any ratio) of any two of the salt, the crystal form of the salt and the free base crystal form, and various pharmaceutically acceptable carriers and/or additional active ingredients in the pharmaceutical composition according to conventional methods.

The term "therapeutically effective amount" refers to an amount of any one of or a mixture (in any ratio) of any two of the salt, the crystal form of the salt and the free base crystal form, the pharmaceutical composition, or the preparation described herein sufficient to effect the intended application (including but not limited to the treatment of the diseases defined above), which may be determined according to the methods known to eligible physicians in the art. Determination of a therapeutically effective dose is well within the capability of the clinician or researcher in the art and may vary depending on the following factors: the intended application (*in vitro* or *in vivo*)*,* or the subject and disease or condition being treated, such as the body weight, age and general health of the subject, severity of the disease or condition, route of administration, and other factors affecting efficacy, such as drug allergy history and the like. The specific administration dose will vary depending on the following factors: the selected particular compound or crystal form, the dosing regimen to be followed, whether to administer in combination with other compounds, the schedule of administration, the tissue to which administration is subjected, and the physical delivery system employed.

The term "room temperature" refers to a temperature of 20-25 °C.

It can be understood by those skilled in the art that the term "free base" or "free base of compound I" used herein refers to compound I (in its non-salt form).

### Beneficial Effects of Present Disclosure:

The inventors have surprisingly found that the free base crystal form, the salt, and the crystal form of the salt of compound I provided by the present disclosure have stable physical and chemical properties; the crystal form of the present disclosure is stable under high temperature or high humidity conditions.

Meanwhile, the free base crystal form, the salt and the crystal form of the salt of compound I provided by the present disclosure exhibit relatively good biological performance both *in vivo* and *in vitro,* which is more beneficial for drug quality control and druggability.

### BRIEF DESCRIPTION OF THE DRAWINGS

FIG. 1-1 shows an XRPD characterization of a free base crystal form I;
FIG. 1-2 shows an XRPD spectrum analysis of the free base crystal form I;
FIG. 1-3 shows a DSC-TGA pattern of the free base crystal form I;
FIG. 1-4 shows a ¹H-NMR spectrum of the free base crystal form I;
FIG. 2-1 shows an XRPD characterization of a crystal form I of hydrochloride;
FIG. 2-2 shows an XRPD spectrum analysis of the crystal form I of hydrochloride;
FIG. 2-3 shows a DSC-TGA pattern of the crystal form I of hydrochloride;
FIG. 2-4 shows a polarizing micrograph of the crystal form I of hydrochloride;
FIG. 2-5 shows a ¹H-NMR spectrum of the crystal form I of hydrochloride;
FIG. 3-1 shows an XRPD characterization of a crystal form I of sulfate;
FIG. 3-2 shows an XRPD spectrum analysis of the crystal form I of sulfate;
FIG. 3-3 shows a DSC-TGA pattern of the crystal form I of sulfate;
FIG. 3-4 shows a polarizing micrograph of the crystal form I of sulfate;
FIG. 3-5 shows a ¹H-NMR spectrum of the crystal form I of sulfate;
FIG. 4-1 shows an XRPD characterization of a crystal form I of*p*-toluenesulfonate;
FIG. 4-2 shows an XRPD spectrum analysis of the crystal form I of*p*-toluenesulfonate;
FIG. 4-3 shows a DSC-TGA pattern of the crystal form I of*p*-toluenesulfonate;
FIG. 4-4 shows a polarizing micrograph of the crystal form I of*p*-toluenesulfonate;
FIG. 4-5 shows a ¹H-NMR spectrum of the crystal form I of*p*-toluenesulfonate;
FIG. 5-1 shows an XRPD characterization of a crystal form I of benzenesulfonate;
FIG. 5-2 shows an XRPD spectrum analysis of the crystal form I of benzenesulfonate;
FIG. 5-3 shows a DSC-TGA pattern of the crystal form I of benzenesulfonate;
FIG. 5-4 shows a polarizing micrograph of the crystal form I of benzenesulfonate;
FIG. 5-5 shows a ¹H-NMR spectrum of the crystal form I of benzenesulfonate;
FIG. 6-1 shows an XRPD characterization of a crystal form I of maleate;
FIG. 6-2 shows an XRPD spectrum analysis of the crystal form I of maleate;
FIG. 6-3 shows a DSC-TGA pattern of the crystal form I of maleate;
FIG. 6-4 shows a polarizing micrograph of the crystal form I of maleate;
FIG. 6-5 shows a ¹H-NMR spectrum of the crystal form I of maleate;
FIG. 6-6 shows an XRPD overlay spectra of the crystal form I of maleate heated to 180 °C;
FIG. 7-1 shows an XRPD characterization of a crystal form II of maleate;
FIG. 7-2 shows an XRPD spectrum analysis of the crystal form II of maleate;
FIG. 7-3 shows a DSC-TGA pattern of the crystal form II of maleate;
FIG. 7-4 shows a polarizing micrograph of the crystal form II of maleate;
FIG. 7-5 shows a ¹H-NMR spectrum of the crystal form II of maleate;
FIG. 7-6 shows an XRPD overlay spectra of the crystal form II of maleate heated to 160 °C;
FIG. 8-1 shows an XRPD characterization of a crystal form III of maleate;
FIG. 8-2 shows an XRPD spectrum analysis of the crystal form III of maleate;
FIG. 8-3 shows a DSC-TGA pattern of the crystal form III of maleate;
FIG. 8-4 shows a polarizing micrograph of the crystal form III of maleate;
FIG. 8-5 shows a ¹H-NMR spectrum of the crystal form III of maleate;
FIG. 9-1 shows an XRPD characterization of a crystal form I of oxalate;
FIG. 9-2 shows an XRPD spectrum analysis of the crystal form I of oxalate;
FIG. 9-3 shows a DSC-TGA pattern of the crystal form I of oxalate;
FIG. 9-4 shows a polarizing micrograph of the crystal form I of oxalate;
FIG. 9-5 shows a ¹H-NMR spectrum of the crystal form I of oxalate;
FIG. 10-1 shows an XRPD characterization of a crystal form I of camphorsulfonate;
FIG. 10-2 shows a DSC-TGA pattern of the crystal form I of camphorsulfonate;
FIG. 10-3 shows a ¹H-NMR spectrum of the crystal form I of camphorsulfonate;
FIG. 11-1 shows an XRPD characterization of a crystal form I of 2-hydroxyethanesulfonate;
FIG. 11-2 shows a DSC-TGA pattern of the crystal form I of 2-hydroxyethanesulfonate;
FIG. 11-3 shows a ¹H-NMR spectrum of the crystal form I of 2-hydroxyethanesulfonate;

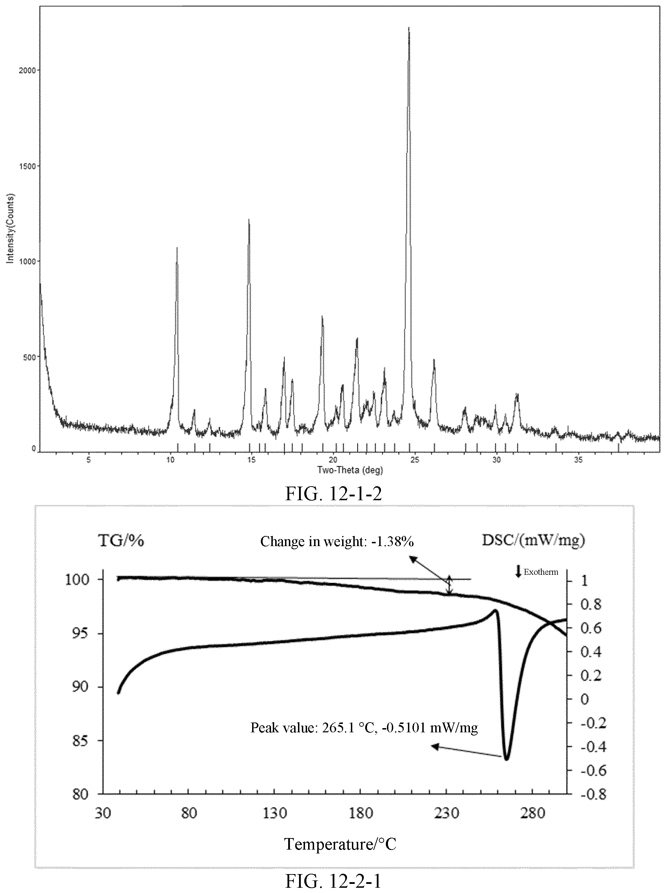

FIG. 12-1-1 shows an XRPD characterization of a crystal form I of ethanesulfonate;
FIG. 12-1-2 shows an XRPD characterization of a crystal form I of ethanesulfonate obtained from scale-up preparation;
FIG. 12-2-1 shows a DSC-TGA pattern of the crystal form I of ethanesulfonate;
FIG. 12-2-2 shows a DSC-TGA pattern of the crystal form I of ethanesulfonate obtained from scale-up preparation;
FIG. 12-3-1 shows a ¹H-NMR spectrum of the crystal form I of ethanesulfonate;
FIG. 12-3-2 shows a ¹H-NMR spectrum of the crystal form I of ethanesulfonate obtained from scale-up preparation;
FIG. 12-3-3 shows a ¹H-NMR spectrum of the crystal form I of ethanesulfonate obtained from scale-up preparation after slurrying with methanol;
FIG. 12-3-4 shows an XRPD characterization of the crystal form I of ethanesulfonate obtained from scale-up preparation before and after slurrying with methanol;
FIG. 12-3-5 shows a PLM image of the crystal form I of ethanesulfonate;
FIG. 13-1 shows an XRPD characterization of a crystal form II of ethanesulfonate;
FIG. 13-2 shows a DSC-TGA pattern of the crystal form II of ethanesulfonate;
FIG. 14-1 shows an XRPD characterization of a crystal form I of L-tartrate;
FIG. 14-2 shows a DSC-TGA pattern of the crystal form I of L-tartrate;
FIG. 14-3 shows a ¹H-NMR spectrum of the crystal form I of L-tartrate;
FIG. 15-1 shows an XRPD characterization of a crystal form I of 1,5-naphthalenedisulfonate;
FIG. 15-2 shows a DSC-TGA pattern of the crystal form I of 1,5-naphthalenedisulfonate;
FIG. 15-3 shows a ¹H-NMR spectrum of the crystal form I of 1,5-naphthalenedisulfonate;
FIG. 16-1 shows an XRPD characterization of a crystal form II of 1,5-naphthalenedisulfonate;
FIG 16-2 shows a DSC-TGA pattern of the crystal form II of 1,5-naphthalenedisulfonate;
FIG. 16-3 shows a ¹H-NMR spectrum of the crystal form II of 1,5-naphthalenedisulfonate;
FIG. 17 shows a DVS profile of the free base crystal form I;

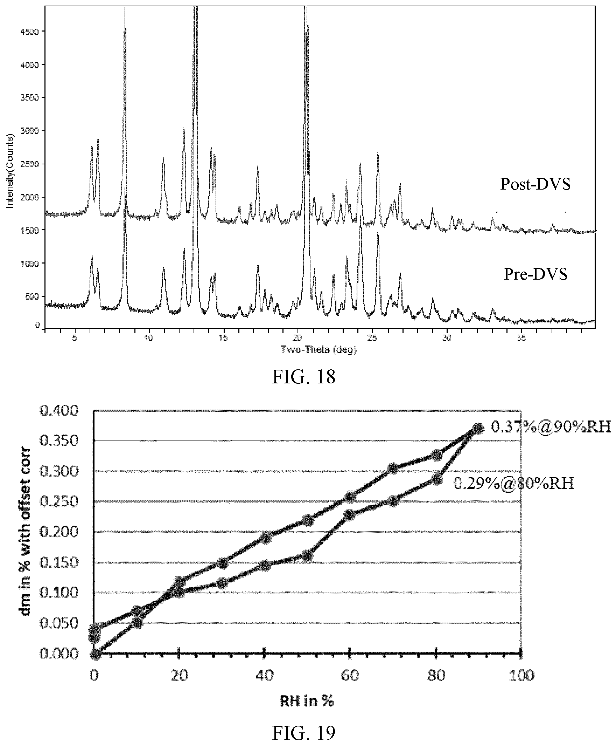

FIG. 18 shows an XRPD overlay spectra of the free base crystal form I before and after DVS test;
FIG. 19 shows a DVS profile of the crystal form III of maleate;

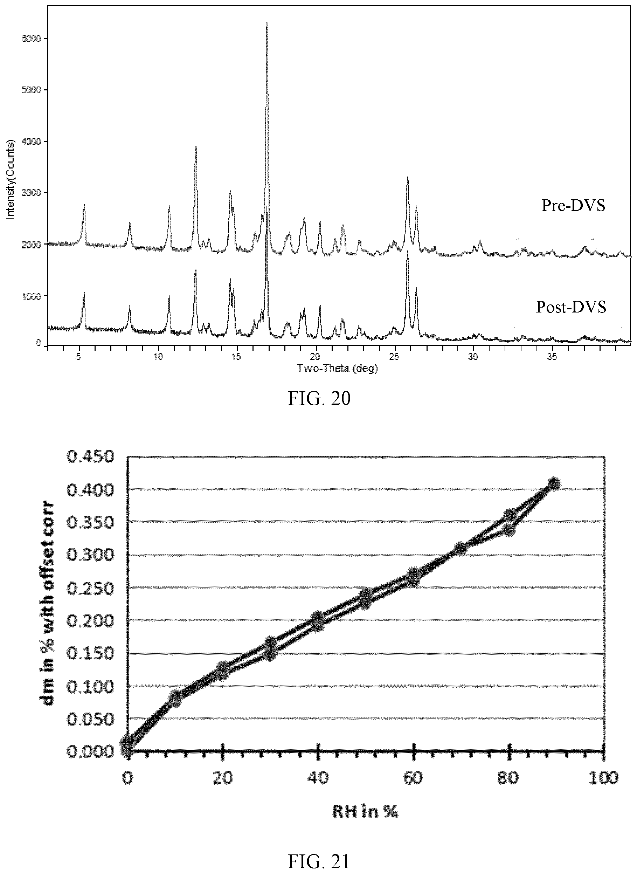

FIG. 20 shows an XRPD overlay spectra of the crystal form III of maleate before and after a DVS test;
FIG. 21 shows a DVS profile of the crystal form I of ethanesulfonate;

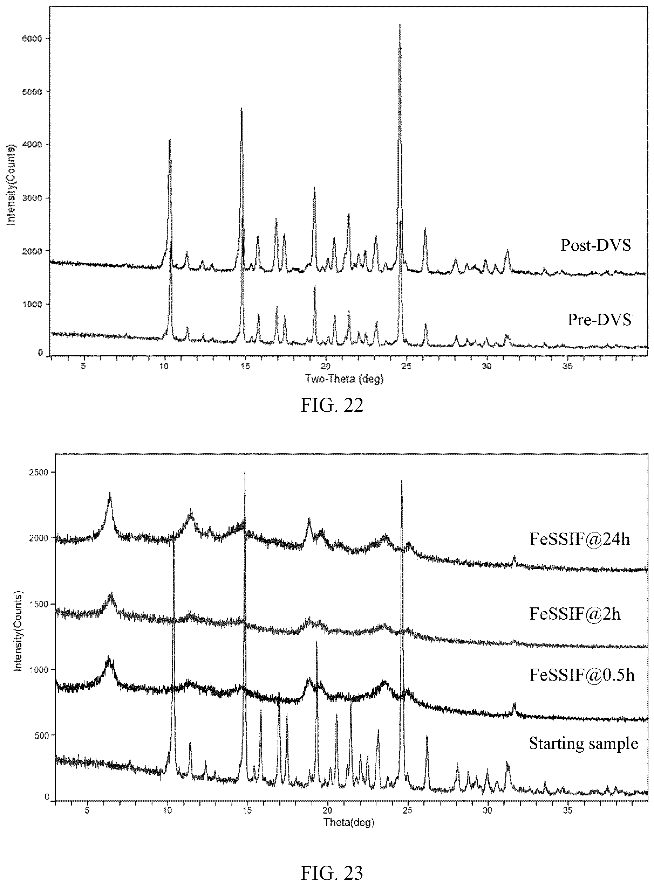

FIG. 22 shows an XRPD overlay spectra of the crystal form I of ethanesulfonate before and after a DVS test; and
FIG. 23 shows an XRPD overlay spectra of the crystal form I of ethanesulfonate before and after a solubility test in a biological medium FeSSIF.

### DETAILED DESCRIPTION

The technical solutions of the present disclosure will be further described in detail with reference to the following specific examples. It should be understood that the following examples are merely exemplary illustration and explanation of the present disclosure and should not be construed as limiting the protection scope of the present disclosure. All techniques implemented based on the contents described above of the present disclosure are encompassed within the protection scope of the present disclosure.

Unless otherwise stated, the starting materials and reagents used in the following examples are all commercially available products or can be prepared by using known methods.

**X-ray powder diffractometer (XRPD)**

The solids obtained in the experiments were subjected to solid morphology analysis using an X-ray powder diffractometer PANalytical Empyrean equipped with a PIXcel^{1D} detector. The target material of the X-ray tube of the instrument was a copper target (K-Alpha (λ = 1.5418 Ǻ)). The light tube voltage and current were 45 KV and 40 mA, respectively. The scanning range for the samples was from 3° 2θ to 40° 2θ, with a detection step size of 0.013° 2θ (except for the crystal forms of tartrate and 1,5-naphthalenedisulfonate); the detection step size for the crystal forms of tartrate and 1,5-naphthalenedisulfonate was 0.0263° 2θ.

### Differential scanning calorimetry (DSC) analysis

The instrument used for DSC analysis was Discovery DSC 250 (TA Instruments, US) or TA Q2000/2500.
① Each sample was subjected to thermal analysis using Discovery DSC 250 (TA Instruments, US): An appropriate amount of the sample was weighed and placed in a DSC sample tray, which was then pricked. The sample was then heated to the final temperature at a rate of 10 °C/min after equilibration at 25 °C.
② A DSC profile was acquired on the TA Q2000/2500 differential scanning calorimeter with the following parameters:

| | |
|---|---|
| **Parameter** | DSC |
| **Method** | Linear heating |
| **Sample tray** | Aluminum tray, with/without cover |
| **Temperature range** | 25 °C-setting endpoint temperature |
| **Purge rate (°C/min)** | 10 |
| **Protective gas** | Nitrogen |

### Thermogravimetric analysis (TGA)

Thermogravimetric analysis (TGA) was performed using TGA 55 (TA Instruments, US) or TA Q5000/5500 thermogravimetric analyzer, with specific procedures as follows:
① Thermogravimetric analysis of each sample was performed using TGA 55 (TA Instruments, US):
The sample was placed in a tared, sealed aluminum sample tray, and after the sample mass was automatically measured in a TGA furnace, the sample was heated from room temperature to the final temperature at a rate of 10 °C/min.
② A TGA profile was acquired on the TA Q5000/5500 thermogravimetric analyzer with the following parameters:

| | |
|---|---|
| **Parameter** | TGA |
| **Method** | Linear heating |
| **Sample tray** | Aluminum tray, open |
| **Temperature range** | Room temperature-setting endpoint temperature |
| **Purge rate (°C/min)** | 10 |
| **Protective gas** | Nitrogen |

### TG-DSC thermal analyzer

Each sample was subjected to thermal analysis using a TG-DSC thermal analyzer STA449F3 (NETZSCH, Germany). An appropriate amount of the sample was weighed and placed in a sample tray. The sample was then heated to the final temperature at a rate of 10 °C/min after equilibration at 25 °C.

### Polarizing microscope (PLM) analysis

The instrument used for PLM was Polarizing Microscope ECLIPSE LV100POL (Nikon, JPN).

### Hydrogen nuclear magnetic resonance spectroscopy (¹H-NMR)

The hydrogen spectrum information of the samples was confirmed by ¹H-NMR. The instrument used for ¹H-NMR analysis was Bruker AVANCE III HD 300/400 equipped with a Sample Xpress 60 autosampler system.

### Dynamic vapor sorption (DVS) analysis

The instrument used for dynamic vapor sorption analysis was Vsorp (ProUmid GmbH & Co. KG, Germany).

Each sample was subjected to vapor sorption/desorption tests using the Vsorp vapor sorption analyzer (ProUmid GmbH & Co. KG, Germany). The sample was placed in a tared sample tray, and the change in the sample mass with humidity (0-90% RH) at 25 °C was recorded. The specific DVS test parameters are as follows:

| | |
|---|---|
| **Equilibrium condition:** | 0.01% per/45 min |
| **Time of cyclic weighing:** | 10 min |
| **Minimum time interval:** | 50 min |
| **Maximum time interval:** | 2.0 h |
| **Equilibrium condition:** | 40 °C @ 0% RH (relative humidity) for 6 h |
| **Sample test temperature:** | 25 °C |
| **Sorption humidity:** | 0, 10, 20, 30, 40, 50, 60, 70, 80, 90 %RH |
| **Desorption humidity:** | 80, 70, 60, 50, 40, 30, 20, 10, 0 %RH |

### High-performance liquid chromatography (HPLC) analysis

The instrument used for HPLC analysis was Agilent HPLC 1260 series or Shimadzu LC-20ADxr.
① When the instrument used was Agilent HPLC 1260 series:
A) The HPLC method used for the solubility test was as follows:

| | |
|---|---|
| **Instrument** | Agilent HPLC 1260 series |
| **Chromatographic column** | Atlantis T3 4.6*150 mm, 3 µm |
| **Mobile phase** | A: 0.01% TFA in water |
| | B: 0.01% TFA in ACN |
| **Gradient (T (min)/B (%))** | 0/20%, 11/70%, 12.0/100%, 14.0/20% |
| **Column temperature** | 35 °C |
| **Detector** | DAD, 290 nm |
| **Flow rate** | 1.0 mL/min |
| **Injection volume** | 3.5 µL |
| **Elution time** | 14.0 min |
| **Post-run time** | 3 min |
| **Diluent** | Salt: ACN/H₂O (1/1, v/v) |
| | Free base: DMSO |

B) The HPLC method used for the stability test was as follows:

| | |
|---|---|
| **Instrument** | Agilent HPLC 1260 series |
| **Chromatographic column** | Xbridge shield RP18 4.6*150 mm, 3 µm |
| **Mobile phase** | A: 0.1% TFA in water; |
| | B: 0.1% TFA in ACN |
| **Gradient (T (min)/B (%))** | 0/10%, 2.0/10%, 20.0/50%, 25.0/80%, 30.0/80%, 30.5/10%, 35.0/10% |
| **Column temperature** | 30 °C |
| **Detector** | DAD, 230 nm |
| **Flow rate** | 1.0 mL/min |
| **Injection volume** | 2 µL |
| **Elution time** | 35 min |
| **Post-run time** | 0 min |
| **Diluent** | Salt: MeOH/ACN/H₂O (2/1/1, v/v/v) |
| | Free base: DMSO |

② When the instrument used was Shimadzu LC-20ADxr:
A) The HPLC method used for the solubility test was as follows: 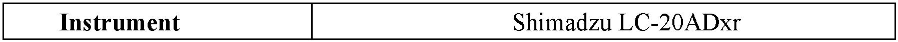 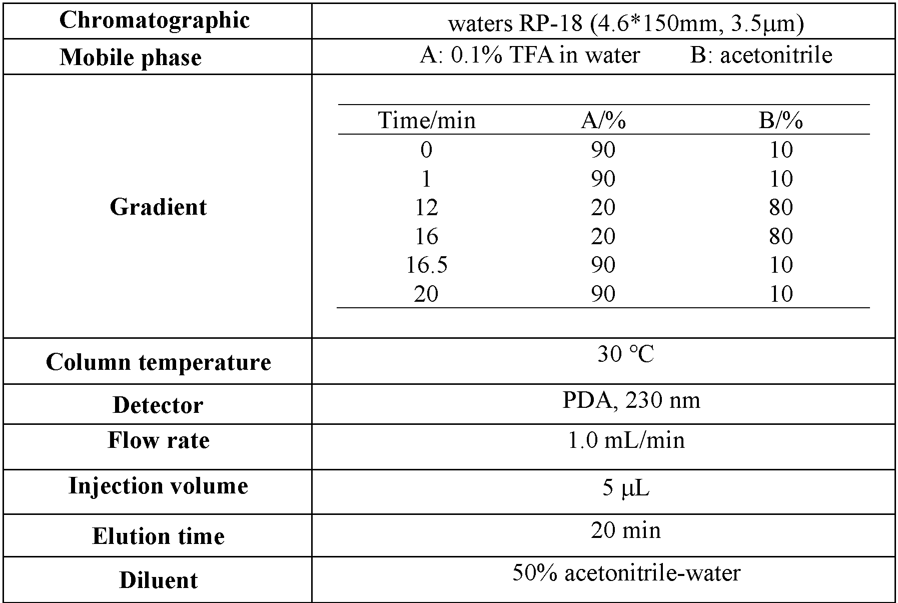
B) The HPLC method used for the stability study was as follows:

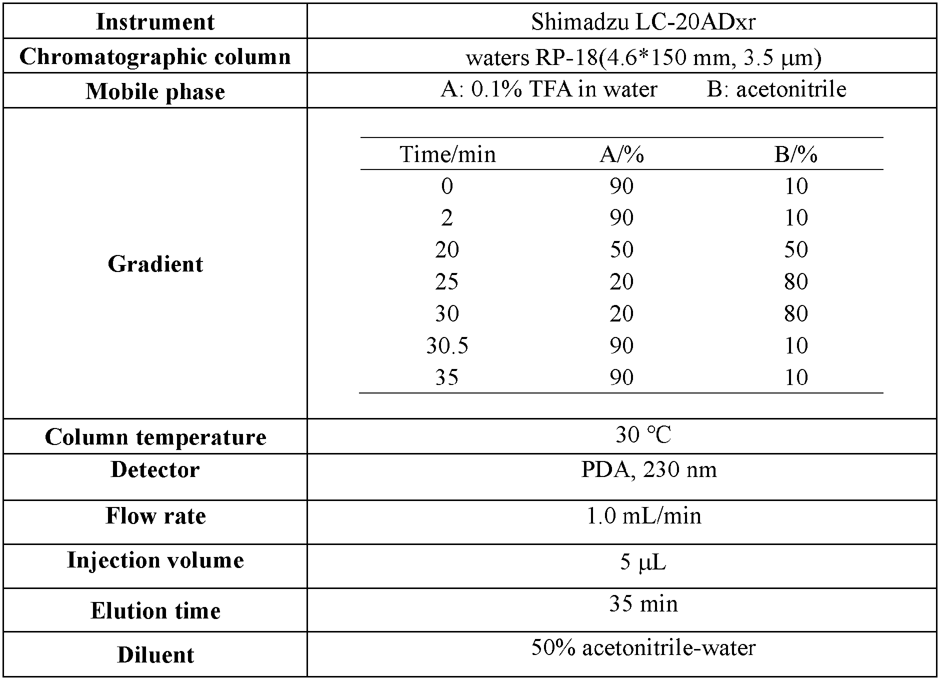

### Ion chromatography (IC)

The instrument used for IC analysis was Thermo ICS-6000. The method used for the ion chromatography test was as follows:

| | |
|---|---|
| **Instrument** | Thermo ICS-6000 |
| **Work station** | Chromeleon Workstation |
| **Eluent generator** | EGC 500 KOH |
| **Suppressor** | Dionx ASRS 300 4 mm |
| **Guard column** | Dionex IonPacTMAG11-HC (4*50 mm) |
| **Chromatographic column** | Dionex IonPacTMAG11-HC (4*250 mm) |
| **Temperature of conductance cell** | 35.0 °C |
| **Concentration of eluent** | 30 mm |
| **Working mode of suppressor** | External mode |
| **Current of suppressor** | 75 mA |
| **Column temperature** | 30 °C |
| **Flow rate** | 1.0 mL/min |
| **Elution gradient** | Isocratic elution |
| **Run time** | 10 min |
| **Injection volume** | 25 µL |
| **Flow rate of external water circulation** | 1.5 mL/min |

### List of reagents

| **Serial number** | **In Chinese** | **In English** | **Abbreviation** |
|---|---|---|---|
| 1 | 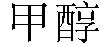 | Methanol | MeOH |
| 2 | 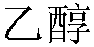 | Ethanol | EtOH |
| 3 | 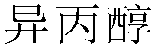 | Isopropyl alcohol | IPA |
| 4 | 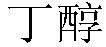 | n-Butanol | n-BuOH |
| 5 | 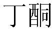 | Butanone | MEK |
| 6 | 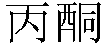 | Acetone | - |
| 7 | 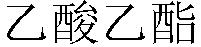 | Ethyl acetate | EA |
| 8 | 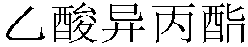 | Isopropyl acetate | IPAC |
| 9 | 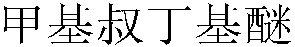 | Methyl tert-butyl ether | MTBE |
| 10 | 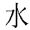 | Water | - |
| 11 | 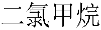 | Dichloromethane | DCM |
| 12 | 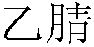 | Acetonitrile | ACN |
| 13 | 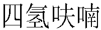 | Tetrahydrofuran | THF |
| 14 | 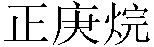 | Heptane | Hept |
| 15 | 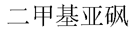 | Dimethyl sulfoxide | DMSO |
| 16 | 2- 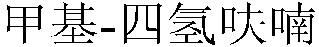 | 2-Methyltetrahydrofuran | 2-Me-THF |
| 17 | 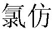 | Chloroform | - |
| 18 | 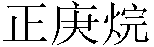 | Heptane | Hep. |
| 19 | 4-Dimethylaminopyridine | - | DMAP |
| 20 | *N*,*N*-Dimethylformamide | - | DMF |
| 21 | *N*,*N*-Diisopropylethylamine | - | DIEA |
| 22 | *O*-(7-Azabenzotriazol-1-yl)-*N*,*N*,*N*',*N*'-tetramethyluronium hexafluorophosphate | - | HATU |

### Example 1: Preparation and Characterization of Compound I

The preparation method for compound I comprised the following steps:

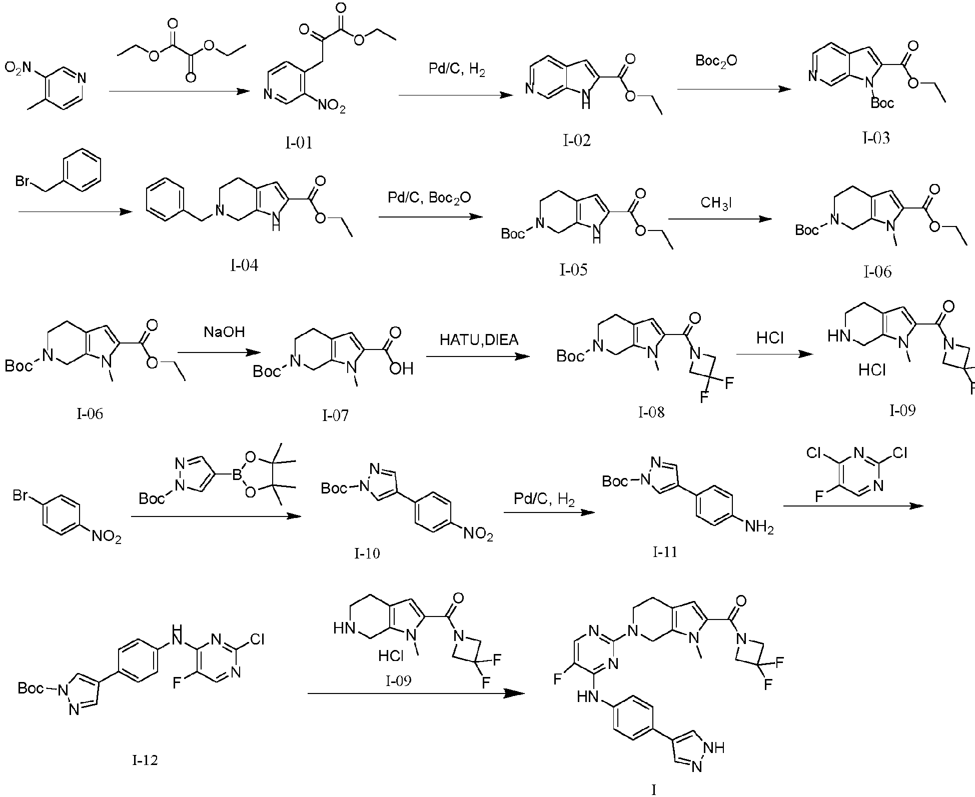

### 1. Preparation of compound ethyl 3-(3-nitropyridin-4-yl)-2-oxopropanoate (I-01)

4-Methyl-3-nitropyridine (10.0 g, 72.0 mmol) was dissolved in diethyl oxalate (50 mL) under a nitrogen atmosphere, and DBU (12.7 g, 83.0 mmol) was added to the reaction liquid. The mixture was stirred at room temperature overnight. Ice water (30.0 g) was added to the reaction system to quench the reaction, and 1 N diluted hydrochloric acid was added to adjust the pH to 4-5. The mixture was filtered, and the filter cake was washed with ethyl acetate (100 mL × 2) and dried in an oven to give a red solid (18.0 g). The crude product was directly used in the next step. LC-MS [M+H]⁺ = 239.1.

### 2. Preparation of compound ethyl 1H-pyrrolo[2,3-c]pyridine-2-carboxylate (I-02)

I-01 (17.0 g, 70.0 mmol) was dissolved in anhydrous dichloromethane (200 mL) under a hydrogen atmosphere, and palladium on carbon (3.4 g, with the mass fraction of Pd in the palladium on carbon being 10%) was added to the reaction liquid. The mixture was reacted at room temperature overnight. Then, the mixture was filtered, and the filtrate was concentrated under reduced pressure. The residue obtained after the concentration was separated by silica gel column chromatography (PE/EA = 1/1) to give a yellow oily liquid (6.0 g, two-step yield: 43.8%). LC-MS [M+H]⁺ = 191.1.

### 3. Preparation of compound 1-(tert-butyl) 2-ethyl 1H-pyrrolo[2,3-c]pyridine-1,2-dicarboxylate (I-03)

I-02 (6.0 g, 31.5 mmol) was dissolved in anhydrous dichloromethane (50 mL) under a nitrogen atmosphere, and Boc anhydride (7.6 g, 34.7 mmol) and DMAP (384 mg,3.2 mmol) were added to the reaction liquid. The mixture was stirred at room temperature overnight. Then, the mixture was concentrated under reduced pressure, and the resulting crude product was separated by silica gel column chromatography (PE/EA = 3/1) to give a yellow oily liquid (7.5 g, yield: 82.4%). LC-MS [M+H]⁺ = 291.0.

### 4. Preparation of compound ethyl 6-benzyl-4,5,6,7-tetrahydro-1H-pyrrolo[2,3-c]pyridine-2-carboxylate (I-04)

I-03 (7.2 g, 24.7 mmol) was dissolved in anhydrous DMF (50 mL) under a nitrogen atmosphere, and benzyl bromide (4.2 g, 24.7 mmol) was added. The mixture was reacted at 80 °C for 4 h and then concentrated under reduced pressure. The resulting solid was dissolved in ethanol (50 mL), and sodium borohydride (934 mg, 24.7 mmol) was added to the reaction liquid. The mixture was reacted at room temperature for 3 h. Water (100 mL) was added to quench the reaction, and the resulting mixture was extracted with ethyl acetate (100 mL × 3). The organic phases were combined, washed with saturated brine (100 mL), dried over anhydrous sodium sulfate, and filtered, and the filtrate was concentrated. The resulting crude product was separated by silica gel column chromatography (PE/EA = 3/1)to give a yellow oily liquid (3.5 g, yield: 49.8%). LC-MS [M+H]⁺ = 285.1.

### 5. Preparation of compound 6-(tert-butyl) 2-ethyl 1,4,5,7-tetrahydro-6H-pyrrolo[2,3-c]pyridine-2,6-dicarboxylate (I-05)

I-04 (3.5 g, 12.3 mmol) was dissolved in methanol (40 mL), and Boc anhydride (4.0 g,18.5 mmol) and palladium on carbon (700 mg, with the mass fraction of Pd in the palladium on carbon being 10%) were added. The mixture was reacted under a hydrogen atmosphere at room temperature overnight. Then, the mixture was filtered, and the filtrate was concentrated. The residue was separated by silica gel column chromatography (PE/EA = 5/1) to give a yellow oily liquid (1.8 g, yield: 50.0%). MS [M/2+H]⁺ = 295.1.

### 6. Preparation of compound 6-(tert-butyl) 2-ethyl 1-methyl-1,4,5,7-tetrahydro-6H-pyrrolo[2,3-c] pyridine-2,6-dicarboxylate (I-06)

I-05 (1.8 g, 6.1 mmol) was dissolved in tetrahydrofuran (20 mL) under a nitrogen atmosphere, and the reaction system was then cooled to 0 °C. Sodium hydride (364 mg, 9.1 mmol, 60%) was added to the reaction system, and the mixture was stirred at this temperature for 30 min, followed by dropwise addition of iodomethane (1.7 g, 12.2 mmol). The mixture was reacted at room temperature for 2 h. Saturated ammonium chloride (20 mL) was added to quench the reaction, and the resulting mixture was extracted with ethyl acetate (30 mL × 3). The organic phases were combined, washed with saturated brine (50 mL), dried over anhydrous sodium sulfate, and filtered, and the filtrate was concentrated to give a yellow oily liquid (1.8 g, crude product). LC-MS [M+H]⁺ = 308.9.

### 7. Preparation of compound 6-(tert-butoxycarbonyl)-1-methyl-4,5,6,7-tetrahydro-1H-pyrrolo [2,3-c]pyridine-2-carboxylic acid (I-07)

I-06 (4.0 g,12.9 mmol) was dissolved in methanol (40 mL), and a sodium hydroxide solution (38.7 mL, 1 N) was added. The mixture was reacted at 50 °C for 2 h. Diluted hydrochloric acid (1 N) was added to adjust the pH to 5-6, and ethyl acetate (50 mL × 3) was added for extraction. The organic phase was washed with saturated brine (50 mL), dried over anhydrous sodium sulfate, and filtered, and the filtrate was concentrated under reduced pressure to give a purple oily liquid (3.2 g). LC-MS [M+H]⁺ = 281.1.

### 8. Preparation of compound tert-butyl 2-(3,3-difluoroazetidine-1-carbonyl)-1-methyl-1,4,5,7-tetrahydro-6H-pyrrolo[2,3-c]pyridine-6-carboxylate (I-08)

I-07 (2.4 g,8.5 mmol) was dissolved in anhydrous DMF (20 mL), and 3,3-difluoroazetidine hydrochloride (1.65 g, 12.8 mmol), HATU (4.9 g, 12.8 mmol) and DIEA (3.3 g, 25.6 mmol) were added. The mixture was reacted at room temperature for 1 h. Water (30 mL) was added to quench the reaction, and the resulting mixture was extracted with ethyl acetate (30 mL × 3). The organic phases were combined, washed with saturated brine (50 mL), dried over anhydrous sodium sulfate, and filtered, and the filtrate was concentrated under reduced pressure. The resulting residue was purified by silica gel column chromatography (PE/EA = 3/1) to give a yellow oily liquid (2.5 g, yield: 83.3%). LC-MS [M+H]⁺ = 355.8.

### 9. Preparation of compound (3,3-difluoroazetidin-1-yl)(1-methyl-4,5,6,7-tetrahydro-1H-pyrrolo [2,3-c]pyridin-2-yl)methanone hydrochloride (I-09):

I-08 (1.8 g, 5.8 mmol) was dissolved in absolute ethanol (20 mL), and a solution of hydrochloric acid in ethanol (5 mL, with the mass fraction of HCl being 33%) was added. The mixture was reacted at room temperature for 3 h. Then, the reaction liquid was concentrated by rotary evaporation under reduced pressure to give a yellow solid (2.0 g). LC-MS [M+H]⁺ = 256.0.

### 10. Preparation of tert-butyl 4-(4-nitrophenyl)-1H-pyrazole-1-carboxylate (I-10)

*tert*-Butyl 4-(4,4,5,5-tetramethyl-1,3,2-dioxaborolan-2-yl)-1*H*-pyrazole-1-carboxylate (58.8 g, 0.2 mol), 1-bromo-4-nitrobenzene (20 g, 0.1 mol), anhydrous potassium carbonate (41.5 g, 0.3 mol), and (1,1'-bis(diphenylphosphino)ferrocene)dichloropalladium(II) (7.3 g, 10 mmol) were added to a mixed solvent of 1,4-dioxane and water (1,4-dioxane:water = 30:1 (v:v), 207 mL), and the reaction system was purged 3 times with nitrogen. Then, the mixture was stirred in an oil bath at 100 °C for 5 h. After the reaction was completed, the mixture was filtered under reduced pressure, and the filtrate was concentrated. The resulting residue was purified by silica gel column chromatography (petroleum ether:ethyl acetate = 4:1) to give a yellow solid (16 g, yield: 46%, MS (M+1-100) = 190.05), which confirmed as product I-10 according to the hydrogen spectrum.

### 11. Preparation of tert-butyl 4-(4-aminophenyl)-1H-pyrazole-1-carboxylate (I-11)

I-10 (16 g, 55.3 mol) was dissolved in methanol (160 mL), and palladium on carbon (3.2 g, with the mass fraction of Pd in the palladium on carbon being 10%) was added. A hydrogen balloon was provided, and the reaction system was purged with hydrogen and reacted at 45 °C under the hydrogen atmosphere for 16 h. Then, the mixture was filtered through celite under reduced pressure. The filtrate was concentrated to give a yellow crude product, which was then purified by silica gel column chromatography (petroleum ether:ethyl acetate = 3:1) to give light white solid (9.6 g, yield: 60%). MS (M+1) = 260.15.

### 12. Preparation of compound tert-butyl 4-(4-((2-chloro-5-fluoropyrimidin-4-yl)amino)phenyl)-1H-pyrazole-1-carboxylate (I-12):

I-11 (1.8 g, 6.9 mmol) was dissolved in ethanol (20 mL), and 2,4-dichloro-5-fluoropyrimidine (2.3 g, 13.8 mmol) and DIEA (3.6 g, 27.6 mmol) were added sequentially. The mixture was stirred and reacted at 40 °C for 2 h. After the reaction was completed, the mixture was cooled to room temperature. Water (20 mL) was added to quench the reaction, and then ethyl acetate (15 mL × 3) was added for extraction. The organic phases were combined, washed with saturated brine (30 mL × 2), dried over anhydrous sodium sulfate, and filtered, and the filtrate was concentrated under reduced pressure to give a crude product. The crude product was purified by silica gel column chromatography (PE/EA = 3/1) to give a brown solid product (2.3 g, yield: 85.2%). LC-MS [M+H]⁺ = 389.9.

### 13. Preparation of compound (6-(4-((4-(1H-pyrazol-4-yl)phenyl)amino)-5-fluoropyrimidin-2-yl)-1-methyl-4,5,6,7-tetrahydro-1H-pyrrolo[2,3-c]pyridin-2-yl)(3,3-difluoroazetidin-1-yl)methanone (compound I):

I-12 (1.6 g, 12.3 mmol) was dissolved in *n*-butanol (16 mL), and I-09 (784 mg, 3.1 mmol) and DIEA (1.2 g, 3.1 mmol) were added to the reaction system. The mixture was stirred at 120 °C overnight. After the reaction was completed, the mixture was cooled to room temperature. Water (20 mL) was added to quench the reaction, and the resulting mixture was extracted with ethyl acetate (20 mL × 3).

The organic phases were combined, washed with saturated brine (20 mL × 3), dried over anhydrous sodium sulfate, and filtered, and the filtrate was concentrated under reduced pressure to give a crude product. The crude product was purified by silica gel column chromatography (DCM/MeOH = 20/1) and then lyophilized to give a white solid (600 mg). LC-MS [M+H]⁺ = 508.7.

¹H NMR (400 MHz, DMSO-*d₆*) δ 12.90 (s, 1H), 9.34 (s, 1H), 8.16 (s, 1H), 8.06 (d, J = 3.7 Hz, 1H), 7.91 (s, 1H), 7.76 (d, J = 8.7 Hz, 2H), 7.59 (d, J = 8.7 Hz, 2H), 6.46 (s, 1H), 4.73 (s, 2H), 4.56 (s, 4H), 3.92 (t, J = 5.5 Hz, 2H), 3.74 (s, 3H), 2.55 (t, J = 5.2 Hz, 2H).

### Characterization

An appropriate amount of the starting material compound I free base was taken and subjected to characterization by PLM, DSC (Discovery DSC 250 (TA Instruments, US)), TGA (TGA 55 (TA Instruments, US)), XRPD, and ¹H-NMR. The results are shown in Table 1-1.

The XRPD spectrum showed that the free base had relatively high crystallinity; the free base was designated as a free base crystal form I (FIGs. 1-1 and 1-2). The ¹H-NMR results showed that the compound I sample contained residual solvents of 0.5% DCM and 0.7% THF (FIG. 1-4). The DSC results showed that the sample only had one endothermic peak with a melting point of 271 °C, which was caused by the melting of the free base crystal form I; TGA shows that the free base crystal form I had no significant weight loss before 150 °C (FIG. 1-3).

**Table 1-1. Characterization results for the starting material compound I**

| **Item** | **Compound I** |
|---|---|
| XRPD | Free base crystal form I |
| ¹H-NMR | 0.5% DCM, 0.7% THF |
| DSC onset temperature/peak temperature of endothermic peak, ΔH | 271/273 °C, 114 J/g |
| TGA weight loss %/@T | ∼0%/RT-150 °C |

### Example 2: Hydrochloride

At room temperature, 30 mg of compound I was weighed and added into a sample bottle, and then 0.5 mL of MeOH was added. 54 µL of hydrochloric acid (12 M) was diluted in 1 mL of MeOH, and the diluted hydrochloric acid solution (100 µL, 1.1 eq.) was added slowly to the suspension of compound I. The mixture was stirred at room temperature overnight (about 21 °C, 16 h). Then, the mixture was filtered, and a sample (filter cake) was collected, dried under vacuum at 40 °C for about 4 h, and then subjected to XRPD characterization. Specific information and results are summarized in Table 2-1.

**Table 2-1. Preparation of hydrochloride**

| **Group** | **Solvent** | **Amount of HCl used (eq.)** | **Result** |
|---|---|---|---|
| 1 | MeOH | 1.1 | Crystal form I of hydrochloride |

| | | | |
|---|---|---|---|
| Note: The "eq." in the table refers to the molar ratio of the acid to compound I. | | | |

In this example, one crystal form of hydrochloride was obtained, which was designated as a crystal form I of hydrochloride. Characterization was performed by PLM, DSC (Discovery DSC 250 (TA Instruments, US)), TGA (TGA 55 (TA Instruments, US)), XRPD, and ¹H-NMR. The relevant characterization results are shown in Table 2-2 and FIG. 2-1 to FIG. 2-5.

The crystal form I of hydrochloride was in the form of very small crystals (< 10 µm, FIG. 2-4). There was a weight loss of about 0.3% before 90 °C. The DSC profile showed two endothermic peaks: a broad endothermic peak at a temperature of about 56 °C, which was probably caused by the dehydration of the hydrochloride, and an endothermic peak at a temperature of about 241 °C, which was caused by the melting and concomitant decomposition of the hydrochloride. The ¹H-NMR analysis showed that the sample contained no residual organic solvent. The IC analysis showed that this sample contained about 1 eq. of chloride ions, so the salt-forming ratio was 1/1 (Table 2-3). The crystal form I of hydrochloride is an anhydrous crystal form with moderate crystallinity.

**Table 2-2. Solid state characterization results for crystal form I of hydrochloride**

| **Crystal form solvation** | **DSC, onset temperature/peak temperature of endothermic peak (°C), ΔH (J/g)** | **TGA weight loss %/ @T (°C)** | **¹H-NMR** | **IC acid/base ratio** |
|---|---|---|---|---|
| I anhydrous crystal form | 25/56, 50 | ∼0.3/RT-90 | No residual organic solvent | 1:1 |
| | 235/241, m/d | | | |

| | | | | |
|---|---|---|---|---|
| Note: m/d: melting and concomitant decomposition; RT = room temperature | | | | |

**Table 2-3. IC data for crystal form I of hydrochloride**

| **Sample** | **Mass (mg)** | **Theoretical concentration (µg/mL)** | **IC peak area (µS*min)** | **Result** |
|---|---|---|---|---|
| Cl⁻-standard solution (Cl⁻-STD) | NA | 10 | 2.86 | NA |
| Cl⁻-standard solution (Cl⁻-STD) | NA | 20 | 5.82 | NA |
| Cl⁻-standard solution (Cl⁻-STD) | NA | 40 | 11.46 | NA |
| Crystal form I of hydrochloride | 4.08 | 26.6 (calculated based on 1 eq.) | 6.95 (~24 µg/mL) | About 1 eq. of Cl⁻ |

### Example 3: Sulfate

At room temperature, 30 mg of compound I was weighed and added into a sample bottle, and then 0.5 mL of MeOH was added. 18 µL of concentrated sulfuric acid (18 M) was diluted in 1 mL of MeOH, and the diluted sulfuric acid solution (100 µL, 0.55 eq.) was added slowly to the suspension of compound I. The mixture was stirred at room temperature overnight (about 21 °C, 16 h). Then, the mixture was filtered, and a sample (filter cake) was collected, dried under vacuum at 40 °C for about 4 h, and then subjected to XRPD characterization. Specific information and results are summarized in Table 3-1.

**Table3-1. Preparation of sulfate**

| **Group** | **Solvent** | **Amount of H₂SO₄ used (eq.)** | **XRPD** |
|---|---|---|---|
| 1 | MeOH | 0.55 | Crystal form I of sulfate |

| | | | |
|---|---|---|---|
| Note: The "eq." in the table refers to the molar ratio of the acid to compound I. | | | |

In this example, one crystal form of sulfate was obtained, which was designated as a crystal form I of sulfate. The crystal form I of sulfate was subjected to characterization by PLM, DSC (Discovery DSC 250 (TA Instruments, US)), TGA (TGA 55 (TA Instruments, US)), XRPD, and ¹H-NMR. The relevant characterization results are shown in Table 3-2 and FIG. 3-1 to FIG. 3-5.

The crystal form I of sulfate was in the form of irregular crystals (FIG. 3-4). There was a weight loss of about 3% before 125 °C, and the ¹H-NMR analysis showed that the sample contained no residual organic solvent, suggesting presumable dehydration of the sulfate (about 1 eq. of water). The DSC profile showed two endothermic peaks: a broad endothermic peak at a temperature of about 104 °C, which was probably caused by the dehydration of the sulfate, and an endothermic peak at a temperature of about 163 °C, which was caused by the melting and concomitant decomposition of the sulfate. The IC analysis showed that this sample contained 0.5 eq. of sulfate ions (Table 3-3), so the salt-forming ratio was 0.5/1. The crystal form I of sulfate was presumed to be a hydrate.

**Table 3-2. Solid state characterization results for crystal form I of sulfate**

| **Crystal form solvation** | **DSC, onset temperature/peak temperature of endothermic peak (°C), ΔH (J/g)** | **TGA weight loss %/ @T (°C)** | **¹H-NMR** | **IC acid:base** |
|---|---|---|---|---|
| I hydrate | 26/104, 146 | ∼3/RT-125 | No residual organic solvent | 0.5:1 |
| | 157/163, 12 | | | |

| | | | | |
|---|---|---|---|---|
| Note: RT = room temperature | | | | |

**Table 3-3. IC data for crystal form I of sulfate**

| **Sample** | **Mass (mg)** | **Theoretical concentration (µg/mL)** | **IC peak area (µS*min)** | **Result** |
|---|---|---|---|---|
| SO₄²⁻ standard solution (SO₄²⁻-STD) | NA | 32.5 | 6.73 | NA |
| Crystal form I of sulfate | 2.02 | About 17.7 (calculated based on 0.5 eq.) | 3.51 | About 0.5 eq. of SO₄²⁻ |

### Example 4: p-Toluenesulfonate

At room temperature, 30 mg of compound I was weighed and added into a sample bottle, and then 0.6 mL of the selected solvent MeOH was added. A *p*-toluenesulfonic acid solid (11.2 mg, 1.1 eq.) was further added, and the suspension was stirred at room temperature overnight (about 21 °C, 16 h). Then, the suspension was filtered, and a sample (filter cake) was collected, dried under vacuum at 40 °C for about 4 h, and then subjected to XRPD characterization. Specific information and results are summarized in Table 4-1.

**Table 4-1. Preparation ofp-toluenesulfonate**

| **Group** | **Solvent** | **Amount of *p-*toluenesulfonic acid used (eq.)** | **XRPD** |
|---|---|---|---|
| 1 | MeOH | 1.1 | Crystal form I of *p-*toluenesulfonate |

| | | | |
|---|---|---|---|
| Note: The "eq." in the table refers to the molar ratio of the acid to compound I. | | | |

In this experiment, one crystal form ofp-toluenesulfonate was identified and defined as a crystal form I of *p*-toluenesulfonate. Characterization was performed by PLM, DSC (Discovery DSC 250 (TA Instruments, US)), TGA (TGA 55 (TA Instruments, US)), XRPD, and ¹H-NMR. The relevant characterization results are shown in Table 4-2 and FIG. 4-1 to FIG. 4-5.

The crystal form I of *p*-toluenesulfonate was in the form of irregular crystals (FIG. 4-3). There was a weight loss of about 2% before 110 °C, and the ¹H-NMR analysis showed that the sample contained a very small amount of residual organic solvent, so the weight loss observed in TGA was primarily attributed to the desorption of water. The DSC profile showed two endothermic peaks: a broad endothermic peak at a temperature of about 94 °C, which was attributed to dehydration, and an endothermic peak at a temperature of about 198 °C, which was caused by melting and concomitant decomposition. Thus, the crystal form I of *p*-toluenesulfonate was presumed to be a hydrate.

**Table 4-2. Solid state characterization results for crystal form I ofp-toluenesulfonate**

| **Crystal form Solvation** | **DSC, onset temperature/peak temperature of endothermic peak (°C), ΔH (J/g)** | **TGA weight loss %/ @T (°C)** | **¹H-NMR** |
|---|---|---|---|
| I Hydrate | 58/94, 78 | ∼2/RT-110 | About 1 eq. of acid |
| | 179/198, m/d | | |

| | | | |
|---|---|---|---|
| Note: m/d: melting and concomitant decomposition; RT = room temperature | | | |

### Example 5: Benzenesulfonate

At room temperature, 30 mg of compound I was weighed and added into a sample bottle, and then 0.6 mL of the selected solvent MeOH was added. A benzenesulfonic acid solid (10.3 mg, 1.1 eq.) was further added, and the suspension was stirred at room temperature overnight (about 21 °C, 16 h). Then, the suspension was filtered, and a sample (filter cake) was collected, dried under vacuum at 40 °C for about 4 h, and then subjected to XRPD characterization. Specific information and results are summarized in Table 5-1.

**Table 5-1. Preparation of benzenesulfonate**

| **Group** | **Solvent** | **Amount of benzenesulfonic acid used (eq.)** | **XRPD** |
|---|---|---|---|
| 1 | MeOH | 1.1 | Crystal form I of benzenesulfonate |

| | | | |
|---|---|---|---|
| Note: The "eq." in the table refers to the molar ratio of the acid to compound I. | | | |

In this experiment, one crystalline salt form was identified and defined, which was designated as a crystal form I of benzenesulfonate. The sample of the crystal form I of benzenesulfonate subjected to characterization by PLM, DSC (Discovery DSC 250 (TA Instruments, US)), TGA (TGA 55 (TA Instruments, US)), XRPD, and ¹H-NMR. The relevant characterization results are shown in Table 5-2 and FIG. 5-1 to FIG. 5-5.

The crystal form I of benzenesulfonate was in the form of fine crystals (FIG. 5-4). There was a weight loss of about 3.9% before 100 °C, and the weight loss observed in TGA was primarily attributed to the desorption of water. The DSC profile showed a broad endothermic peak at a temperature of about 109 °C, which was attributed to desorption of water, and an endothermic peak at a temperature of about 176 °C, which was attributed to melting and concomitant decomposition. The crystal form I of benzenesulfonate was determined to be a hydrate.

**Table 5-2. Solid state characterization results for crystal form I of benzenesulfonate**

| **Crystal form Solvation** | **DSC, onset temperature/peak temperature of endothermic peak (°C), ΔH (J/g)** | **TGA weight loss %/ @T (°C)** | **¹H-NMR** |
|---|---|---|---|
| I Hydrate | 79/109, 120 | ∼3.9/RT- 100 | 1 eq. of acid |
| | 168/176, m/d | | |

| | | | |
|---|---|---|---|
| Note: m/d: melting and concomitant decomposition; RT = room temperature | | | |

### Example 6: Maleate

### Method 1:

At room temperature, 30 mg of compound I was weighed, added into a sample bottle, and then suspended in 0.6 mL of the corresponding solvent in Table 6-1. A maleic acid solid (7.5 mg, 1.1 eq.) was further added, and the suspension was stirred at room temperature overnight (about 21 °C, 16 h).

Then, the suspension was filtered, and a sample (filter cake) was collected, dried under vacuum at 40 °C for about 4 h, and then subjected to XRPD characterization.

In this experiment, 2 crystal forms of maleate were discovered, which were designated as a crystal form I of maleate and a crystal form II of maleate, respectively. Specific information and results are summarized in Table 6-1.

**Table 6-1. Preparation of maleate**

| **Group** | **Solvent** | **Amount of maleic acid used (eq.)** | **XRPD** |
|---|---|---|---|
| 1 | MeOH | 1.1 | Crystal form I of maleate |
| 2 | ACN | | Crystal form II of maleate |
| 3 | THF | | Crystal form II of maleate |

| | | | |
|---|---|---|---|
| Note: The "eq." in the table refers to the molar ratio of the acid to compound I. | | | |

### Method 2:

At room temperature, 20 mg of compound I was weighed and added into a sample bottle, and then the corresponding solvent shown in Table 6-2 was added. 5.03 mg of a maleic acid solid was dissolved in 0.1 mL of the selected solvent (with the exception of IPAC which was just mixed with the maleic acid solid), and the resulting solution was added dropwise and slowly to the suspension of free base.

The mixture was stirred at room temperature overnight (about 22 °C, 24 h). Then, the mixture was filtered, and a sample (filter cake) was collected, dried under vacuum at 40 °C for about 4 h, and then subjected to XRPD characterization.

In this experiment, one new crystal form of maleate was discovered, which was designated as a crystal form III of maleate. Specific information and results are summarized in Table 6-2.

**Table 6-2. Preparation of maleate**

| **Group** | **Solvent** | **Volume of solvent (mL)** | **Amount of maleic acid used (eq.)** | **XRPD** |
|---|---|---|---|---|
| 4 | Acetone | 0.3 | 1.1 | Crystal form III of maleate |
| 5 | IPA | 0.3 | | Crystal form II of maleate |
| 6 | IPAC | 0.4 | | Crystal forms II+III of maleate |
| 7 | n-BuOH | 0.3 | | Crystal form III of maleate |
| 8 | ACN | 0.3 | | Crystal form III of maleate |

| | | | | |
|---|---|---|---|---|
| Note: The "eq." in the table refers to the molar ratio of the acid to compound I. | | | | |

The 3 crystal forms of maleate obtained in the above experiments were subjected to PLM, DSC (Discovery DSC 250 (TA Instruments, US)), TGA (TGA 55 (TA Instruments, US)), XRPD, and ¹H-NMR. The relevant characterization results are shown in Table 6-3, FIG. 6-1 to FIG. 6-5, FIG. 7-1 to FIG. 7-5, and FIG. 8-1 to FIG. 8-5.

The crystal form I of maleate (group 1) was in the form of fine crystals. There was a weight loss of about 2.6% before 180 °C, and the ¹H-NMR analysis showed that the sample contained about 2.9% MeOH, so the weight loss observed in TGA was attributed to the removal of MeOH. The DSC profile showed an endothermic peak at a peak temperature of about 198 °C, which was caused by melting and concomitant decomposition. The XRPD spectra of the sample before and after heating to 180 °C were inconsistent (FIG. 6-6), so the crystal form I of maleate was a MeOH solvate.

The crystal form II of maleate (group 3) was in the form of agglomerated particles. There was no significant weight loss before 100 °C, and the ¹H-NMR analysis showed that the sample contained about 2.8% THF, so the weight loss observed in TGA was attributed to the desorption of THF. The DSC profile showed two endothermic peaks: a broad endothermic peak at a peak temperature of about 48 °C, which was attributed to the desorption of surface water, and an endothermic peak at a peak temperature of about 190 °C, which was caused by melting and concomitant decomposition. The XRPD spectra of the sample before and after heating to 160 °C were consistent (FIG. 7-6), so the crystal form II of maleate was an anhydrous crystal form.

The crystal form III of maleate (group 4) was in the form of fine crystals. There was no significant weight loss before 100 °C, and the ¹H-NMR analysis showed that the sample contained about 0.5% acetone. The DSC profile showed an endothermic peak at a peak temperature of about 197 °C, which was caused by melting and concomitant decomposition. The crystal form III of maleate was an anhydrous crystal form.

**Table 6-3 Solid state characterization results for maleate**

| **Group** | **Crystal form** | **DSC,** | **TGA** | **¹H-NMR** |
|---|---|---|---|---|
| | **Solvation** | **onset temperature/peak temperature of endothermic peak (°C), ΔH (J/g)** | **weight loss %/ @T (°C)** | |
| 1 | I MeOH solvate | 195/198, m/d | ∼2.6/RT-180 | 2.9% MeOH; about 1 eq. of acid |
| 3 | II anhydrous crystal form | 27/48, 14 | ∼0/RT-100 | 2.8% THF; about 1 eq. of acid |
| | | 185/190, m/d | | |
| 4 | III anhydrous crystal form | 195/197, m/d | ∼0/RT-150 | 0.5% acetone; about 1 eq. of acid |

| | | | | |
|---|---|---|---|---|
| Note: m/d: melting and concomitant decomposition; RT = room temperature | | | | |

### Example 7: Oxalate

At room temperature, 30 mg of compound I was weighed and added into a sample bottle, and then 0.6 mL of the selected solvent MeOH was added. An oxalic acid solid (5.9 mg, 1.1 eq.) was further added to prepare a suspension, and the suspension was stirred at room temperature overnight (about 21 °C, 16 h). Then, the suspension was filtered, and a sample (filter cake) was collected, dried under vacuum at 40 °C for about 4 h, and then subjected to XRPD characterization. Specific information and results are summarized in Table 7-1.

**Table 7-1. Preparation of oxalate**

| **Group** | **Solvent** | **Amount of oxalic acid used (eq.)** | **XRPD** |
|---|---|---|---|
| 1 | MeOH | 1.1 | Crystal form I of oxalate |

| | | | |
|---|---|---|---|
| Note: The "eq." in the table refers to the molar ratio of the acid to compound I. | | | |

In this example, one crystal form of oxalate was obtained, which was designated as a crystal form I of oxalate. The oxalate sample was subjected to characterization by PLM, DSC (Discovery DSC 250 (TA Instruments, US)), TGA (TGA 55 (TA Instruments, US)), XRPD, and ¹H-NMR. The relevant characterization results are shown in Table 7-2 and FIG. 9-1 to FIG. 9-5.

The crystal form I of oxalate was in the form of fine crystals (FIG. 9-4). There was a weight loss of about 4.1% at 60-160 °C, and the ¹H-NMR analysis showed that the sample contained 3.9% residual MeOH (FIG. 9-5), so the weight loss observed in TGA was attributed to the removal of MeOH. The DSC profile showed a broad endothermic peak at a peak temperature of about 127 °C, which was attributed to the removal of MeOH (FIG. 9-3). The IC results showed that the sample had an acid/base ratio of about 1/1 (Table 7-3). The crystal form I of oxalate was determined to be a MeOH solvate.

**Table 7-2. Solid state characterization results for crystal form I of oxalate**

| **Crystal form solvation** | **DSC, onset temperature/peak temperature of endothermic peak (°C), ΔH (J/g)** | **TGA weight loss %/ @T (°C)** | **¹H-NMR** | **IC** |
|---|---|---|---|---|
| I MeOH solvate | 87/127, 68 | ∼4.1/60-160 | 3.9% MeOH | About 1 eq. of acid |

**Table 7-3. IC data for crystal form I of oxalate**

| **Sample** | **Mass (mg)** | **Theoretical concentration (mM)** | **IC peak area (µS*min)** | **Result** |
|---|---|---|---|---|
| C₂O₄²⁻ standard solution (C₂O₄²⁻-STD) | NA | 0.5 | 6.75 | NA |
| Crystal form I of oxalate | 1.98 | 0.66 (calculated based on 1 eq.) | 7.79 | About 1 eq. of C₂O₄²⁻ |

### Example 8: Camphorsulfonate

At room temperature, 20 mg of compound I was weighed and added into a sample bottle, and then the corresponding solvent according to Table 8-1 (0.4 mL, 20 V) was added. Camphorsulfonic acid (9.2 mg, 1.0 eq.) was further added, and the suspension was stirred at room temperature for 5 days and then centrifuged. The solid sample was collected, blast-dried at room temperature for 24 h, and subjected to XRPD characterization. Specific information and results are shown in Table 8-1, Table 1', and FIG. 10-1:

**Table 8-1. Preparation of camphorsulfonate**

| **Group** | **Solvent** | **Amount of solvent used** | **Amount of camphorsulfonic acid used (eq.)** | **XRPD** |
|---|---|---|---|---|
| 1 | EtOH | 20V | 1 | Crystal form I of camphorsulfonate |
| 2 | ACN | | | Crystal form I of camphorsulfonate |
| 3 | THF | | | Crystal form I of camphorsulfonate |
| 4 | MEK | | | Crystal form I of camphorsulfonate |
| 5 | IPAC | | | Crystal form I of camphorsulfonate |

| | | | | |
|---|---|---|---|---|
| Note: The "eq." in the table refers to the molar ratio of the acid to compound I; the "V" in the table represents the ratio of the volume (mL) of the solvent to the mass (g) of compound I in the salt-forming system. | | | | |

**Table 1'. XRPD analysis of crystal form I of camphorsulfonate**

| **2θ/°** | **Relative intensity I/%** | **2θ/°** | **Relative intensity I/%** |
|---|---|---|---|
| 3.648 | 82.5 | 20.221 | 34.2 |
| 7.299 | 24.9 | 21.492 | 26.8 |
| 9.636 | 61.3 | 21.848 | 26.3 |
| 11.956 | 14.4 | 23.765 | 27.9 |
| 12.619 | 100 | 24.853 | 6.9 |
| 13.02 | 18.1 | 25.353 | 28.9 |
| 13.943 | 6.3 | 25.737 | 11.5 |
| 14.835 | 74.4 | 26.795 | 14.6 |
| 16.53 | 82.1 | 28.111 | 33.8 |
| 17.411 | 49.4 | 28.881 | 13.4 |
| 17.983 | 6.6 | 30.02 | 6.6 |
| 19.037 | 55.9 | 35.253 | 5.6 |

In this example, one crystal form of camphorsulfonate was obtained, which was designated as a crystal form I of camphorsulfonate. The camphorsulfonate sample was subjected to characterization by TGA (TG-DSC thermal analyzer), DSC (TG-DSC thermal analyzer), and ¹H-NMR. The relevant characterization results are shown in FIG. 10-2 and FIG. 10-3.

TGA showed that the crystal form I of camphorsulfonate had a weight loss of about 5% before 180 °C (FIG. 10-2). DSC showed two endothermic peaks: a broad endothermic peak at a temperature around 83 °C, which was probably caused by the dehydration of the sample, and an endothermic peak at around 198 °C, which was probably caused by the melting of the sample (FIG. 10-2). ¹H NMR showed that the salt-forming ratio of the free base and camphorsulfonic acid was 1/1, indicating no residual solvent (FIG. 10-3). The crystal form I of camphorsulfonate was a hydrate, containing about 2 eq. of water.

### Example 9: 2-Hydroxyethanesulfonate

At room temperature, 20 mg of compound I was weighed and added into a sample bottle, and then the selected solvent EtOH (0.4 mL, 20 V) was added. 2-Hydroxyethanesulfonic acid (5.8 mg, 85 wt%, 1.0 eq.) was further added, and the suspension was slurried at room temperature for 5 days and then centrifuged. The solid sample was collected, blast-dried at room temperature for 24 h, and subjected to XRPD characterization. Specific information and results as shown in Table 9-1, Table 2', and FIG. 11-1:

**Table 9-1. Preparation of 2-hydroxyethanesulfonate**

| **Group** | **Solvent** | **Amount of solvent used** | **Amount of hydroxyethanesulfonic acid used (eq.)** | **XRPD** |
|---|---|---|---|---|
| 1 | EtOH | 20V | 1 | Crystal form I of 2-hydroxyethanesulfonate |

| | | | | |
|---|---|---|---|---|
| Note: The "eq." in the table refers to the molar ratio of the acid to compound I; the "V" in the table represents the ratio of the volume (mL) of the solvent to the mass (g) of compound I in the salt-forming system. | | | | |

**Table 2'. XRPD analysis of crystal form I of 2-hydroxyethanesulfonate**

| **2θ/°** | **Relative intensity I/%** | **2θ/°** | **Relative intensity I/%** |
|---|---|---|---|
| 6.828 | 18 | 20.65 | 25.1 |
| 8.548 | 36.9 | 22.266 | 24.6 |
| 8.914 | 30.1 | 22.555 | 25.7 |
| 10.41 | 15.6 | 24.657 | 23.6 |
| 10.751 | 19.6 | 25.367 | 100 |
| 11.875 | 9.8 | 26.088 | 25.1 |
| 13.22 | 39.5 | 27.628 | 9.6 |
| 15.362 | 20.6 | 29.833 | 8.6 |
| 16.042 | 89.6 | 32.735 | 8.8 |
| 17.972 | 44.5 | 35.205 | 10.2 |
| 18.75 | 55.5 | 36.359 | 7.8 |
| 19.626 | 16.6 | | |

The XRPD test results for 2-hydroxyethanesulfonate are shown in FIG. 11-1. In this example, one crystal form of 2-hydroxyethanesulfonate was obtained in ethanol, which was designated as a crystal form I of 2-hydroxyethanesulfonate. The sample of the crystal form I of 2-hydroxyethanesulfonate was subjected to characterization by TGA (TG-DSC thermal analyzer), DSC (TG-DSC thermal analyzer), and ¹H-NMR. The relevant characterization results are shown in FIG. 11-2 and FIG. 11-3.

TGA showed that there was no weight loss before 150 °C, and there was a weight loss of about 5.4% before 300 °C (FIG. 11-2). DSC showed an exothermic peak at around 258 °C, which was probably caused by the decomposition of the sample (FIG. 11-2). ¹H NMR showed that the salt-forming ratio of the free base and hydroxyethanesulfonic acid was 1/1, indicating no residual solvent (FIG. 11-3).

### Example 10: Ethanesulfonate

### Example 10-1

At room temperature, 20 mg of compound I was weighed and added into a sample bottle, and then the corresponding solvent in Table 10-1 (0.4 mL, 20 V) was added. Ethanesulfonic acid (4.33 mg, 1.0 eq.) was further added, and the suspension was slurried at room temperature for 5 days and then centrifuged. The solid sample was collected, blast-dried at room temperature for 24 h, and subjected to XRPD characterization. Specific information and results are shown in Table 10-1.

**Table 10-1. Preparation of ethanesulfonate**

| **Group** | **Solvent** | **Amount of solvent used** | **Amount of ethanesulfonic acid used (eq.)** | **XRPD** |
|---|---|---|---|---|
| 1 | EtOH | 20V | 1 | Crystal form I of ethanesulfonate |
| 2 | ACN | | | Crystal form I of ethanesulfonate |
| 3 | MEK | | | Crystal form I of ethanesulfonate |
| 4 | IPAC | | | Crystal form II of ethanesulfonate |
| 5 | EtOH | 10V | 1.1 | Crystal form I of ethanesulfonate |

| | | | | |
|---|---|---|---|---|
| Note: The "eq." in the table refers to the molar ratio of the acid to compound I; the "V" in the table represents the ratio of the volume (mL) of the solvent to the mass (g) of compound I in the salt-forming system. | | | | |

In this experiment, 2 crystal forms of ethanesulfonate were obtained, which were designated as a crystal form I of ethanesulfonate and a crystal form II of ethanesulfonate, respectively. The 2 crystal forms of ethanesulfonate obtained were subjected to characterization by XRPD, TGA (TG-DSC thermal analyzer), DSC (TG-DSC thermal analyzer), ¹H-NMR, and PLM. The characterization results are shown in FIGs. 12-1-1, 12-2-1, 12-3-1, 12-3-5, 13-1 and 13-2, Table 4', and Table 5'. PLM showed that the crystal form I of ethanesulfonate was in the form of small-particle crystals (about 10 µm) with relatively high crystallinity. TGA showed the crystal form I of ethanesulfonate had a weight loss of about 1.4% before 230 °C, which was attributed to the removal of the solvent. DSC showed that the crystal form I of ethanesulfonate had an exothermic peak at around 265 °C, which was probably caused by the decomposition of the sample (FIG. 12-2-1). ¹H NMR of the crystal form I of ethanesulfonate showed that the salt-forming ratio of the free base and ethanesulfonic acid was 1/1, indicating about 1.3 wt% of residual ethanol (FIG. 12-3-1).

The crystal form II of ethanesulfonate had a weight loss of about 2.3% before 150 °C. The crystal form II of ethanesulfonate had two endothermic peaks and one exothermic peak; the endothermic peak at a temperature of around 85 °C was a broad peak and probably caused by the removal of the solvent in the sample, the endothermic peak at a temperature of around 177 °C was probably caused by the melting of the sample, and the exothermic peak at a temperature of around 222 °C was probably caused by the decomposition of the sample (FIG. 13-2). The crystal form II of ethanesulfonate was presumed to be a solvate.

**Table 4'. XRPD analysis of crystal form I of ethanesulfonate**

| **2θ/°** | **Relative intensity I/%** | **2θ/°** | **Relative intensity I/%** |
|---|---|---|---|
| 10.383 | 36.8 | 22.447 | 8.1 |
| 11.436 | 6.6 | 23.005 | 13.1 |
| 12.372 | 4.4 | 23.722 | 4 |
| 14.81 | 45.9 | 24.58 | 100 |
| 15.794 | 10.1 | 26.18 | 14.9 |
| 16.936 | 19.2 | 28.034 | 5.4 |
| 17.448 | 12.8 | 29.15 | 4.2 |
| 19.249 | 26.1 | 29.879 | 4.5 |
| 20.143 | 5.5 | 30.524 | 3.9 |
| 20.51 | 11.8 | 31.274 | 10.2 |
| 21.427 | 23.6 | 33.520 | 3.1 |
| 21.969 | 6.1 | | |

**Table 5'. XRPD analysis of crystal form II of ethanesulfonate**

| **2θ/°** | **Relative intensity I/%** | **2θ/°** | **Relative intensity I/%** |
|---|---|---|---|
| 4.489 | 54.9 | 19.181 | 63.4 |
| 9.007 | 65.6 | 20.049 | 23.3 |
| 10.634 | 100 | 20.507 | 13.2 |
| 11.419 | 16.5 | 21.48 | 8.6 |
| 12.771 | 10.9 | 22.66 | 36.4 |
| 13.549 | 38.3 | 25.17 | 20.6 |
| 14.732 | 35.8 | 26.929 | 29 |
| 16.226 | 34.8 | 27.706 | 16.3 |
| 17.528 | 80.7 | 28.776 | 9.7 |
| 18.051 | 31.3 | 31.642 | 8.4 |

### Example 10-2: Scale-Up Preparation of Crystal Form I of Ethanesulfonate

About 200 mg of free base was weighed and then dispersed in 2 mL of absolute ethanol. 43.3 mg (1.0 eq.) of ethanesulfonic acid was dissolved in 2 mL of ethanol, and the solution of ethanesulfonic acid in ethanol was added dropwise and slowly to the suspension of free base. The mixture was stirred at room temperature for 3 days and then filtered, and the filter cake was collected. The sample (filter cake) was dried under vacuum at 45 °C for about 24 h, and the dried sample was subjected to characterization by XRPD, TGA (TG-DSC thermal analyzer), DSC (TG-DSC thermal analyzer), and ¹H-NMR. The test results are shown in FIG. 12-1-2, FIG. 12-2-2, and FIG. 12-3-2.

The XRPD test results for the crystal form (Table 6') were consistent with those for the crystal form I of ethanesulfonate (Table 4'). TGA showed that there was a weight loss of about 1.3% before 230 °C. DSC showed an endothermic peak at around 264 °C, which was probably caused by the melting and concomitant decomposition of the sample, and an exothermic peak at around 269 °C, which was probably caused by the decomposition of the sample. ¹H NMR showed that the salt-forming ratio of the free base and ethanesulfonic acid was 1/1, indicating 1.3 wt% of residual ethanol. After slurring with methanol at 50 °C for 24 h, there was no residual ethanol solvent (FIG. 12-3-3). The solid sample obtained after slurring with methanol was subjected to XRPD test, and it was found that the crystal form remained unchanged (FIG. 12-3-4), still being the crystal form I of ethanesulfonate. Therefore, the crystal form I of ethanesulfonate was an anhydrous crystal form.

**Table 6'. XRPD analysis of crystal form I of ethanesulfonate obtained from scale-up experiment**

| **2θ/°** | **Relative intensity I/%** | **2θ/°** | **Relative intensity I/%** |
|---|---|---|---|
| 10.435 | 46.6 | 22.031 | 6 |
| 11.498 | 5.8 | 22.54 | 8 |
| 12.421 | 4.1 | 23.132 | 14.5 |
| 14.836 | 53.6 | 23.726 | 2.8 |
| 15.44 | 2.3 | 24.644 | 100 |
| 15.822 | 10.8 | 26.168 | 17.1 |
| 16.989 | 19.2 | 28.097 | 5.9 |
| 17.463 | 13.3 | 28.796 | 3.4 |
| 18.055 | 1.8 | 29.924 | 6.1 |
| 19.327 | 28.7 | 30.543 | 4.2 |
| 20.154 | 5.6 | 31.275 | 9.3 |
| 20.61 | 10.9 | 33.637 | 2.4 |
| 21.479 | 21.1 | 37.431 | 1.8 |

### Example 11: L-tartrate

According to Table 11-1, compound I and L-tartaric acid were weighed and added into a glass vial, and then the corresponding solvent in the corresponding volume was added for mixing to give a suspension. The suspension was stirred at a temperature of Ti for a period of t₁, and the solid was separated out by centrifugation and dried under vacuum at 50 °C to give a solid. The XRPD characterization results for the resulting solid are shown in Table 11-3. Compound I had poor solubility, and the tartrate was prepared by suspension and slurring. In some experiments, the salt formation was incomplete, resulting in the presence of free base crystal form.

**Table 11-1. Preparation parameters for L-tartrate**

| **Group** | **Mass of compound I/mg** | **Mass of L-tartaric acid/mg** | **Solvent** | **Volume of solvent/mL** | **T₁/°C** | **t₁ /days** | **XRPD** |
|---|---|---|---|---|---|---|---|
| 1 | 20 | 5.9 (1eq) | MEK | 0.5 (25V) | Room temperature | 6 | Crystal form I of L-tartrate |
| 2 | 20 | 5.9 (1eq) | EA | 0.5 (25V) | Room temperature | 6 | Crystal form I of L-tartrate |
| 3 | 250.0 | 74.2 (1eq) | MEK | 6.0 (24V) | Room temperature | 2 | Crystal form I of L-tartrate |
| 4 | 50 | 14.8 (1eq) | THF | 0.5 (10V) | 30 | 3 | Crystal form I of L-tartrate + free base crystal form I |
| 5 | 50 | 14.8 (1eq) | MEK | 1.75 (35V) | 30 | 3 | Crystal form I of L-tartrate + free base crystal form I |
| 6 | 50 | 14.8 (1eq) | MEK | 1.75 (35V) | 50 | 3 | Crystal form I of L-tartrate + free base crystal form I |
| 7 | 50 | 7.4 (0.5eq) | MEK | 1.75 (35V) | 30 | 3 | Crystal form I of L-tartrate + free base crystal form I |
| 8 | 50 | 2.0eq | MEK | 1.75 (35V) | 30 | 3 | Crystal form I of L-tartrate |

| | | | | | | | |
|---|---|---|---|---|---|---|---|
| Note: The "eq" in the table refers to the molar ratio of the acid to compound I; the "V" in the table represents the ratio of the volume (mL) of the solvent to the mass (g) of compound I in the salt-forming system. | | | | | | | |

In this example, 1 crystal form of L-tartrate was obtained, which was designated as a crystal form I of L-tartrate. The L-tartrate sample obtained (group 1) was subjected to characterization by XRPD, TGA (TA Q5000/5500 thermogravimetric analyzer), DSC (TA Q2000/2500 differential scanning calorimeter), and ¹H NMR. The relevant characterization results are summarized in Tables 11-2 and 11-3 and FIGs. 14-1, 14-2 and 14-3.

**Table 11-2. Solid state characterization results for crystal form I of L-tartrate**

| **Salt form** | **DSC, onset temperature/peak temperature of endothermic peak (°C)** | **TGA weight loss %/ @T (°C)** | **¹H-NMR** | **Molar ratio (acid/base)** |
|---|---|---|---|---|
| Crystal form I of L-tartrate (group 1) | 207/213 | ∼6.8/RT-150 | 0.7 % MEK | 1.0 |

The TGA results showed that the crystal form I of L-tartrate had a weight loss of about 6.8% when heated to 150 °C. The DSC results showed that the sample had 1 endothermic peak at around 207 °C (onset temperature). ¹H NMR was measured in DMSO-*d₆*, and the results showed that the molar ratio of the residual solvent MEK to compound I was 0.06 (corresponding to a weight loss of 0.7%), and the acid/base molar ratio of L-tartaric acid to compound I was 1.0.

**Table 11-3. XRPD analysis of crystal form I of L-tartrate in group 1**

| 2θ/° | Relative intensity/% | 2θ/° | Relative intensity/% | 2θ/° | Relative intensity/% |
|---|---|---|---|---|---|
| 3.2262 | 25.27 | 17.4438 | 2.90 | 29.3763 | 4.72 |
| 3.4813 | 23.71 | 17.6669 | 4.42 | 29.8907 | 2.62 |
| 3.6341 | 23.57 | 18.0439 | 8.19 | 30.0284 | 2.32 |
| 3.9116 | 28.74 | 18.3737 | 18.29 | 30.4969 | 1.27 |
| 4.4187 | 19.09 | 18.7344 | 31.03 | 31.2452 | 6.17 |
| 4.6467 | 19.01 | 19.4410 | 21.96 | 31.5310 | 1.82 |
| 4.9932 | 16.99 | 19.9128 | 3.58 | 32.1296 | 1.03 |
| 5.2019 | 15.21 | 20.7677 | 72.26 | 32.4678 | 2.63 |
| 5.3838 | 14.96 | 21.1183 | 49.44 | 32.8489 | 4.00 |
| 5.6639 | 13.88 | 21.7589 | 10.95 | 33.2050 | 5.22 |
| 5.9524 | 12.57 | 22.2696 | 18.60 | 33.4055 | 6.02 |
| 6.1286 | 12.74 | 22.6222 | 23.51 | 33.9481 | 2.53 |
| 6.8777 | 12.02 | 22.9289 | 10.90 | 34.2060 | 4.06 |
| 7.2321 | 24.58 | 23.0973 | 10.07 | 34.6836 | 5.26 |
| 7.7336 | 24.89 | 23.4336 | 16.69 | 35.1489 | 3.88 |
| 8.7593 | 8.67 | 23.5799 | 15.75 | 35.3217 | 3.99 |
| 9.1788 | 30.82 | 24.1799 | 3.55 | 36.0671 | 1.65 |
| 9.9792 | 5.51 | 24.5044 | 1.86 | 36.5829 | 1.49 |
| 10.1852 | 5.81 | 24.9586 | 8.16 | 36.9373 | 0.84 |
| 10.6629 | 5.87 | 25.2896 | 8.10 | 37.1153 | 1.48 |
| 11.6186 | 100.00 | 26.1522 | 1.54 | 37.2774 | 0.63 |
| 12.0541 | 24.75 | 26.4646 | 1.18 | 37.7422 | 0.39 |
| 12.5110 | 4.93 | 27.1084 | 9.71 | 37.9127 | 0.64 |
| 12.6676 | 4.03 | 27.2200 | 8.46 | 38.1772 | 1.09 |
| 12.9377 | 3.27 | 27.5135 | 5.55 | 38.3654 | 1.06 |
| 14.4600 | 24.23 | 28.0434 | 2.12 | 38.7626 | 0.66 |
| 15.5243 | 62.32 | 28.2058 | 2.67 | 38.9186 | 0.37 |
| 16.3570 | 2.57 | 28.4350 | 2.71 | 39.0808 | 0.53 |
| 16.7369 | 2.24 | 28.8294 | 1.76 | 39.6234 | 1.35 |
| 17.0581 | 2.55 | | | | |

### Example 12: 1,5-Naphthalenedisulfonate

About 20 mg of compound I and an equimolar amount (1 eq.) of 1,5-naphthalenedisulfonic acid were weighed and added into an HPLC vial, and then 0.5 mL of the corresponding solvent in Table 12-1 was added for mixing to give a suspension. The suspension was stirred at room temperature for about 6 days, and the solid was separated out by centrifugation and dried under vacuum at 50 °C overnight. The XRPD characterization results for the resulting solid are shown in Table 12-1.

**Table 12-1. Preparation of 1,5- naphthalenedisulfonate**

| **Group** | **Solvent** | **1,5- Naphthalenedisulfonic acid (eq.)** | **XRPD** |
|---|---|---|---|
| 1 | MeOH | 1 | Crystal form I of 1,5-naphthalenedisulfonate |
| 2 | MEK/H₂O (19:1, v/v) | | Crystal form II of 1,5-naphthalenedisulfonate |

| | | | |
|---|---|---|---|
| Note: The "eq." in the table refers to the molar ratio of the acid to compound I. | | | |

In this experiment, 2 crystal forms of 1,5-naphthalenedisulfonate were obtained, which were designated as a crystal form I of 1,5-naphthalenedisulfonate and a crystal form II of 1,5-naphthalenedisulfonate, respectively. The 1,5-naphthalenedisulfonate samples were subjected to characterization by TGA (TA Q5000/5500 thermogravimetric analyzer), DSC (TA Q2000/2500 differential scanning calorimeter), and ¹H NMR. The relevant characterization results are summarized in Tables 12-2, 12-3 and 12-4 and FIGs. 15-1, 15-2, 15-3, 16-1, 16-2 and 16-3.

**Table 12-2. Solid state characterization results for 1,5-naphthalenedisulfonate**

| **Salt form** | **DSC endothermic peak (peak temperature, °C)** | **TGA weight loss %/ @T (°C)** | **¹H-NMR** | **Molar ratio (acid/base)** |
|---|---|---|---|---|
| Crystal form I of 1,5-naphthalenedisulfonate | 84.0 | ∼8.3/RT-150 | No MeOH | 0.9 |
| | 119.6 | | | |
| Crystal form II of 1,5-naphthalenedisulfonate | 125.4 | ∼8.0/RT-180 | 0.6% MEK | 0.9 |
| | 170.5 | | | |
| | 217.3 | | | |

The TGA results showed that the crystal form I of 1,5-naphthalenedisulfonate had a weight loss of about 8.3% when heated to 150 °C. The DSC results showed that the crystal form I of 1,5-naphthalenedisulfonate had 2 endothermic peaks at 84.0 °C and 119.6 °C (peak temperatures), respectively (FIG. 15-2). ¹H NMR was measured in DMSO-*d6*, and the results showed that no residual solvent MeOH was detected, and the molar ratio of 1,5-naphthalenedisulfonic acid to compound I was 0.9.

The TGA results showed that the crystal form II of 1,5-naphthalenedisulfonate had a weight loss of about 8.0% when heated to 180 °C. The DSC results showed that the sample had 3 endothermic peaks at 125.4 °C, 170.5 °C and 217.3 °C (peak temperatures), respectively (FIG. 16-2). ¹H NMR was measured in DMSO-d6, and the results showed that the molar ratio of the solvent MEK to API was 0.06 (0.6 wt%), and the molar ratio of 1,5-naphthalenedisulfonic acid to compound I was 0.9.

**Table 12-3. XRPD analysis of crystal form I of 1,5-naphthalenedisulfonate**

| 2θ/° | Relative intensity/% | 2θ/° | Relative intensity/% | 2θ/° | Relative intensity/% |
|---|---|---|---|---|---|
| 3.0646 | 45.80 | 17.7116 | 26.52 | 30.7800 | 6.26 |
| 3.3027 | 40.81 | 17.9875 | 56.20 | 30.9580 | 4.60 |
| 3.4532 | 39.07 | 18.8498 | 10.94 | 31.2193 | 4.92 |
| 3.5243 | 37.34 | 19.4754 | 25.92 | 31.3518 | 4.57 |
| 3.7840 | 34.29 | 19.7983 | 27.40 | 31.5190 | 3.82 |
| 3.9698 | 32.91 | 20.0711 | 18.83 | 31.6741 | 2.66 |
| 4.7887 | 25.91 | 20.6380 | 16.53 | 31.9264 | 0.66 |
| 4.9539 | 24.70 | 20.8840 | 24.74 | 32.1677 | 0.60 |
| 6.8055 | 19.71 | 21.2920 | 10.01 | 32.5011 | 2.74 |
| 7.6788 | 100.00 | 21.6228 | 8.31 | 32.8330 | 1.45 |
| 7.9572 | 26.32 | 22.1388 | 14.48 | 32.9393 | 1.28 |
| 8.6356 | 24.08 | 22.4677 | 28.22 | 33.4849 | 1.10 |
| 9.1480 | 17.81 | 22.5806 | 30.17 | 33.7589 | 3.42 |
| 9.4893 | 10.34 | 22.7072 | 25.88 | 34.2082 | 2.01 |
| 9.7164 | 9.31 | 23.5282 | 76.48 | 34.3683 | 2.22 |
| 9.9865 | 11.75 | 24.0259 | 34.27 | 34.4932 | 2.35 |
| 10.4244 | 12.12 | 24.1982 | 25.47 | 34.6933 | 2.79 |
| 10.8753 | 30.05 | 24.8437 | 39.63 | 35.0150 | 2.96 |
| 11.1188 | 10.08 | 25.4955 | 23.75 | 35.5629 | 3.17 |
| 11.7229 | 24.07 | 26.0756 | 13.57 | 35.8184 | 2.25 |
| 11.8750 | 27.15 | 26.2501 | 12.06 | 35.9817 | 1.58 |
| 12.2951 | 8.96 | 26.7301 | 16.07 | 36.2941 | 0.98 |
| 12.7503 | 18.76 | 26.8864 | 15.35 | 36.5446 | 1.21 |
| 13.0719 | 9.67 | 27.0483 | 14.48 | 36.7791 | 2.11 |
| 13.6206 | 7.25 | 27.7563 | 8.52 | 37.1980 | 1.50 |
| 13.8301 | 6.23 | 28.3682 | 14.65 | 37.3466 | 1.48 |
| 14.5445 | 13.63 | 28.5349 | 13.51 | 37.7367 | 1.82 |
| 15.0315 | 14.61 | 28.6958 | 8.51 | 38.2358 | 0.68 |
| 15.3750 | 36.95 | 28.7784 | 7.88 | 38.3932 | 0.77 |
| 15.8241 | 12.44 | 29.2960 | 5.25 | 38.5741 | 1.85 |
| 16.2412 | 6.78 | 29.5306 | 4.19 | 39.4598 | 1.79 |
| 16.7841 | 24.16 | 30.0993 | 2.26 | 39.6440 | 0.79 |
| 17.2859 | 19.51 | | | 39.8030 | 0.24 |

**Table 12-4. XRPD analysis of crystal form II of 1,5-naphthalenedisulfonate**

| 2θ/° | Relative intensity/% | 2θ/° | Relative intensity/% | 2θ/° | Relative intensity/% |
|---|---|---|---|---|---|
| 3.1250 | 49.34 | 16.9372 | 19.74 | 28.4129 | 6.83 |
| 3.9469 | 33.28 | 17.2418 | 14.17 | 28.8049 | 3.28 |
| 4.3172 | 27.13 | 17.5877 | 5.14 | 29.1233 | 4.85 |
| 5.0927 | 24.14 | 17.9886 | 9.15 | 29.6787 | 9.15 |
| 5.5773 | 20.17 | 18.1161 | 8.11 | 29.8238 | 9.87 |
| 5.7418 | 19.96 | 18.5378 | 5.51 | 29.9535 | 7.21 |
| 5.9993 | 18.50 | 18.9267 | 17.74 | 30.4078 | 2.13 |
| 6.1875 | 19.96 | 19.2096 | 7.29 | 30.9615 | 6.99 |
| 6.3380 | 18.22 | 19.5104 | 18.13 | 31.2470 | 2.82 |
| 6.7591 | 15.38 | 19.7538 | 9.67 | 31.4257 | 4.05 |
| 7.0398 | 15.37 | 20.1466 | 17.26 | 31.5287 | 4.57 |
| 7.2771 | 14.26 | 20.4864 | 28.45 | 31.8481 | 6.20 |
| 7.7997 | 16.26 | 20.8907 | 26.98 | 32.1341 | 4.24 |
| 8.4157 | 29.01 | 21.1902 | 10.31 | 32.3129 | 3.12 |
| 8.6151 | 43.99 | 21.7180 | 14.99 | 32.4895 | 2.46 |
| 8.9583 | 11.60 | 22.0106 | 20.48 | 32.7895 | 3.56 |
| 9.5646 | 19.01 | 22.4366 | 19.61 | 33.1906 | 0.54 |
| 10.2273 | 49.42 | 22.9725 | 8.73 | 33.6847 | 1.80 |
| 10.6543 | 7.79 | 23.2306 | 9.03 | 34.0518 | 1.10 |
| 11.4489 | 23.31 | 23.4625 | 14.98 | 34.4056 | 0.78 |
| 11.9039 | 7.89 | 23.8102 | 30.72 | 34.5767 | 1.55 |
| 12.6851 | 36.32 | 24.0763 | 14.69 | 34.9096 | 1.46 |
| 13.1029 | 8.84 | 24.8994 | 7.61 | 35.3592 | 2.28 |
| 13.4259 | 7.74 | 25.4753 | 100.00 | 35.5005 | 2.42 |
| 13.9447 | 41.23 | 26.3860 | 19.70 | 36.1522 | 4.66 |
| 14.3975 | 26.30 | 26.6894 | 13.29 | 37.0212 | 0.41 |
| 14.7332 | 12.68 | 26.8822 | 10.81 | 37.5896 | 0.98 |
| 15.1584 | 9.13 | 27.2502 | 4.35 | 38.1115 | 0.35 |
| 15.4234 | 21.54 | 27.4258 | 4.52 | 38.9886 | 2.03 |
| 15.6216 | 26.92 | 28.0391 | 12.32 | 39.7738 | 1.06 |
| 16.0934 | 14.17 | 28.1748 | 10.55 | | |

### Example 13: Performance Testing

### 1. Hygroscopicity test

### 1.1. Hygroscopicity test on free base crystal form I, crystal form III of maleate, and crystal form I of ethanesulfonate

About 90 mg of the free base crystal form I, 80 mg of the crystal form III of maleate (group 8), and 80 mg of the crystal form I of ethanesulfonate (group 1) were each weighed and added into a tared DVS tray, and then subjected to hygroscopicity test and evaluation in their solid-state forms using a Vsorp (ProUmid GmbH & Co. KG, Germany) vapor sorption analyzer.

The DVS data showed that the free base crystal form I had a weight gain of 0.30%/0.46% in the range of 0.0% RH to 80%/90% RH, indicating that the free base crystal form I was slightly hygroscopic. The XRPD spectra of the free base crystal form I before and after the DVS test showed no changes, indicating that the crystal form remained unchanged. The detailed characterization results are shown in FIGs. 17-18.

The DVS data showed that the crystal form III of maleate (group 8) had a weight gain of 0.29%/0.37% in the range of 0.0% RH to 80%/90% RH, indicating that the crystal form III of maleate was slightly hygroscopic. The XRPD spectra of the crystal form III of maleate before and after the DVS test were consistent, indicating that the crystal form remained unchanged. The detailed characterization results are shown in FIGs. 19-20.

The DVS test results showed that the crystal form I of ethanesulfonate gained had a weight gain of about 0.3% from 0.0% RH to 80% RH, indicating that crystal form I of ethanesulfonate was slightly hygroscopic. The XRPD spectra of the crystal form I of ethanesulfonate before and after the DVS test showed no changes, indicating that the crystal form remained unchanged. The detailed characterization results are shown in FIGs. 21-22.

### 2. Solubility test

### 2.1. Solubility test on free base crystal form I and crystal form III of maleate

The free base crystal form I and the crystal form III of maleate (group 8) were tested for solubility in relevant biological media (SGF, FaSSIF, and FeSSIF) at 37 °C.

15 mg of each sample was weighed and dispersed in 3.0 mL of biologically relevant medium. The dispersion was shaken on a shaker at a rotation speed of 800 rpm at 37 °C. 1 mL of the dispersion was taken at 0.5 h, 2 h, and 24 h, and filtered. The filtrate was tested for solubility by Agilent HPLC 1260 series and for pH value by a pH meter. The filter cake was characterized for the crystal form by XRPD.

In the three biologically relevant media, the solubility of both the free base crystal form I and the crystal form III of maleate was relatively low (< 0.05 mg/mL). The free base crystal form I remained unchanged in FaSSIF and FeSSIF over 24 h, and underwent a crystal form transformation in SGF. The crystal form III of maleate underwent a crystal form transformation in FaSSIF, FeSSIF and SGF over 24 h. The relevant characterization results are summarized in Table 13-1.

**Table 13-1. Results of solubility in biologically relevant media**

| **Sample** | **Medium** | **Solubility (µg/mL)** | | | **XRPD (24 h)** | **pH** | |
|---|---|---|---|---|---|---|---|
| | | **0.5 h** | **2 h** | **24 h** | | **0 h** | **24 h** |
| Free base crystal form I | FaSSIF | 0.29 | 0.76 | 0.39 | Remained unchanged | 6.54 | 6.53 |
| | FeSSIF | 0.40 | 1.79 | 3.35 | Remained unchanged | 5.01 | 5.02 |
| | SGF | 11.50 | 6.69 | 8.57 | Crystal form transformation | 1.20 | 1.30 |
| Crystal form III of maleate | FaSSIF | 0.14 | 0.16 | 1.11 | Crystal form transformation | 6.54 | 5.33 |
| | FeSSIF | 0.74 | 2.47 | 3.90 | Crystal form transformation | 5.01 | 4.90 |
| | SGF | 24.00 | 2.18 | 6.45 | Crystal form transformation | 1.20 | 1.29 |

### 2.2. Solubility test on crystal form I of tartrate, crystal form I of ethanesulfonate, crystal form I of 2-hydroxyethanesulfonate, and crystal form I of sulfate

The crystal form I of tartrate, the crystal form I of ethanesulfonate, the crystal form I of 2-hydroxyethanesulfonate, and the crystal form I of sulfate were tested for solubility in relevant biological media (SGF, FaSSIF, and FeSSIF) and in purified water at 37 °C.

4 parallel samples of each of the crystal form I of tartrate, the crystal form I of ethanesulfonate, and the crystal form I of 2-hydroxyethanesulfonate (with about 5 mg per sample) were weighed and each placed into a 10 mL centrifuge tube, and then 5 mL of the corresponding biological medium solution was added to each tube; the tubes were capped, and the samples were suspended homogeneously. 10 parallel samples of the crystal form I of sulfate (with 1 mg per sample) were weighed and placed in centrifuge tubes, then 2 mL of the corresponding biological medium solution was added to each tube, and the samples were suspended homogeneously. The centrifuge tubes were shaken on a constant temperature incubator shaker (at a temperature of 37 °C, with a shaking frequency of 100 times/min). At 1 h, 2 h, 4 h, and 24 h, about 1 mL of the dispersion was taken, filtered, and then tested for solubility by calculating the concentration of the filtrate using Shimadzu LC-20Adxr.

In the three biological media, the solubility of the crystal form I of tartrate was highest in SGF, reaching up to around 50 µg/mL, and the solubility was lower in FeSSIF, being 16-26 µg/mL over a period of 0-24 h. The solubility was lowest in FaSSIF, being only 1-2 µg/mL within 24 h.

The solubility of the crystal form I of ethanesulfonate was highest in SGF, being around 100 µg/mL in the first 4 h, and reaching up to 72 µg/mL at 24 h. The solubility was lower in FeSSIF, being 85 µg/mL in the first 1 h, and only 12 µg/mL at 24 h. The solubility was lowest in FaSSIF, being only 2-3 µg/mL within 24 h.

The solubility of the crystal form I of 2-hydroxyethanesulfonate in SGF did not change significantly over time, remaining around 56 µg/mL. The solubility in FeSSIF was highest at 1 h, reaching up to 85 µg/mL, but was only 28 µg/mL at 24 h. The solubility data in FaSSIF was close to that of ethanesulfonate, being 3-5 µg/mL.

The solubility of the crystal form I of sulfate in SGF was 24.57 µg/mL at 24 h, and the crystal form was not detected in other biological medium solutions and in purified water.

In the solubility test on the crystal form I of tartrate, the crystal form I of ethanesulfonate, and the crystal form I of 2-hydroxyethanesulfonate in three biological media, the pH of all solutions did not change significantly. However, the XRPD spectra of the crystal form I of tartrate, the crystal form I of ethanesulfonate, and the crystal form I of 2-hydroxyethanesulfonate in the three biological media all changed, with a certain degree of decrease in crystallinity observed; in particular, the crystal form I of ethanesulfonate became almost amorphous in FeSSIF. The transformation from crystal form to amorphous form or the decrease in crystallinity in biological media is beneficial to increase the exposure of the active ingredient *in vivo.*

The XRPD overlay spectra of the crystal form I of ethanesulfonate before and after the solubility test in the biological medium FeSSIF is shown in FIG. 23.

The relevant characterization results are summarized in Table 13-2.

**Table 13-2. Results of solubility in biologically relevant media**

| **Sample** | **Biological medium** | **Solubility (µg/mL)** | | | | **XRPD (24 h)** |
|---|---|---|---|---|---|---|
| | | **1h** | **2h** | **4h** | **24h** | |
| **Crystal form I of tartrate** | SGF(pH1.2) | 46 | 50 | 50 | 51 | Changed, decreased in crystallinity |
| | FeSSIF(pH5.0) | 26 | 21 | 19 | 16 | Changed, decreased in crystallinity |
| | FaSSIF(pH6.5) | 2 | 1 | 2 | 1 | Changed, decreased in crystallinity |
| | Purified water | 0 | 0 | 0 | 0 | Remained unchanged |
| **Crystal form I of ethanesulfonate** | SGF(pH1.2) | 101 | 101 | 97 | 72 | Changed, decreased in crystallinity |
| | FeSSIF(pH5.0) | 85 | 76 | 61 | 12 | Changed, becoming almost amorphous |
| | FaSSIF(pH6.5) | 3 | 3 | 3 | 2 | Changed, decreased in crystallinity |
| | Purified water | 0 | 0 | 0 | 0 | Remained unchanged |
| **Crystal form I of 2-hydroxyethanesulf onate** | SGF(pH1.2) | 55 | 56 | 56 | 57 | Changed, decreased in crystallinity |
| | FeSSIF(pH5.0) | 85 | 76 | 62 | 28 | Changed, decreased in crystallinity |
| | FaSSIF(pH6.5) | 4 | 5 | 3 | 3 | Changed, decreased in crystallinity |
| | Purified water | 0 | 0 | 0 | 0 | Remained unchanged |
| **Crystal form I of sulfate** | SGF(pH1.2) | - | - | - | 24.57 | - |
| | FeSSIF(pH5.0) | - | - | - | 0 | - |
| | FaSSIF(pH6.5) | - | - | - | 0 | - |
| | Purified water | - | - | - | 0 | - |

### 3. Solid-state stability test

### 3.1. Solid-state stability test on free base crystal form I, crystal form III of maleate, and crystal form I of ethanesulfonate

15 mg of each of the free base crystal form I, the crystal form III of maleate, and the crystal form I of ethanesulfonate was taken and placed under conditions shown in Table 13-3. The samples after being placed for 0 days, 1 week, 2 weeks and 30 days were taken and each dissolved in a diluent to prepare a solution at about 1.0 mg/mL, which was then tested for chemical stability by HPLC analysis using Agilent HPLC 1260 series. The solid samples after being placed for 2 weeks and 30 days were tested for physical stability by XRPD analysis.

The relevant characterization results are summarized in Table 13-3. The stability results showed that the free base crystal form I was substantially stable in physical and chemical properties under the four conditions of 60 °C/sealed, 60 °C/sealed/N₂, 40 °C/75% RH (open), and RT/sealed for 2 weeks, but the crystal form III of maleate had slight degradation. The crystal form I of ethanesulfonate was substantially stable in physical and chemical properties under the conditions of 40 °C/75% RH (open) and 60 °C/sealed.

**Table 13-3. Evaluation results of stability of free base crystal form I, crystal form III of maleate, and crystal form I of ethanesulfonate**

| **Sample** | **Purity (%), 230 nm** | | | | | | | | | | **XRPD** | |
|---|---|---|---|---|---|---|---|---|---|---|---|---|
| | **Day 0** | **RT/sealed** | **40 °C/75% RH (open)** | | | **60 °C/sealed** | | | **60 °C/sealed/N₂** | | | |
| | | **Week 2** | **Week 1** | **Week 2** | **Day 30** | **Week 1** | **Week 2** | **Day 30** | **Week 1** | **Week 2** | **Week 2 ^{[1]}** | **Day 30 ^{[2]}** |
| Free base crystal form I | 97.90 | 97.93 | 97.82 | 97.88 | - | 97.82 | 97.85 | - | 97.83 | 97.84 | Not changed | - |
| Crystal form III of maleate | 98.11 | 98.02 | 98.00 | 97.92 | - | 97.96 | 97.85 | - | 97.95 | 97.97 | Not changed | - |
| Crystal form I of ethanesulfonate | 99.48 | - | - | 99.49 | 99.48 | - | 99.50 | 99.49 | - | - | - | Not changed |

| | | | | | | | | | | | | |
|---|---|---|---|---|---|---|---|---|---|---|---|---|
| Note: [1] The "week 2" refers to the XRPD results after placement under the conditions of RT/sealed, 40 °C/75% RH (open), 60 °C/sealed, or 60 °C/sealed/N₂ for 2 weeks; [2] the "day 30" refers to the XRPD results after placement under the conditions of 40 °C/75% RH (open) or 60 °C/sealed for 30 days. | | | | | | | | | | | | |

### 3.2. Solubility test on crystal form I of tartrate, crystal form I of ethanesulfonate, and crystal form I of 2-hydroxyethanesulfonate

Two samples of each of the crystal form I of tartrate, the crystal form I of ethanesulfonate, and the crystal form I of 2-hydroxyethanesulfonate (with about 25 mg per sample) were weighed and each added into a 2 mL liquid phase vial. The vials were sealed with tin foil and then separately placed under the conditions of 40 °C temperature/75% humidity or 25 °C temperature/60% humidity for about 3 months. The samples after being placed for 0 months, 2 weeks, 1 month and 3 months were taken and each dissolved in a diluent to prepare a solution at about 1.0 mg/mL, which was then tested for chemical stability by HPLC analysis using Shimadzu LC-20ADxr.

The relevant characterization results are summarized in Table 13-4. The stability results showed that the crystal form I of tartrate was substantially stable in chemical properties under the two conditions of 25 °C/60% humidity (sealed and protected from light) and 40 °C/75% humidity (sealed and protected from light); the crystal form I of ethanesulfonate and the crystal form I of 2-hydroxyethanesulfonate were substantially stable in chemical properties under the conditions of 40 °C/75% humidity (sealed and protected from light).

**Table 13-4. Solubility of crystal form I of tartrate, crystal form I of ethanesulfonate, and crystal form I of 2-hydroxyethanesulfonate**

| Evaluation results | | | | | | | | | | |
|---|---|---|---|---|---|---|---|---|---|---|
| | | **Purity (%), 230 nm** | | | | | | | | |
| **Condition** | | **25 °C/60% humidity, sealed and protected from light** | | | **40 °C/75% humidity, sealed and protected from light** | | | | | |
| **Sample** | **Crystal form I of tartrate** | | | **Crystal form I of tartrate** | | | **Crystal form I of ethanesulfonate** | | **Crystal form I of 2-hydroxyethanesul fonate** | |
| **Time** | **Day 0** | **Month 1** | **Month 3** | **Month 1** | **Month 2** | **Month 3** | **Day 0** | **Day 13** | **Day 0** | **Day 13** |
| Compound I | 98.98 | 99.00 | 99.02 | 98.95 | 99.18 | 99.04 | 98.68 | 98.69 | 98.76 | 98.7 |

### Example 14: Assay on Biological Activity of Compound

### 1. In-vitro evaluation experiment on kinase activity

### 1) In-vitro evaluation of ROCK2 kinase activity

ROCK2 activity screening: ROCK2 activity was detected using a 96-well (Cisbio) time-resolved fluorometric assay. The assay for ROCK2 was performed in the following assay buffer: 5 mM MgCl₂ (Sigma), 1 mM DTT (Sigma), and 1× kinase buffer. The kinase buffer was used for dilution. 7.5 µL of ROCK2 kinase (Invitrogen, PV3759) was first added to a 96-well microplate to achieve a final concentration of 0.4 ng/µL, and then 0.25 µL of compound I (DMSO content: 1% (volume fraction)) was added. The mixture was incubated at room temperature for 0.5 h. To start the reaction, the kinase buffer was used to mix ATP (Aladdin) and the substrate STK-substrate 2-biotin. 7.5 µL of the mixed solution was added to the microplate to achieve final concentrations of 6.739 µM and 1 µM. The resulting mixture was incubated at room temperature for another 2 h. 5 mL of detection buffer was mixed with STK antibody-Cryptate, and then an appropriate volume of the mixture was mixed with an equal volume of streptavidin-XL665. 10 µL of the resulting mixture was added to the microplate to stop the reaction. After further incubation for about 1 h, the microplate was read on a Molecular Devices Spectra Max i3x multifunctional microplate reader. The kinase buffer, the STK-substrate 2-biotin, the detection buffer, the STK antibody-Cryptate, and the streptavidin-XI,665 were all from HTRF KinEASE-STK kit (Cisbio, 1000 tests, 61GSTXLA).

### 2) In-vitro evaluation of ROCK1 kinase selectivity

ROCK1 activity was detected using a 96-well (Cisbio) time-resolved fluorometric assay. The assay for ROCK1 was performed in the following assay buffer: 5 mM MgCl₂ (Sigma), 1 mM DTT (Sigma), and 1× kinase buffer. The kinase buffer was used for dilution. 7.5 µL of ROCK1 kinase (Invitrogen) was first added to a 96-well microplate to achieve a final concentration of 0.4 ng/µL, and then 0.25 µL of the compound (DMSO content: 1%) was added. The mixture was incubated at room temperature for 0.5 h. To start the reaction, the kinase buffer was also used to mix ATP (Aladdin) and the substrate STK-substrate 2-biotin. 7.5 µL of the mixed solution was added to the microplate to achieve final concentrations of 3.53 µM and 1 µM. The resulting mixture was incubated at room temperature for another 2 h. 5 mL of detection buffer was mixed with STK antibody-Cryptate, and then an appropriate volume of the mixture was mixed with an equal volume of streptavidin-XL665.

10 µL of the resulting mixture was added to stop the reaction. After further incubation for about 1 h, the microplate was read on a Molecular Devices SpectraMax i3x multifunctional microplate reader. The kinase buffer, the STK-substrate 2-biotin, the detection buffer, the STK antibody-Cryptate, and the streptavidin-XI,665 were all from HTRF KinEASE-STK kit (Cisbio, 1000 tests, 61GSTXLA). The *in vitro* evaluation experiment on kinase activity showed that compound I had an IC₅₀ value of 36.48 nM for ROCK2 and an IC₅₀ value of > 4.5 µM for ROCK1, demonstrating excellent ROCK inhibitory activity; particularly, it showed relatively good selective inhibition against ROCK2 kinase.

### 2. In-vitro cytotoxicity Assay

*In-vitro* cytotoxicity assay for compound I was performed in HepG2 cells using the CCK-8 method. HepG2 cells (Beina Bio) in the logarithmic growth phase were collected, the concentration of cell suspension was adjusted, and then the cells were plated on a 96-well cell culture plate at 50000 cells/well. The cells were then incubated in a cell incubator (5%, 37 °C) overnight. After 80%-90% cell confluence in the plate was achieved, medium change was performed, and then the test compound at each concentration gradient or vehicle (DMSO) was added. The resulting mixture was incubated in the cell incubator (5%, 37 °C) for 48 h. After the treatment was completed, the medium in the plate was discarded. The plate was washed twice with PBS, and a CCK-8 working solution (Beyotime) was added at 100 µL per well. The resulting mixture was then incubated at 37 °C for 1.5 h in the dark. The absorbance at OD_{450 nm} was measured for each well on a microplate reader, and the CC₅₀ value of each compound was analyzed and calculated.

The results showed that the CC₅₀ of compound I was consistently greater than 100 µM, indicating relatively good safety.

### 3. Pharmacokinetic study in rats

Compound I, 2-hydroxyethanesulfonate of compound I, ethanesulfonate (crystal form I) of compound I, tartrate of compound I, and sulfate of compound I were each accurately weighed, and the required volume of vehicle was added. The mixture was stirred for 5 min, then sonicated below 25 °C until no large particles were visible to the naked eye, and further stirred for 30 min until a homogeneous suspension was obtained. The suspension was then used for administration.

Male SPF-grade SD rats (Beijing Vital River Laboratory Animal Technology Co., Ltd.) were given compound I, the 2-hydroxyethanesulfonate of compound I, the ethanesulfonate of compound I (crystal form I), the tartrate of compound I, and the sulfate of compound I by single intragastric administration after an acclimation period (with free access to water and food). Blood was collected from the orbital venous plexus of the rats at 0.167 h, 0.5 h, 1 h, 2 h, 3 h, 4 h, 6 h, 9 h, 12 h, and 24 h after administration. Each blood sample was centrifuged at 3500 ×g for 10 min at 4 °C. The supernatant was then collected, and the concentration of compound I in the plasma was measured by LC-MS/MS (AB Sciex TRIPLE QUAD 3500). The data were analyzed using a non-compartmental model in WinNonlin (version 5.2.1 Pharsight, Mountain View, CA) to obtain PK parameters. The experimental data are shown in Table 14-1, indicating that the *in-vivo* exposure levels of the salt forms of compound I were all significantly higher than those of compound I.

**Table 14-1. PK experiment on compound disclosed herein in rats**

| **Sample** | **Vehicle** | **Solution state** | **Administrati on dose (mg/kg)*** | **Administrati on volume (mL/kg)** | **Cmax (ng/mL)** | **AUCₗₐₛₜ (ng/mL*hr)** |
|---|---|---|---|---|---|---|
| 2-Hydroxyethane sulfonate | 0.5% CMC-Na aqueous solution | Suspension | 10 | 10 | 827 | 9449 |
| Ethanesulfonate | 0.5% CMC-Na aqueous solution | Suspension | 10 | 10 | 1153 | 13416 |
| Tartrate | 0.5% CMC-Na aqueous solution | Suspension | 10 | 10 | 575 | 5452 |
| Sulfate | 5%TPGS | Suspension | 10 | 10 | 1052 | 13429 |
| Compound I | 0.5% CMC-Na aqueous solution | Suspension | 10 | 10 | 251 | 2036 |

In the table: the administration dose marked with * refers to the amount calculated based on compound I.

The embodiments of the present disclosure have been described above. However, the present disclosure is not limited to the embodiments described above. Any modification, equivalent replacement, improvement, and the like made without departing from the spirit and principle of the present disclosure shall fall within the protection scope of the present disclosure.

## Claims

1. A salt of compound I, wherein the salt is an acid addition salt; for example, the salt is an acid addition salt of compound I with any one of the following acids: hydrochloric acid, sulfuric acid, p-toluenesulfonic acid, benzenesulfonic acid, maleic acid, tartaric acid (comprising L-tartaric acid or R-tartaric acid), oxalic acid, ethanesulfonic acid, 2-hydroxyethanesulfonic acid, camphorsulfonic acid, and 1,5-naphthalenedisulfonic acid, preferably with sulfuric acid, tartaric acid (comprising L-tartaric acid or R-tartaric acid), ethanesulfonic acid, or 2-hydroxyethanesulfonic acid;

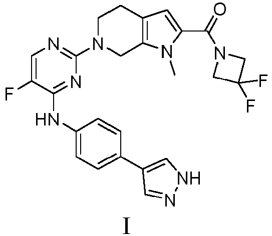

2. The salt as claimed in claim 1, wherein a hydrochloride of compound I is an acid addition salt of compound I with hydrochloric acid, wherein preferably, the molar ratio of compound I to the hydrochloric acid is 1:(0.9-1.2);
preferably, a sulfate of compound I is an acid addition salt of compound I with sulfuric acid, wherein preferably, the molar ratio of compound I to the sulfuric acid is 1:(0.4-0.6);
preferably, a *p*-toluenesulfonate of compound I is an acid addition salt of compound I with *p-*toluenesulfonic acid, wherein preferably, the molar ratio of compound I to the *p*-toluenesulfonic acid is 1:(0.9-1.2);
preferably, a maleate of compound I is an acid addition salt of compound I with maleic acid, wherein preferably, the molar ratio of compound I to the maleic acid is 1:(0.9-1.2);
preferably, an oxalate of compound I is an acid addition salt of compound I with oxalic acid, wherein preferably, the molar ratio of compound I to the oxalic acid is 1:(0.9-1.2);
preferably, a camphorsulfonate of compound I is an acid addition salt of compound I with camphorsulfonic acid, wherein preferably, the molar ratio of compound I to the camphorsulfonic acid is 1:(0.9-1.2);
preferably, a 2-hydroxyethanesulfonate of compound I is an acid addition salt of compound I with 2-hydroxyethanesulfonic acid, wherein preferably, the molar ratio of compound I to the 2-hydroxyethanesulfonic acid is 1:(0.9-1.2);
preferably, an ethanesulfonate of compound I is an acid addition salt of compound I with ethanesulfonic acid, wherein preferably, the molar ratio of compound I to the ethanesulfonic acid is 1:(0.9-1.2);
preferably, a tartrate of compound I is an acid addition salt of compound I with tartaric acid, wherein preferably, the molar ratio of compound I to the tartaric acid is 1:(0.9-1.2);
preferably, a 1,5-naphthalenedisulfonate of compound I is an acid addition salt of compound I with 1,5-naphthalenedisulfonic acid, wherein preferably, the molar ratio of compound I to the 1,5-naphthalenedisulfonic acid is 1:(0.8-1.1);
preferably, a benzenesulfonate of compound I is an acid addition salt of compound I with benzenesulfonic acid, wherein preferably, the molar ratio of compound I to the benzenesulfonic acid is 1:(0.9-1.2);
preferably, the salt of compound I comprises water of crystallization or does not comprise water of crystallization (e.g., the salt of compound I is an organic solvate);
preferably, the salt of compound I is in an amorphous form or a crystal form.

3. A free base crystal form of compound I as claimed in claim 1, wherein
preferably, the free base crystal form is a free base crystal form I;
preferably, by X-ray powder diffraction using Cu-Kα radiation, the free base crystal form I has characteristic peaks at 2θ angles of 13.1±0.2° and 20.5±0.2°, further has a characteristic peak at a 2θ angle of 8.4±0.2°, and still further has characteristic peaks at 2θ angles of 6.2±0.2°, 6.5±0.2°, 10.9±0.2°, 12.3±0.2°, 14.1±0.2°, 14.4±0.2°, 24.2±0.2°, and 25.3±0.2°;
preferably, the free base crystal form I has an X-ray powder diffraction pattern substantially as shown in FIG. 1-1;
preferably, by X-ray powder diffraction using Cu-Kα radiation, the free base crystal form I has characteristic peaks at 2θ angles as shown in FIG. 1-2, with an error range of ±0.2°;
preferably, the free base crystal form I is an anhydrate.

4. A crystal form of the salt of compound I as claimed in claim 1, being selected from crystal forms of the acid addition salts of compound I, wherein
preferably, the crystal form of the salt is selected from one, two, or more of a crystal form of hydrochloride, a crystal form of sulfate, a crystal form of *p*-toluenesulfonate, a crystal form of benzenesulfonate, a crystal form of maleate, a crystal form of oxalate, a crystal form of camphorsulfonate, a crystal form of 2-hydroxyethanesulfonate, a crystal form of ethanesulfonate, a crystal form of tartrate, and a crystal form of 1,5-naphthalenedisulfonate of compound I described above;
preferably, the crystal form of the salt comprises or does not comprise a solvent 1, wherein, for example, the solvent 1 is selected from an organic solvent 1 or water;
preferably, the organic solvent 1 is selected from one, two, or more of methanol, ethanol, isopropanol, butanol, acetone, butanone, ethyl acetate, isopropyl acetate, methyl *tert*-butyl ether, dichloromethane, acetonitrile, tetrahydrofuran, n-heptane, dimethylsulfoxide, 2-methyltetrahydrofuran, and chloroform;
preferably, the water is water of crystallization or non-crystal water;
preferably, the crystal form of hydrochloride is a crystal form I of hydrochloride;
preferably, by X-ray powder diffraction using Cu-Kα radiation, the crystal form I of hydrochloride has characteristic peaks at 2θ angles of 13.1±0.2°, 22.6±0.2°, 23.0±0.2°, and 24.4±0.2°, further has characteristic peaks at 2θ angles of 8.0±0.2°, 8.3±0.2°, 18.2±0.2°, 18.7±0.2°, and 30.3±0.2°, and still further has characteristic peaks at 2θ angles of 15.8±0.2°, 16.2±0.2°, and 21.5±0.2°;
preferably, the crystal form I of hydrochloride has an X-ray powder diffraction pattern substantially as shown in FIG. 2-1;
preferably, by X-ray powder diffraction using Cu-Kα radiation, the crystal form I of hydrochloride has characteristic peaks at 2θ angles as shown in FIG. 2-2, with an error range of ±0.2°;
preferably, the crystal form I of hydrochloride is an anhydrate;
preferably, the crystal form of sulfate is a crystal form I of sulfate;
preferably, by X-ray powder diffraction using Cu-Kα radiation, the crystal form I of sulfate has characteristic peaks at 2θ angles of 8.4±0.2°, 11.0±0.2°, 13.7±0.2°, and 19.3±0.2°, further has characteristic peaks at 2θ angles of 5.3±0.2°, 16.2±0.2°, and 18.0±0.2°, and still further has characteristic peaks at 2θ angles of 10.6±0.2°, 14.5±0.2°, 17.0±0.2°, 18.8±0.2°, 19.6±9.2°, 22.3±0.2°, and 24.9±0.2°;
preferably, the crystal form I of sulfate has an X-ray powder diffraction pattern substantially as shown in FIG. 3-1;
preferably, by X-ray powder diffraction using Cu-Kα radiation, the crystal form I of sulfate has characteristic peaks at 2θ angles as shown in FIG. 3-2, with an error range of ±0.2°;
preferably, the crystal form I of sulfate is a hydrate;
preferably, the crystal form of *p*-toluenesulfonate is a crystal form I of*p*-toluenesulfonate;
preferably, by X-ray powder diffraction using Cu-Kα radiation, the crystal form I of p-toluenesulfonate has characteristic peaks at 2θ angles of 5.1±0.2° and 26.1±0.2°, further has characteristic peaks at 2θ angles of 11.8±0.2° and 26.5±0.2°, and still further has characteristic peaks at 2θ angles of 10.2±0.2°, 11.0±0.2°, 18.9±0.2°, 19.2±0.2°, and 21.0±0.2°;
preferably, the crystal form I of *p*-toluenesulfonate has an X-ray powder diffraction pattern substantially as shown in FIG. 4-1;
preferably, by X-ray powder diffraction using Cu-Kα radiation, the crystal form I of *p-*toluenesulfonate has characteristic peaks at 2θ angles as shown in FIG. 4-2, with an error range of ±0.2°;
preferably, the crystal form I of *p*-toluenesulfonate is a hydrate;
preferably, the crystal form benzenesulfonate is a crystal form I of benzenesulfonate; by X-ray powder diffraction using Cu-Kα radiation, the crystal form I of benzenesulfonate has characteristic peaks at 2θ angles of 6.0±0.2° and 12.0±0.2°, further has characteristic peaks at 2θ angles of 13.2±0.2°, 18.2±0.2°, and 22.2±0.2°, and still further has characteristic peaks at 2θ angles of 8.7±0.2°, 17.4±0.2°, 18.6±0.2°, 19.4±0.2°, 29.3±9.2°, 24.2±0.2°, and 30.5±0.2°;
preferably, the crystal form I of benzenesulfonate has an X-ray powder diffraction pattern substantially as shown in FIG. 5-1;
preferably, by X-ray powder diffraction using Cu-Kα radiation, the crystal form I of benzenesulfonate has characteristic peaks at 2θ angles as shown in FIG. 5-2, with an error range of ±0.2°;
preferably, the crystal form I of benzenesulfonate is a hydrate;
preferably, the crystal form of maleate comprises a crystal form I of maleate, a crystal form II of maleate, and a crystal form III of maleate;
preferably, by X-ray powder diffraction using Cu-Kα radiation, the crystal form I of maleate has a characteristic peak at a 2θ angle of 24.8±0.2°, further has characteristic peaks at 2θ angles of 12.0±0.2° and 20.5±0.2°, and still further has characteristic peaks at 2θ angles of 9.3±0.2°, 13.2±0.2°, 17.4±0.2°, and 20.0±0.2°;
preferably, the crystal form I of maleate has an X-ray powder diffraction pattern substantially as shown in FIG. 6-1;
preferably, by X-ray powder diffraction using Cu-Kα radiation, the crystal form I of maleate has characteristic peaks at 2θ angles as shown in FIG. 6-2, with an error range of ±0.2°; preferably, the crystal form I of maleate is a solvate;
preferably, by X-ray powder diffraction using Cu-Kα radiation, the crystal form II of maleate has characteristic peaks at 2θ angles of 11.0±0.2°, 14.4±0.2°, 16.2±0.2°, and 16.8±0.2°, further has characteristic peaks at 2θ angles of 5.4±0.2°, 12.1±0.2°, 13.4±0.2°, 17.2±0.2°, 20.3±0.2°, and
20.6±0.2°, and still further has characteristic peaks at 2θ angles of 7.2±0.2°, 17.8±0.2°, and 25.0±0.2°; preferably, the crystal form II of maleate has an X-ray powder diffraction pattern substantially as shown in FIG. 7-1;
preferably, by X-ray powder diffraction using Cu-Kα radiation, the crystal form II of maleate has characteristic peaks at 2θ angles as shown in FIG. 7-2, with an error range of ±0.2°;
preferably, the crystal form II of maleate is an anhydrate;
preferably, by X-ray powder diffraction using Cu-Kα radiation, the crystal form III of maleate has characteristic peaks at 2θ angles of 14.6±0.2°, 16.9±0.2°, and 25.8±0.2°, further has characteristic peaks at 2θ angles of 5.3±0.2°, 10.7±0.2°, and 26.3±0.2°, and still further has characteristic peaks at 2θ angles of 12.4±0.2°, 16.1±0.2°, 19.2±0.2°, and 20.2±0.2°;
preferably, the crystal form III of maleate has an X-ray powder diffraction pattern substantially as shown in FIG. 8-1;
preferably, by X-ray powder diffraction using Cu-Kα radiation, the crystal form III of maleate has characteristic peaks at 2θ angles as shown in FIG. 8-2, with an error range of ±0.2°;
preferably, the crystal form III of maleate is an anhydrate;
preferably, the crystal form of oxalate is a crystal form I of oxalate; by X-ray powder diffraction using Cu-Kα radiation, the crystal form I of oxalate has characteristic peaks at 2θ angles of 18.4±0.2° and 24.3±0.2°, further has characteristic peaks at 2θ angles of 10.6±0.2° and 15.2±0.2°, and still further has characteristic peaks at 2θ angles of 7.6±0.2°, 11.2±0.2°, and 12.6±0.2°;
preferably, the crystal form I of oxalate has an X-ray powder diffraction pattern substantially as shown in FIG. 9-1;
preferably, by X-ray powder diffraction using Cu-Kα radiation, the crystal form I of oxalate has characteristic peaks at 2θ angles as shown in FIG. 9-2, with an error range of ±0.2°;
preferably, the crystal form I of oxalate is a solvate;
preferably, the crystal form of camphorsulfonate is a crystal form I of camphorsulfonate; preferably, by X-ray powder diffraction using Cu-Kα radiation, the crystal form I of camphorsulfonate has characteristic peaks at 2θ angles of 3.6±0.2°, 12.6±0.2°, and 16.5±0.2°, further has characteristic peaks at 2θ angles of 9.6±0.2°, 14.8±0.2°, 17.4±0.2°, and 19.0±0.2°, and still further has characteristic peaks at 2θ angles of 7.3±0.2°, 20.2±0.2°, 21.5±0.2°, 21.8±0.2°, 23.8±0.2°, 25.4±0.2°, and 28.1±0.2°;
preferably, the crystal form I of camphorsulfonate has an X-ray powder diffraction pattern substantially as shown in FIG. 10-1;
preferably, the crystal form I of camphorsulfonate is a hydrate;
preferably, the crystal form of 2-hydroxyethanesulfonate is a crystal form I of 2-hydroxyethanesulfonate; by X-ray powder diffraction using Cu-Kα radiation, the crystal form I of 2-hydroxyethanesulfonate has characteristic peaks at 2θ angles of 16.0±0.2° and 25.4±0.2°, further has characteristic peaks at 2θ angles of 18.0±0.2° and 18.8±0.2°, still further has characteristic peaks at 2θ angles of 8.5±0.2°, 8.9±0.2°, and 13.2±0.2°, and yet still further has characteristic peaks at 2θ angles of 15.4±0.2°, 20.7±0.2°, 22.3±0.2°, 22.6±0.2°, 24.7±0.2°, and 26.1±0.2°;
preferably, the crystal form I of 2-hydroxyethanesulfonate has an X-ray powder diffraction pattern substantially as shown in FIG. 11-1;
preferably, the crystal form I of 2-hydroxyethanesulfonate is an anhydrate.

5. The crystal form of the salt as claimed in claim 4, wherein the crystal form of ethanesulfonate comprises a crystal form I of ethanesulfonate and a crystal form II of ethanesulfonate;
preferably, by X-ray powder diffraction using Cu-Kα radiation, the crystal form I of ethanesulfonate has a characteristic peak at a 2θ angle of 24.6±0.2°, further has characteristic peaks at 2θ angles of 10.4±0.2° and 14.8±0.2°, still further has characteristic peaks at 2θ angles of 16.9±0.2°, 19.3±0.2°, and 21.4±0.2°, and yet still further has characteristic peaks at 2θ angles of 15.8±0.2°, 17.4±0.2°, 20.5±0.2°, 23.0±0.2°, and 26.2±0.2°;
preferably, the crystal form I of ethanesulfonate has an X-ray powder diffraction pattern substantially as shown in FIG. 12-1-1 or FIG. 12-1-2;
preferably, the crystal form I of ethanesulfonate is an anhydrate;
preferably, by X-ray powder diffraction using Cu-Kα radiation, the crystal form II of ethanesulfonate has characteristic peaks at 2θ angles of 10.6±0.2° and 17.5±0.2°, further has characteristic peaks at 2θ angles of 4.5±9.2°, 9.0±0.2°, and 19.2±0.2°, and still further has characteristic peaks at 2θ angles of 13.5±0.2°, 14.7±0.2°, 16.2±0.2°, 18.0±0.2°, 22.7±0.2°, 25.2±0.2°, and 26.9±0.2°;
preferably, the crystal form II of ethanesulfonate has an X-ray powder diffraction pattern substantially as shown in FIG. 13-1;
preferably, the crystal form II of ethanesulfonate is a solvate, such as an organic solvate or a hydrate; preferably, the crystal form of tartrate comprises a crystal form of L-tartrate and a crystal form of D-tartrate, preferably a crystal form I of L-tartrate;
preferably, by X-ray powder diffraction using Cu-Kα radiation, the crystal form I of L-tartrate has characteristic peaks at 2θ angles of 11.6±0.2°, 15.5±0.2°, and 20.8±0.2°, further has characteristic peaks at 2θ angles of 9.2±0.2°, 18.7±0.2°, and 21.1±0.2°, and still further has characteristic peaks at 2θ angles of 7.2±0.2°, 7.7±0.2°, 12.1±0.2°, 14.5±0.2°, 19.4±0.2°, and 22.6±0.2°;
preferably, the crystal form I of L-tartrate has an X-ray powder diffraction pattern substantially as shown in FIG. 14-1;
preferably, the crystal form I of L-tartrate is an anhydrate.

6. The crystal form of the salt as claimed in claim 4, wherein the crystal form of 1,5-naphthalenedisulfonate comprises a crystal form I of 1,5-naphthalenedisulfonate and a crystal form II of 1,5-naphthalenedisulfonate;
preferably, by X-ray powder diffraction using Cu-Kα radiation, the crystal form I of 1,5-naphthalenedisulfonate has characteristic peaks at 2θ angles of 7.7±0.2°, 18.0±0.2°, and 23.5±0.2°, further has characteristic peaks at 2θ angles of 15.4±0.2°, 24.0±0.2°, and 24.8±0.2°, and still further has characteristic peaks at 2θ angles of 8.0±0.2°, 8.6±0.2°, 10.9±0.2°, 11.9±0.2°, 16.8±0.2°, 17.7±0.2°, 19.8±0.2°, and 22.6±0.2°;
preferably, the crystal form I of 1,5-naphthalenedisulfonate has an X-ray powder diffraction pattern substantially as shown in FIG. 15-1;
preferably, the crystal form I of 1,5-naphthalenedisulfonate is a hydrate;
preferably, by X-ray powder diffraction using Cu-Kα radiation, the crystal form II of 1,5-naphthalenedisulfonate has characteristic peaks at 2θ angles of 25.5±0.2° and 10.2±0.2°, further has characteristic peaks at 2θ angles of 3.1±0.2°, 3.9±0.2°, 8.6±0.2°, 12.7±0.2°, and 13.9±0.2°, and still further has characteristic peaks at 2θ angles of 8.4±0.2°, 11.4±0.2°, 14.4±0.2°, 15.6±0.2°, 20.5±0.2°, 20.9±0.2°, 22.0±0.2°, and 23.8±0.2°;
preferably, the crystal form II of 1,5-naphthalenedisulfonate has an X-ray powder diffraction pattern substantially as shown in FIG. 16-1;
preferably, the crystal form II of 1,5-naphthalenedisulfonate is a hydrate.

7. A preparation method for the salt of compound I as claimed in any one of claims 1-2 or the crystal form of the salt as claimed in any one of claims 4-6, comprising mixing compound I with an acid and then reacting in a solvent 2 to give the salt of compound I or the crystal form of the salt, wherein preferably, the solvent 2 is selected from one, two, or more of an organic solvent 2, water, and a mixed solvent of the organic solvent 2 and water;
preferably, the organic solvent 2 is selected from one, two, or more of methanol, ethanol, isopropanol, butanol, acetone, butanone, ethyl acetate, isopropyl acetate, methyl *tert*-butyl ether, dichloromethane, acetonitrile, tetrahydrofuran, *n*-heptane, dimethylsulfoxide, 2-methyl-tetrahydrofuran, and chloroform.

8. A pharmaceutical composition, comprising an active ingredient and optionally a pharmaceutically acceptable carrier, wherein the active ingredient is the salt of compound I as claimed in any one of claims 1-2, the free base crystal form as claimed in claim 3, or the crystal form of the salt of the compound as claimed in any one of claims 4-6;
preferably, the pharmaceutical composition can further comprise an additional active ingredient, such as an additional ROCK inhibitor.

9. Use of the salt of compound I as claimed in any one of claims 1-2, the free base crystal form as claimed in claim 3, the crystal form of the salt of the compound as claimed in any one of claims 4-6, or the pharmaceutical composition as claimed in claim 8 for the manufacturing of a preparation,
wherein
preferably, the preparation is a ROCK antagonist;
preferably, the ROCK antagonist is for use in the prevention and/or treatment of one or more diseases caused by high expression or excessive activation of ROCK;
preferably, the disease is selected from cardiovascular and cerebrovascular diseases, neurological diseases, fibrosis diseases, ocular diseases, tumors, arterial thrombotic disorders, radiation damage, respiratory system diseases, autoimmune diseases, microbial infections, muscular dystrophy, and diseases related to impaired lymphatic drainage; preferably, the disease comprises atherosclerosis, acute coronary syndrome, hypertension, cerebral vasospasm, cerebral ischemia, ischemic stroke, restenosis, heart disease, heart failure, myocardial hypertrophy, myocardial ischemia-reperfusion injury, diabetes, diabetic nephropathy, cancer, neuronal degeneration, nerve injury diseases, spinal cord injury, erectile dysfunction, platelet aggregation, leukocyte aggregation, glaucoma, ocular hypertension, asthma, osteoporosis, pulmonary fibrosis (e.g., idiopathic interstitial pulmonary fibrosis), hepatic fibrosis, renal fibrosis, COPD, renal dialysis, glomerulosclerosis, neuronal degeneration inflammation, fungal infections, bacterial infections, viral infections, Duchenne muscular dystrophy, fatty liver disease, and steatohepatitis.

10. A preparation, comprising the salt of compound I as claimed in any one of claims 1-2, the free base crystal form as claimed in claim 3, or the crystal form of the salt of the compound as claimed in any one of claims 4-6, wherein
preferably, the preparation comprises the pharmaceutical composition as claimed in claim 8.

11. A method for preventing and/or treating a disease caused by high expression or excessive activation of ROCK, comprising administering to a subject a therapeutically effective amount of the salt of compound I as claimed in any one of claims 1-2, the free base crystal form as claimed in claim 3, the crystal form of the salt of the compound as claimed in any one of claims 4-6, the pharmaceutical composition as claimed in claim 8, or the preparation as claimed in claim 10.
